# EUROPEAN PATENT SPECIFICATION

(11) **EP 2 635 684 B1**
(45) Date of publication and mention of the grant of the patent: **26.10.2016**
(21) Application number: 11837364.6
(22) Date of filing: 03.11.2011
(51) Int. Cl.: C07K 14/005, C12N 15/82

(54) **PLANT EXPRESSION SYSTEM**
PFLANZENEXPRESSIONSSYSTEM
SYSTÈME D'EXPRESSION VÉGÉTALE

(30) Priority: 04.11.2010 US 410241 P
(43) Date of publication of application: 11.09.2013
(73) Proprietor: Medicago Inc., Québec, Québec G1V 3V9 (CA)
(72) Inventor: D'AOUST, Marc-Andre, Quebec Quebec G1X 4N4 (CA); LAVOIE, Pierre-Olivier, Quebec Quebec G1N 2I7 (CA); VEZINA, Louis-Philippe, Neuville Quebec G0A 2R0 (CA)
(74) Representative: van Kooij, Adriaan
(86) International application number: PCT/CA2011/001228
(87) International publication number: WO 2012/058762

(56) References cited:
- WO-A1-2008/087391
- WO-A1-2010/003225
- WO-A1-2010/025285
- HUTANG ET AL.: 'A DNA replicon system for rapid high-level production of virus- like particles in plants' BIOTECHNOL BIOENG. vol. 103, no. 4, 01 July 2009, pages 706 - 714, XP055027289
- HUANG ET AL.: 'High-level rapid production of full-sized monoclonal antibodies in plants by a single-vector DNA replicon system' BIOTECHNOL BIOENG. vol. 106, no. 1, 01 May 2010, pages 9 - 17, XP055106021
- REGNARD ET AL.: 'High level protein expression in plants through the use of a novel autonomously replicating geminivirus shuttle vector' PLANT BIOTECHNOLOGY JOURNAL. vol. 8, no. 1, January 2010, pages 38 - 46, XP055106017
- SAINSBURY ET AL.: 'Extremely high-level and rapid transient protein production in plants without the use of viral replication' PLANT PHYSIOLOGY vol. 148, November 2008, pages 1212 - 1218, XP002523655

### SEQUENCE LISTING

<110> MEDICAGO INC., ET AL
   D'AOUST, Marc-Andre
   LAVOIE" Pierre-Olivier
   VEZINA " Louis-Philippe
<120> Plant Expression System
<130> V83266WO
<150> US 61/410,241
   <151> 2010-11-04
<160> 23
<170> PatentIn version 3.5
<210> 1
   <211> 3505
   <212> DNA
   <213> Artificial sequence
<220>
   <223> Synthesized expression cassette from PacI site
<400> 1
   ttaattaagt cgacaagctt gcatgcctgc aggtcaacat ggtggagcac gacacacttg      60
   tctactccaa aaatatcaaa gatacagtct cagaagacca aagggcaatt gagacttttc     120
   aacaaagggt aatatccgga aacctcctcg gattccattg cccagctatc tgtcacttta     180
   ttgtgaagat agtggaaaag gaaggtggct cctacaaatg ccatcattgc gataaaggaa     240
   aggccatcgt tgaagatgcc tctgccgaca gtggtcccaa agatggaccc ccacccacga     300
   ggagcatcgt ggaaaaagaa gacgttccaa ccacgtcttc aaagcaagtg gattgatgtg     360
   ataacatggt ggagcacgac acacttgtct actccaaaaa tatcaaagat acagtctcag     420
   aagaccaaag ggcaattgag acttttcaac aaagggtaat atccggaaac ctcctcggat     480
   tccattgccc agctatctgt cactttattg tgaagatagt ggaaaaggaa ggtggctcct     540
   acaaatgcca tcattgcgat aaaggaaagg ccatcgttga agatgcctct gccgacagtg     600
   gtcccaaaga tggaccccca cccacgagga gcatcgtgga aaaagaagac gttccaacca     660
   cgtcttcaaa gcaagtggat tgatgtgata tctccactga cgtaagggat gacgcacaat     720
   cccactatcc ttcgcaagac ccttcctcta tataaggaag ttcatttcat ttggagaggt     780
   attaaaatct taataggttt tgataaaagc gaacgtgggg aaacccgaac caaaccttct     840
   tctaaactct ctctcatctc tcttaaagca aacttctctc ttgtctttct tgcgtgagcg     900
   atcttcaacg ttgtcagatc gtgcttcggc accagtacaa cgttttcttt cactgaagcg     960
   aaatcaaaga tctctttgtg gacacgtagt gcggcgccat taaataacgt gtacttgtcc    1020
   tattcttgtc ggtgtggtct tgggaaaaga aagcttgctg gaggctgctg ttcagcccca    1080
   tacattactt gttacgattc tgctgacttt cggcgggtgc aatatctcta cttctgcttg    1140
   acgaggtatt gttgcctgta cttctttctt cttcttcttg ctgattggtt ctataagaaa    1200
   tctagtattt tctttgaaac agagttttcc cgtggttttc gaacttggag aaagattgtt    1260
   aagcttctgt atattctgcc caaatttgtc gggcccatgg agaaaatagt gcttcttctt    1320
   gcaatagtca gtcttgttaa aagtgatcag atttgcattg gttaccatgc aaacaattca    1380
   acagagcagg ttgacacaat catggaaaag aacgttactg ttacacatgc ccaagacata    1440
   ctggaaaaga cacacaacgg gaagctctgc gatctagatg gagtgaagcc tctaatttta    1500
   agagattgta gtgtagctgg atggctcctc gggaacccaa tgtgtgacga attcatcaat    1560
   gtaccggaat ggtcttacat agtggagaag gccaatccaa ccaatgacct ctgttaccca    1620
   gggagtttca acgactatga agaactgaaa cacctattga gcagaataaa ccattttgag    1680
   aaaattcaaa tcatccccaa aagttcttgg tccgatcatg aagcctcatc aggagttagc    1740
   tcagcatgtc catacctggg aagtccctcc ttttttagaa atgtggtatg gcttatcaaa    1800
   aagaacagta catacccaac aataaagaaa agctacaata ataccaacca agaggatctt    1860
   ttggtactgt ggggaattca ccatcctaat gatgcggcag agcagacaag gctatatcaa    1920
   aacccaacca cctatatttc cattgggaca tcaacactaa accagagatt ggtaccaaaa    1980
   atagctacta gatccaaagt aaacgggcaa agtggaagga tggagttctt ctggacaatt    2040
   ttaaaaccta atgatgcaat caacttcgag agtaatggaa atttcattgc tccagaatat    2100
   gcatacaaaa ttgtcaagaa aggggactca gcaattatga aaagtgaatt ggaatatggt    2160
   aactgcaaca ccaagtgtca aactccaatg ggggcgataa actctagtat gccattccac    2220
   aacatacacc ctctcaccat cggggaatgc cccaaatatg tgaaatcaaa cagattagtc    2280
   cttgcaacag ggctcagaaa tagccctcaa agagagagca gaagaaaaaa gagaggacta    2340
   tttggagcta tagcaggttt tatagaggga ggatggcagg gaatggtaga tggttggtat    2400
   gggtaccacc atagcaatga gcaggggagt gggtacgctg cagacaaaga atccactcaa    2460
   aaggcaatag atggagtcac caataaggtc aactcaatca ttgacaaaat gaacactcag    2520
   tttgaggccg ttggaaggga atttaataac ttagaaagga gaatagagaa tttaaacaag    2580
   aagatggaag acgggtttct agatgtctgg acttataatg ccgaacttct ggttctcatg    2640
   gaaaatgaga gaactctaga ctttcatgac tcaaatgtta agaacctcta cgacaaggtc    2700
   cgactacagc ttagggataa tgcaaaggag ctgggtaacg gttgtttcga gttctatcac    2760
   aaatgtgata atgaatgtat ggaaagtata agaaacggaa cgtacaacta tccgcagtat    2820
   tcagaagaag caagattaaa aagagaggaa ataagtgggg taaaattgga atcaatagga     2880
   acttaccaaa tactgtcaat ttattcaaca gtggcgagtt ccctagcact ggcaatcatg     2940
   atggctggtc tatctttatg gatgtgctcc aatggatcgt tacaatgcag aatttgcatt     3000
   taaaggccta ttttctttag tttgaattta ctgttattcg gtgtgcattt ctatgtttgg     3060
   tgagcggttt tctgtgctca gagtgtgttt attttatgta atttaatttc tttgtgagct     3120
   cctgtttagc aggtcgtccc ttcagcaagg acacaaaaag attttaattt tattaaaaaa     3180
   aaaaaaaaaa aagaccggga attcgatatc aagcttatcg acctgcagat cgttcaaaca     3240
   tttggcaata aagtttctta agattgaatc ctgttgccgg tcttgcgatg attatcatat     3300
   aatttctgtt gaattacgtt aagcatgtaa taattaacat gtaatgcatg acgttattta     3360
   tgagatgggt ttttatgatt agagtcccgc aattatacat ttaatacgcg atagaaaaca     3420
   aaatatagcg cgcaaactag gataaattat cgcgcgcggt gtcatctatg ttactagatc     3480
   tctagagtct caagcttggc gcgcc                                           3505
<210> 2
   <211> 3520
   <212> DNA
   <213> Artificial Sequence
<220>
   <223> Synthesized expression cassette 560 from PacI site
<400> 2
   ttaattaagt cgacaagctt gcatgcctgc aggtcaacat ggtggagcac gacacacttg    60
   tctactccaa aaatatcaaa gatacagtct cagaagacca aagggcaatt gagacttttc   120
   aacaaagggt aatatccgga aacctcctcg gattccattg cccagctatc tgtcacttta   180
   ttgtgaagat agtggaaaag gaaggtggct cctacaaatg ccatcattgc gataaaggaa   240
   aggccatcgt tgaagatgcc tctgccgaca gtggtcccaa agatggaccc ccacccacga   300
   ggagcatcgt ggaaaaagaa gacgttccaa ccacgtcttc aaagcaagtg gattgatgtg   360
   ataacatggt ggagcacgac acacttgtct actccaaaaa tatcaaagat acagtctcag   420
   aagaccaaag ggcaattgag acttttcaac aaagggtaat atccggaaac ctcctcggat   480
   tccattgccc agctatctgt cactttattg tgaagatagt ggaaaaggaa ggtggctcct   540
   acaaatgcca tcattgcgat aaaggaaagg ccatcgttga agatgcctct gccgacagtg   600
   gtcccaaaga tggaccccca cccacgagga gcatcgtgga aaaagaagac gttccaacca   660
   cgtcttcaaa gcaagtggat tgatgtgata tctccactga cgtaagggat gacgcacaat   720
   cccactatcc ttcgcaagac ccttcctcta tataaggaag ttcatttcat ttggagaggt   780
   attaaaatct taataggttt tgataaaagc gaacgtgggg aaacccgaac caaaccttct   840
   tctaaactct ctctcatctc tcttaaagca aacttctctc ttgtctttct tgcgtgagcg      900
   atcttcaacg ttgtcagatc gtgcttcggc accagtacaa cgttttcttt cactgaagcg      960
   aaatcaaaga tctctttgtg gacacgtagt gcggcgccat taaataacgt gtacttgtcc     1020
   tattcttgtc ggtgtggtct tgggaaaaga aagcttgctg gaggctgctg ttcagcccca     1080
   tacattactt gttacgattc tgctgacttt cggcgggtgc aatatctcta cttctgcttg     1140
   acgaggtatt gttgcctgta cttctttctt cttcttcttg ctgattggtt ctataagaaa     1200
   tctagtattt tctttgaaac agagttttcc cgtggttttc gaacttggag aaagattgtt     1260
   aagcttctgt atattctgcc caaatttgtc gggcccatgg cgaaaaacgt tgcgattttc     1320
   ggcttattgt ttctcttcct tgtgttggtt ccttctcaga tcttcgctga cacattatgt     1380
   ataggttatc atgcgaacaa ttcaacagac actgtagaca cagtactaga aaagaatgta     1440
   acagtaacac actctgttaa ccttctagaa gacaagcata acgggaaact atgcaaacta     1500
   agaggggtag ccccattgca tttgggtaaa tgtaacattg ctggctggat cctgggaaat     1560
   ccagagtgtg aatcactctc cacagcaagc tcatggtcct acattgtgga aacacctagt     1620
   tcagacaatg gaacgtgtta cccaggagat ttcatcgatt atgaggagct aagagagcaa     1680
   ttaagctcag tgtcatcatt tgaaaggttt gagatattcc ccaagacaag ttcatggccc     1740
   aatcatgact cgaacaaagg tgtaacggca gcatgtcctc atgctggagc aaaaagcttc     1800
   tacaaaaatt taatatggct agttaaaaaa ggaaattcat acccaaagct cagcaaatcc     1860
   tacattaatg ataaagggaa agaagtcctc gtgctatggg gcattcacca tccatctact     1920
   agtgctgacc aacaaagtct ctatcagaat gcagatacat atgtttttgt ggggtcatca     1980
   agatacagca agaagttcaa gccggaaata gcaataagac ccaaagtgag ggatcaagaa     2040
   gggagaatga actattactg gacactagta gagccgggag acaaaataac attcgaagca     2100
   actggaaatc tagtggtacc gagatatgca ttcgcaatgg aaagaaatgc tggatctggt     2160
   attatcattt cagatacacc agtccacgat tgcaatacaa cttgtcaaac acccaagggt     2220
   gctataaaca ccagcctccc atttcagaat atacatccga tcacaattgg aaaatgtcca     2280
   aaatatgtaa aaagcacaaa attgagactg gccacaggat tgaggaatat cccgtctatt     2340
   caatctagag gactatttgg ggccattgcc ggtttcattg aaggggggtg gacagggatg     2400
   gtagatggat ggtacggtta tcaccatcaa aatgagcagg ggtcaggata tgcagccgac     2460
   ctgaagagca cacagaatgc cattgacgag attactaaca aagtaaattc tgttattgaa     2520
   aagatgaata cacagttcac agcagtaggt aaagagttca accacctgga aaaaagaata     2580
   gagaatttaa ataaaaaagt tgatgatggt ttcctggaca tttggactta caatgccgaa     2640
   ctgttggttc tattggaaaa tgaaagaact ttggactacc acgattcaaa tgtgaagaac     2700
   ttatatgaaa aggtaagaag ccagctaaaa aacaatgcca aggaaattgg aaacggctgc     2760
   tttgaatttt accacaaatg cgataacacg tgcatggaaa gtgtcaaaaa tgggacttat     2820
   gactacccaa aatactcaga ggaagcaaaa ttaaacagag aagaaataga tggggtaaag     2880
   ctggaatcaa caaggattta ccagattttg gcgatctatt caactgtcgc cagttcattg     2940
   gtactggtag tctccctggg ggcaatcagt ttctggatgt gctctaatgg gtctctacag     3000
   tgtagaatat gtatttaaag gcctattttc tttagtttga atttactgtt attcggtgtg     3060
   catttctatg tttggtgagc ggttttctgt gctcagagtg tgtttatttt atgtaattta     3120
   atttctttgt gagctcctgt ttagcaggtc gtcccttcag caaggacaca aaaagatttt     3180
   aattttatta aaaaaaaaaa aaaaaaagac cgggaattcg atatcaagct tatcgacctg     3240
   cagatcgttc aaacatttgg caataaagtt tcttaagatt gaatcctgtt gccggtcttg     3300
   cgatgattat catataattt ctgttgaatt acgttaagca tgtaataatt aacatgtaat     3360
   gcatgacgtt atttatgaga tgggttttta tgattagagt cccgcaatta tacatttaat     3420
   acgcgataga aaacaaaata tagcgcgcaa actaggataa attatcgcgc gcggtgtcat     3480
   ctatgttact agatctctag agtctcaagc ttggcgcgcc                           3520
<210> 3
   <211> 3523
   <212> DNA
   <213> Artificial Sequence
<220>
   <223> Synthesized expression cassette 971 from PacI site
<400> 3
   ttaattaagt cgacaagctt gcatgcctgc aggtcaacat ggtggagcac gacacacttg 60
   tctactccaa aaatatcaaa gatacagtct cagaagacca aagggcaatt gagacttttc 120
   aacaaagggt aatatccgga aacctcctcg gattccattg cccagctatc tgtcacttta 180
   ttgtgaagat agtggaaaag gaaggtggct cctacaaatg ccatcattgc gataaaggaa 240
   aggccatcgt tgaagatgcc tctgccgaca gtggtcccaa agatggaccc ccacccacga 300
   ggagcatcgt ggaaaaagaa gacgttccaa ccacgtcttc aaagcaagtg gattgatgtg 360
   ataacatggt ggagcacgac acacttgtct actccaaaaa tatcaaagat acagtctcag 420
   aagaccaaag ggcaattgag acttttcaac aaagggtaat atccggaaac ctcctcggat 480
   tccattgccc agctatctgt cactttattg tgaagatagt ggaaaaggaa ggtggctcct 540
   acaaatgcca tcattgcgat aaaggaaagg ccatcgttga agatgcctct gccgacagtg 600
   gtcccaaaga tggaccccca cccacgagga gcatcgtgga aaaagaagac gttccaacca 660
   cgtcttcaaa gcaagtggat tgatgtgata tctccactga cgtaagggat gacgcacaat 720
   cccactatcc ttcgcaagac ccttcctcta tataaggaag ttcatttcat ttggagaggt 780
   attaaaatct taataggttt tgataaaagc gaacgtgggg aaacccgaac caaaccttct 840
   tctaaactct ctctcatctc tcttaaagca aacttctctc ttgtctttct tgcgtgagcg 900
   atcttcaacg ttgtcagatc gtgcttcggc accagtacaa cgttttcttt cactgaagcg 960
   aaatcaaaga tctctttgtg gacacgtagt gcggcgccat taaataacgt gtacttgtcc 1020
   tattcttgtc ggtgtggtct tgggaaaaga aagcttgctg gaggctgctg ttcagcccca 1080
   tacattactt gttacgattc tgctgacttt cggcgggtgc aatatctcta cttctgcttg 1140
   acgaggtatt gttgcctgta cttctttctt cttcttcttg ctgattggtt ctataagaaa 1200
   tctagtattt tctttgaaac agagttttcc cgtggttttc gaacttggag aaagattgtt 1260
   aagcttctgt atattctgcc caaatttgtc gggcccatgg cgaaaaacgt tgcgattttc 1320
   ggcttattgt tttctcttct tgtgttggtt ccttctcaga tcttcgctca aaaacttccc 1380
   ggaaatgaca acagcacggc aacgctgtgc cttgggcacc atgcagtacc aaacggaacg 1440
   atagtgaaaa caatcacgaa tgaccaaatt gaagttacta atgctactga gctggttcag 1500
   agttcctcaa caggtgaaat atgcgacagt cctcatcaga tccttgatgg agaaaactgc 1560
   acactaatag atgctctatt gggagaccct cagtgtgatg gcttccaaaa taagaaatgg 1620
   gacctttttg ttgaacgcag caaagcctac agcaactgtt acccttatga tgtgccggat 1680
   tatgcctccc ttaggtcact agttgcctca tccggcacac tggagtttaa caatgaaagt 1740
   ttcaattgga ctggagtcac tcaaaacgga acaagctctg cttgcataag gagatctaat 1800
   aacagtttct ttagtagatt gaattggttg acccacttaa aattcaaata cccagcattg 1860
   aacgtgacta tgccaaacaa tgaaaaattt gacaaattgt acatttgggg ggttcaccac 1920
   ccgggtacgg acaatgacca aatcttcctg tatgctcaag catcaggaag aatcacagtc 1980
   tctaccaaaa gaagccaaca aactgtaatc ccgaatatcg gatctagacc cagagtaagg 2040
   aatatcccca gcagaataag catctattgg acaatagtaa aaccgggaga catacttttg 2100
   attaacagca cagggaatct aattgctcct aggggttact tcaaaatacg aagtgggaaa 2160
   agctcaataa tgagatcaga tgcacccatt ggcaaatgca attctgaatg catcactcca 2220
   aacggaagca ttcccaatga caaaccattc caaaatgtaa acaggatcac atacggggcc 2280
   tgtcccagat atgttaagca aaacactctg aaattggcaa cagggatgcg aaatgtacca 2340
   gagaaacaaa ctagaggcat atttggcgca atcgcgggtt tcatagaaaa tggttgggag 2400
   ggaatggtgg atggttggta tggtttcagg catcaaaatt ctgagggaat aggacaagca 2460
   gcagatctca aaagcactca agcagcaatc gatcaaatca atgggaagct gaataggttg 2520
   atcgggaaaa ccaacgagaa attccatcag attgaaaaag agttctcaga agtcgaaggg 2580
   agaatccagg accttgagaa atatgttgag gacaccaaaa tagatctctg gtcatacaac 2640
   gcggagcttc ttgttgccct ggagaaccaa catacaattg atctaactga ctcagaaatg 2700
   aacaaactgt ttgaaaaaac aaagaagcaa ctgagggaaa atgctgagga tatgggcaat 2760
   ggttgtttca aaatatacca caaatgtgac aatgcctgca taggatcaat cagaaatgga 2820
   acttatgacc acgatgtata cagagatgaa gcattaaaca accggttcca gatcaagggc 2880
   gttgagctga agtcaggata caaagattgg atactatgga tttcctttgc catatcatgt 2940
   tttttgcttt gtgttgcttt gttggggttc atcatgtggg cctgccaaaa aggcaacatt 3000
   aggtgcaaca tttgcatttg aaggcctatt ttctttagtt tgaatttact gttattcggt 3060
   gtgcatttct atgtttggtg agcggttttc tgtgctcaga gtgtgtttat tttatgtaat 3120
   ttaatttctt tgtgagctcc tgtttagcag gtcgtccctt cagcaaggac acaaaaagat 3180
   tttaatttta ttaaaaaaaa aaaaaaaaaa gaccgggaat tcgatatcaa gcttatcgac 3240
   ctgcagatcg ttcaaacatt tggcaataaa gtttcttaag attgaatcct gttgccggtc 3300
   ttgcgatgat tatcatataa tttctgttga attacgttaa gcatgtaata attaacatgt 3360
   aatgcatgac gttatttatg agatgggttt ttatgattag agtcccgcaa ttatacattt 3420
   aatacgcgat agaaaacaaa atatagcgcg caaactagga taaattatcg cgcgcggtgt 3480
   catctatgtt actagattct agagtctcaa gcttcggcgc gcc 3523
<210> 4
   <211> 3580
   <212> DNA
   <213> Artificial Sequence
<220>
   <223> Synthesized expression cassette 973 from PacI site
<400> 4
   ttaattaagt cgacaagctt gcatgcctgc aggtcaacat ggtggagcac gacacacttg        60
   tctactccaa aaatatcaaa gatacagtct cagaagacca aagggcaatt gagacttttc       120
   aacaaagggt aatatccgga aacctcctcg gattccattg cccagctatc tgtcacttta       180
   ttgtgaagat agtggaaaag gaaggtggct cctacaaatg ccatcattgc gataaaggaa       240
   aggccatcgt tgaagatgcc tctgccgaca gtggtcccaa agatggaccc ccacccacga       300
   ggagcatcgt ggaaaaagaa gacgttccaa ccacgtcttc aaagcaagtg gattgatgtg       360
   ataacatggt ggagcacgac acacttgtct actccaaaaa tatcaaagat acagtctcag       420
   aagaccaaag ggcaattgag acttttcaac aaagggtaat atccggaaac ctcctcggat       480
   tccattgccc agctatctgt cactttattg tgaagatagt ggaaaaggaa ggtggctcct       540
   acaaatgcca tcattgcgat aaaggaaagg ccatcgttga agatgcctct gccgacagtg     600
   gtcccaaaga tggaccccca cccacgagga gcatcgtgga aaaagaagac gttccaacca     660
   cgtcttcaaa gcaagtggat tgatgtgata tctccactga cgtaagggat gacgcacaat     720
   cccactatcc ttcgcaagac ccttcctcta tataaggaag ttcatttcat ttggagaggt     780
   attaaaatct taataggttt tgataaaagc gaacgtgggg aaacccgaac caaaccttct     840
   tctaaactct ctctcatctc tcttaaagca aacttctctc ttgtctttct tgcgtgagcg     900
   atcttcaacg ttgtcagatc gtgcttcggc accagtacaa cgttttcttt cactgaagcg     960
   aaatcaaaga tctctttgtg gacacgtagt gcggcgccat taaataacgt gtacttgtcc    1020
   tattcttgtc ggtgtggtct tgggaaaaga aagcttgctg gaggctgctg ttcagcccca    1080
   tacattactt gttacgattc tgctgacttt cggcgggtgc aatatctcta cttctgcttg    1140
   acgaggtatt gttgcctgta cttctttctt cttcttcttg ctgattggtt ctataagaaa    1200
   tctagtattt tctttgaaac agagttttcc cgtggttttc gaacttggag aaagattgtt    1260
   aagcttctgt atattctgcc caaatttgtc gggcccatgg cgaaaaacgt tgcgattttc    1320
   ggcttattgt ttctcttctt tgtgttggtt ccttctcaga tcttcgctga tcgaatctgc    1380
   actggaataa catcttcaaa ctcacctcat gtggtcaaaa cagccactca aggggaggtc    1440
   aatgtgactg gtgtgatacc actaacaaca acaccaacaa aatcttattt tgcaaatctc    1500
   aaaggaacaa ggaccagagg gaaactatgc ccagactgtc tcaactgcac agatctggat    1560
   gtggctttgg gcagaccaat gtgtgtgggg accacacctt cggcgaaggc ttcaatactc    1620
   cacgaagtca aacctgttac atccgggtgc tttcctataa tgcacgacag aacaaaaatc    1680
   aggcaactac ccaatcttct cagaggatat gaaaatatca ggctatcaac ccaaaacgtc    1740
   atcgatgcgg aaaaggcacc aggaggaccc tacagacttg gaacctcagg atcttgccct    1800
   aacgctacca gtaagagcgg atttttcgca acaatggctt gggctgtccc aaaggacaac    1860
   aacaaaaatg caacgaaccc actaacagta gaagtaccat acatttgtac agaaggggaa    1920
   gaccaaatca ctgtttgggg gttccattca gataacaaaa cccaaatgaa gaacctctat    1980
   ggagactcaa atcctcaaaa gttcacctca tctgctaatg gagtaaccac acactatgtt    2040
   tctcagattg gcagcttccc agatcaaaca gaagacggag gactaccaca aagcggcagg    2100
   attgttgttg attacatgat gcaaaaacct gggaaaacag gaacaattgt ctaccaaaga    2160
   ggtgttttgt tgcctcaaaa ggtgtggtgc gcgagtggca ggagcaaagt aataaaaggg    2220
   tccttgcctt taattggtga agcagattgc cttcatgaaa aatacggtgg attaaacaaa    2280
   agcaagcctt actacacagg agaacatgca aaagccatag gaaattgccc aatatgggtg    2340
   aaaacacctt tgaagctcgc caatggaacc aaatatagac ctcctgcaaa actattaaag    2400
   gaaaggggtt tcttcggagc tattgctggt ttcctagaag gaggatggga aggaatgatt      2460
   gcaggctggc acggatacac atctcacgga gcacatggag tggcagtggc ggcggacctt      2520
   aagagtacgc aagaagctat aaacaagata acaaaaaatc tcaattcttt gagtgagcta      2580
   gaagtaaaga atcttcaaag actaagtggt gccatggatg aactccacaa cgaaatactc      2640
   gagctggatg agaaagtgga tgatctcaga gctgacacta taagctcgca aatagaactt      2700
   gcagtcttgc tttccaacga aggaataata aacagtgaag atgagcatct attggcactt      2760
   gagagaaaac taaagaaaat gctgggtccc tctgctgtag agataggaaa tggatgcttc      2820
   gaaaccaaac acaagtgcaa ccagacctgc ttagacagga tagctgctgg cacctttaat      2880
   gcaggagaat ttctctcccc cacttttgat tcactgaaca ttactgctgc atctttaaat      2940
   gatgatggat tggataacca tactatactg ctctattact caactgctgc ttctagtttg      3000
   gctgtaacat tgatgctagc tatttttatt gtttatatgg tctccagaga caacgtttca      3060
   tgctccatct gtctataaag gcctattttc tttagtttga atttactgtt attcggtgtg      3120
   catttctatg tttggtgagc ggttttctgt gctcagagtg tgtttatttt atgtaattta      3180
   atttctttgt gagctcctgt ttagcaggtc gtcccttcag caaggacaca aaaagatttt      3240
   aattttatta aaaaaaaaaa aaaaaaagac cgggaattcg atatcaagct tatcgacctg      3300
   cagatcgttc aaacatttgg caataaagtt tcttaagatt gaatcctgtt gccggtcttg      3360
   cgatgattat catataattt ctgttgaatt acgttaagca tgtaataatt aacatgtaat      3420
   gcatgacgtt atttatgaga tgggttttta tgattagagt cccgcaatta tacatttaat      3480
   acgcgataga aaacaaaata tagcgcgcaa actaggataa attatcgcgc gcggtgtcat      3540
   ctatgttact agattctaga gtctcaagct tcggcgcgcc                            3580
<210> 5
   <211> 52
   <212> DNA
   <213> Artificial Sequence
<220>
   <223> Synthesized PCR fragment containing BeYDV LIR amplified using MfeI-MluI-AscI-LIR.c
<400> 5
   caattgacgc gtggcgcgcc ctagcagaag gcatgttgtt gtgactccga gg 52
<210> 6
   <211> 46
   <212> DNA
   <213> Artificial Sequence
<220>
   <223> Synthesized PCR fragment containing BeYDV LIR amplified using SbfI-AflII-HindIII-LIR.r
<400> 6
   cctgcaggct taagaagctt gtacgaataa ttcgtatccg acggaa 46
<210> 7
   <211> 44
   <212> DNA
   <213> Artificial Sequence
<220>
   <223> Synthesized fragment containing BeYDV LIR and SIR sequences amplified using SacI-EcoRI-SIR.c
<400> 7
   taccgagctc gaattccgag tgtacttcaa gtcagttgga aatc 44
<210> 8
   <211> 48
   <212> DNA
   <213> Artificial Sequence
<220>
   <223> Synthesized fragment containing BeYDV LIR and SIR sequences amplified using SpeI-FseI-LIR.r
<400> 8
   actagtggcc ggccgtacga ataattcgta tccgacggaa atacctga 48
<210> 9
   <211> 1303
   <212> DNA
   <213> Artificial Sequence
<220>
   <223> Synthesized annotated LIR-MCS-SIR+LIR
<400> 9
   ctagcagaag gcatgttgtt gtgactccga ggggttgcct caaactctat cttataaccg    60
   gcgtggaggc atggaggcaa gggcattttg gtaatttaag tagttagtgg aaaatgacgt   120
   catttactta aagacgaagt cttgcgacaa gggggcccca cgccgaattt taatattacc   180
   ggcgtggccc caccttatcg cgagtgcttt agcacgagcg gtccagattt aaagtagaaa   240
   agttcccgcc cactagggtt aaaggtgttc acactataaa agcatatacg atgtgatggt   300
   atttgataaa gcgtatattg tatcaggtat ttccgtcgga tacgaattat tcgtacaagc   360
   ttcttaagcc tgcaggtcga ctctagagga tccccgggta ccgagctcga attccgagtg   420
   tacttcaagt cagttggaaa tcaataaaat gattatttta tgaatatatt tcattgtgca   480
   agtagataga aattacatat gttacataac acacgaaata aacaaaaaaa cacaatccaa   540
   aacaaacacc ccaaacaaaa taacactata tatatcctcg tatgaggaga ggcacgttca   600
   gtgactcgac gattcccgag caaaaaaagt ctccccgtca cacatatagt gggtgacgca   660
   attatcttca aagtaatcct tctgttgact tgtcattgat aacatccagt cttcgtcagg   720
   attccaaaga attatagaag ggatccctaa tacaaggttt tttatcaagc tggataagag      780
   catgatagtg ggtagtgcca tcttgatgaa gctcagaagc aacaccaagg aagaaaataa      840
   gaaaaggtgt gagtttctcc cagagaaact ggaataaatc atctctttga gatgagcact      900
   tggggtaggt aaggaaaaca tatttagatt ggagtctgaa gttcttgcta gcagaaggca      960
   tgttgttgtg actccgaggg gttgcctcaa actctatctt ataaccggcg tggaggcatg     1020
   gaggcaaggg cattttggta atttaagtag ttagtggaaa atgacgtcat ttacttaaag     1080
   acgaagtctt gcgacaaggg gggcccacgc cgaattttaa tattaccggc gtggccccac     1140
   cttatcgcga gtgctttagc acgagcggtc cagatttaaa gtagaaaagt tcccgcccac     1200
   tagggttaaa ggtgttcaca ctataaaagc atatacgatg tgatggtatt tgatggagcg     1260
   tatattgtat caggtatttc cgtcggatac gaattattcg tac                       1303
<210> 10
   <211> 5069
   <212> DNA
   <213> Artificial Sequence
<220>
   <223> Synthesized construct No. 853
<400> 10
   ggcgcgccct agcagaaggc atgttgttgt gactccgagg ggttgcctca aactctatct      60
   tataaccggc gtggaggcat ggaggcaagg gcattttggt aatttaagta gttagtggaa     120
   aatgacgtca tttacttaaa gacgaagtct tgcgacaagg ggggcccacg ccgaatttta     180
   atattaccgg cgtggcccca ccttatcgcg agtgctttag cacgagcggt ccagatttaa     240
   agtagaaaag ttcccgccca ctagggttaa aggtgttcac actataaaag catatacgat     300
   gtgatggtat ttgataaagc gtatattgta tcaggtattt ccgtcggata cgaattattc     360
   gtacaagctt cttaagcctg caggtcaaca tggtggagca cgacacactt gtctactcca     420
   aaaatatcaa agatacagtc tcagaagacc aaagggcaat tgagactttt caacaaaggg     480
   taatatccgg aaacctcctc ggattccatt gcccagctat ctgtcacttt attgtgaaga     540
   tagtggaaaa ggaaggtggc tcctacaaat gccatcattg cgataaagga aaggccatcg     600
   ttgaagatgc ctctgccgac agtggtccca aagatggacc cccacccacg aggagcatcg     660
   tggaaaaaga agacgttcca accacgtctt caaagcaagt ggattgatgt gataacatgg     720
   tggagcacga cacacttgtc tactccaaaa atatcaaaga tacagtctca gaagaccaaa     780
   gggcaattga gacttttcaa caaagggtaa tatccggaaa cctcctcgga ttccattgcc     840
   cagctatctg tcactttatt gtgaagatag tggaaaagga aggtggctcc tacaaatgcc     900
   atcattgcga taaaggaaag gccatcgttg aagatgcctc tgccgacagt ggtcccaaag     960
   atggaccccc acccacgagg agcatcgtgg aaaaagaaga cgttccaacc acgtcttcaa      1020
   agcaagtgga ttgatgtgat atctccactg acgtaaggga tgacgcacaa tcccactatc      1080
   cttcgcaaga cccttcctct atataaggaa gttcatttca tttggagagg tattaaaatc      1140
   ttaataggtt ttgataaaag cgaacgtggg gaaacccgaa ccaaaccttc ttctaaactc      1200
   tctctcatct ctcttaaagc aaacttctct cttgtctttc ttgcgtgagc gatcttcaac      1260
   gttgtcagat cgtgcttcgg caccagtaca acgttttctt tcactgaagc gaaatcaaag      1320
   atctctttgt ggacacgtag tgcggcgcca ttaaataacg tgtacttgtc ctattcttgt      1380
   cggtgtggtc ttgggaaaag aaagcttgct ggaggctgct gttcagcccc atacattact      1440
   tgttacgatt ctgctgactt tcggcgggtg caatatctct acttctgctt gacgaggtat      1500
   tgttgcctgt acttctttct tcttcttctt gctgattggt tctataagaa atctagtatt      1560
   ttctttgaaa cagagttttc ccgtggtttt cgaacttgga gaaagattgt taagcttctg      1620
   tatattctgc ccaaatttgt cgggcccatg gcgaaaaacg ttgcgatttt cggcttattg      1680
   ttttctcttc ttgtgttggt tccttctcag atcttcgctg atcgaatctg cactggaata      1740
   acatcttcaa actcacctca tgtggtcaaa acagccactc aaggggaggt caatgtgact      1800
   ggtgtgatac cactaacaac aacaccaaca aaatcttatt ttgcaaatct caaaggaaca      1860
   aggaccagag ggaaactatg cccagactgt ctcaactgca cagatctgga tgtggctttg      1920
   ggcagaccaa tgtgtgtggg gaccacacct tcggcgaagg cttcaatact ccacgaagtc      1980
   aaacctgtta catccgggtg ctttcctata atgcacgaca gaacaaaaat caggcaacta      2040
   cccaatcttc tcagaggata tgaaaatatc aggctatcaa cccaaaacgt catcgatgcg      2100
   gaaaaggcac caggaggacc ctacagactt ggaacctcag gatcttgccc taacgctacc      2160
   agtaagagcg gatttttcgc aacaatggct tgggctgtcc caaaggacaa caacaaaaat      2220
   gcaacgaacc cactaacagt agaagtacca tacatttgta cagaagggga agaccaaatc      2280
   actgtttggg ggttccattc agataacaaa acccaaatga agaacctcta tggagactca      2340
   aatcctcaaa agttcacctc atctgctaat ggagtaacca cacactatgt ttctcagatt      2400
   ggcagcttcc cagatcaaac agaagacgga ggactaccac aaagcggcag gattgttgtt      2460
   gattacatga tgcaaaaacc tgggaaaaca ggaacaattg tctaccaaag aggtgttttg      2520
   ttgcctcaaa aggtgtggtg cgcgagtggc aggagcaaag taataaaagg gtccttgcct      2580
   ttaattggtg aagcagattg ccttcatgaa aaatacggtg gattaaacaa aagcaagcct      2640
   tactacacag gagaacatgc aaaagccata ggaaattgcc caatatgggt gaaaacacct      2700
   ttgaagctcg ccaatggaac caaatataga cctcctgcaa aactattaaa ggaaaggggt      2760
   ttcttcggag ctattgctgg tttcctagaa ggaggatggg aaggaatgat tgcaggctgg      2820
   cacggataca catctcacgg agcacatgga gtggcagtgg cggcggacct taagagtacg 2880
   caagaagcta taaacaagat aacaaaaaat ctcaattctt tgagtgagct agaagtaaag 2940
   aatcttcaaa gactaagtgg tgccatggat gaactccaca acgaaatact cgagctggat 3000
   gagaaagtgg atgatctcag agctgacact ataagctcgc aaatagaact tgcagtcttg 3060
   ctttccaacg aaggaataat aaacagtgaa gatgagcatc tattggcact tgagagaaaa 3120
   ctaaagaaaa tgctgggtcc ctctgctgta gagataggaa atggatgctt cgaaaccaaa 3180
   cacaagtgca accagacctg cttagacagg atagctgctg gcacctttaa tgcaggagaa 3240
   ttttctctcc ccacttttga ttcactgaac attactgctg catctttaaa tgatgatgga 3300
   ttggataacc atactatact gctctattac tcaactgctg cttctagttt ggctgtaaca 3360
   ttgatgctag ctatttttat tgtttatatg gtctccagag acaacgtttc atgctccatc 3420
   tgtctataaa ggcctatttt ctttagtttg aatttactgt tattcggtgt gcatttctat 3480
   gtttggtgag cggttttctg tgctcagagt gtgtttattt tatgtaattt aatttctttg 3540
   tgagctcctg tttagcaggt cgtcccttca gcaaggacac aaaaagattt taattttatt 3600
   aaaaaaaaaa aaaaaaaaga ccgggaattc gatatcaagc ttatcgacct gcagatcgtt 3660
   caaacatttg gcaataaagt ttcttaagat tgaatcctgt tgccggtctt gcgatgatta 3720
   tcatataatt tctgttgaat tacgttaagc atgtaataat taacatgtaa tgcatgacgt 3780
   tatttatgag atgggttttt atgattagag tcccgcaatt atacatttaa tacgcgatag 3840
   aaaacaaaat atagcgcgca aactaggata aattatcgcg cgcggtgtca tctatgttac 3900
   tagatctcta gaggatcccc gggtaccgag ctcgaattcc gagtgtactt caagtcagtt 3960
   ggaaatcaat aaaatgatta ttttatgaat atatttcatt gtgcaagtag atagaaatta 4020
   catatgttac ataacacacg aaataaacaa aaaaacacaa tccaaaacaa acaccccaaa 4080
   caaaataaca ctatatatat cctcgtatga ggagaggcac gttcagtgac tcgacgattc 4140
   ccgagcaaaa aaagtctccc cgtcacacat atagtgggtg acgcaattat cttcaaagta 4200
   atccttctgt tgacttgtca ttgataacat ccagtcttcg tcaggattcc aaagaattat 4260
   agaagggatc cctaatacaa ggttttttat caagctggat aagagcatga tagtgggtag 4320
   tgccatcttg atgaagctca gaagcaacac caaggaagaa aataagaaaa ggtgtgagtt 4380
   tctcccagag aaactggaat aaatcatctc tttgagatga gcacttgggg taggtaagga 4440
   aaacatattt agattggagt ctgaagttct tgctagcaga aggcatgttg ttgtgactcc 4500
   gaggggttgc ctcaaactct atcttataac cggcgtggag gcatggaggc aagggcattt 4560
   tggtaattta agtagttagt ggaaaatgac gtcatttact taaagacgaa gtcttgcgac 4620
   aaggggggcc cacgccgaat tttaatatta ccggcgtggc cccaccttat cgcgagtgct     4680
   ttagcacgag cggtccagat ttaaagtaga aaagttcccg cccactaggg ttaaaggtgt     4740
   tcacactata aaagcatata cgatgtgatg gtatttgatg gagcgtatat tgtatcaggt     4800
   atttccgtcg gatacgaatt attcgtacgg ccggccacta gcgcgtcaat tgagcttggc     4860
   actggccgtc gttttacaac gtcgtgactg ggaaaaccct ggcgttaccc aacttaatcg     4920
   ccttgcagca catccccctt tcgccagctg gcgtaatagc gaagaggccc gcaccgatcg     4980
   cccttcccaa cagttgcgca gcctgaatgg cgaatgctag agcagcttga gcttggatca     5040
   gattgtcgtt tcccgccttc agtttaaac                                       5069
<210> 11
   <211> 5009
   <212> DNA
   <213> Synthesized construct number 561
<400> 11
   ggcgcgccct agcagaaggc atgttgttgt gactccgagg ggttgcctca aactctatct      60
   tataaccggc gtggaggcat ggaggcaagg gcattttggt aatttaagta gttagtggaa     120
   aatgacgtca tttacttaaa gacgaagtct tgcgacaagg ggggcccacg ccgaatttta     180
   atattaccgg cgtggcccca ccttatcgcg agtgctttag cacgagcggt ccagatttaa     240
   agtagaaaag ttcccgccca ctagggttaa aggtgttcac actataaaag catatacgat     300
   gtgatggtat ttgataaagc gtatattgta tcaggtattt ccgtcggata cgaattattc     360
   gtacaagctt cttaagcctg caggtcaaca tggtggagca cgacacactt gtctactcca     420
   aaaatatcaa agatacagtc tcagaagacc aaagggcaat tgagactttt caacaaaggg     480
   taatatccgg aaacctcctc ggattccatt gcccagctat ctgtcacttt attgtgaaga     540
   tagtggaaaa ggaaggtggc tcctacaaat gccatcattg cgataaagga aaggccatcg     600
   ttgaagatgc ctctgccgac agtggtccca aagatggacc cccacccacg aggagcatcg     660
   tggaaaaaga agacgttcca accacgtctt caaagcaagt ggattgatgt gataacatgg     720
   tggagcacga cacacttgtc tactccaaaa atatcaaaga tacagtctca gaagaccaaa     780
   gggcaattga gacttttcaa caaagggtaa tatccggaaa cctcctcgga ttccattgcc     840
   cagctatctg tcactttatt gtgaagatag tggaaaagga aggtggctcc tacaaatgcc     900
   atcattgcga taaaggaaag gccatcgttg aagatgcctc tgccgacagt ggtcccaaag     960
   atggaccccc acccacgagg agcatcgtgg aaaaagaaga cgttccaacc acgtcttcaa    1020
   agcaagtgga ttgatgtgat atctccactg acgtaaggga tgacgcacaa tcccactatc    1080
   cttcgcaaga cccttcctct atataaggaa gttcatttca tttggagagg tattaaaatc    1140
   ttaataggtt ttgataaaag cgaacgtggg gaaacccgaa ccaaaccttc ttctaaactc    1200
   tctctcatct ctcttaaagc aaacttctct cttgtctttc ttgcgtgagc gatcttcaac 1260
   gttgtcagat cgtgcttcgg caccagtaca acgttttctt tcactgaagc gaaatcaaag 1320
   atctctttgt ggacacgtag tgcggcgcca ttaaataacg tgtacttgtc ctattcttgt 1380
   cggtgtggtc ttgggaaaag aaagcttgct ggaggctgct gttcagcccc atacattact 1440
   tgttacgatt ctgctgactt tcggcgggtg caatatctct acttctgctt gacgaggtat 1500
   tgttgcctgt acttctttct tcttcttctt gctgattggt tctataagaa atctagtatt 1560
   ttctttgaaa cagagttttc ccgtggtttt cgaacttgga gaaagattgt taagcttctg 1620
   tatattctgc ccaaatttgt cgggcccatg gcgaaaaacg ttgcgatttt cggcttattg 1680
   ttttctcttc ttgtgttggt tccttctcag atcttcgctg acacattatg tataggttat 1740
   catgcgaaca attcaacaga cactgtagac acagtactag aaaagaatgt aacagtaaca 1800
   cactctgtta accttctaga agacaagcat aacgggaaac tatgcaaact aagaggggta 1860
   gccccattgc atttgggtaa atgtaacatt gctggctgga tcctgggaaa tccagagtgt 1920
   gaatcactct ccacagcaag ctcatggtcc tacattgtgg aaacacctag ttcagacaat 1980
   ggaacgtgtt acccaggaga tttcatcgat tatgaggagc taagagagca attaagctca 2040
   gtgtcatcat ttgaaaggtt tgagatattc cccaagacaa gttcatggcc caatcatgac 2100
   tcgaacaaag gtgtaacggc agcatgtcct catgctggag caaaaagctt ctacaaaaat 2160
   ttaatatggc tagttaaaaa aggaaattca tacccaaagc tcagcaaatc ctacattaat 2220
   gataaaggga aagaagtcct cgtgctatgg ggcattcacc atccatctac tagtgctgac 2280
   caacaaagtc tctatcagaa tgcagataca tatgtttttg tggggtcatc aagatacagc 2340
   aagaagttca agccggaaat agcaataaga cccaaagtga gggatcaaga agggagaatg 2400
   aactattact ggacactagt agagccggga gacaaaataa cattcgaagc aactggaaat 2460
   ctagtggtac cgagatatgc attcgcaatg gaaagaaatg ctggatctgg tattatcatt 2520
   tcagatacac cagtccacga ttgcaataca acttgtcaaa cacccaaggg tgctataaac 2580
   accagcctcc catttcagaa tatacatccg atcacaattg gaaaatgtcc aaaatatgta 2640
   aaaagcacaa aattgagact ggccacagga ttgaggaata tcccgtctat tcaatctaga 2700
   ggactatttg gggccattgc cggtttcatt gaaggggggt ggacagggat ggtagatgga 2760
   tggtacggtt atcaccatca aaatgagcag gggtcaggat atgcagccga cctgaagagc 2820
   acacagaatg ccattgacga gattactaac aaagtaaatt ctgttattga aaagatgaat 2880
   acacagttca cagcagtagg taaagagttc aaccacctgg aaaaaagaat agagaattta 2940
   aataaaaaag ttgatgatgg tttcctggac atttggactt acaatgccga actgttggtt 3000
   ctattggaaa atgaaagaac tttggactac cacgattcaa atgtgaagaa cttatatgaa 3060
   aaggtaagaa gccagctaaa aaacaatgcc aaggaaattg gaaacggctg ctttgaattt 3120
   taccacaaat gcgataacac gtgcatggaa agtgtcaaaa atgggactta tgactaccca 3180
   aaatactcag aggaagcaaa attaaacaga gaagaaatag atggggtaaa gctggaatca 3240
   acaaggattt accagatttt ggcgatctat tcaactgtcg ccagttcatt ggtactggta 3300
   gtctccctgg gggcaatcag tttctggatg tgctctaatg ggtctctaca gtgtagaata 3360
   tgtatttaaa ggcctatttt ctttagtttg aatttactgt tattcggtgt gcatttctat 3420
   gtttggtgag cggttttctg tgctcagagt gtgtttattt tatgtaattt aatttctttg 3480
   tgagctcctg tttagcaggt cgtcccttca gcaaggacac aaaaagattt taattttatt 3540
   aaaaaaaaaa aaaaaaaaga ccgggaattc gatatcaagc ttatcgacct gcagatcgtt 3600
   caaacatttg gcaataaagt ttcttaagat tgaatcctgt tgccggtctt gcgatgatta 3660
   tcatataatt tctgttgaat tacgttaagc atgtaataat taacatgtaa tgcatgacgt 3720
   tatttatgag atgggttttt atgattagag tcccgcaatt atacatttaa tacgcgatag 3780
   aaaacaaaat atagcgcgca aactaggata aattatcgcg cgcggtgtca tctatgttac 3840
   tagatctcta gaggatcccc gggtaccgag ctcgaattcc gagtgtactt caagtcagtt 3900
   ggaaatcaat aaaatgatta ttttatgaat atatttcatt gtgcaagtag atagaaatta 3960
   catatgttac ataacacacg aaataaacaa aaaaacacaa tccaaaacaa acaccccaaa 4020
   caaaataaca ctatatatat cctcgtatga ggagaggcac gttcagtgac tcgacgattc 4080
   ccgagcaaaa aaagtctccc cgtcacacat atagtgggtg acgcaattat cttcaaagta 4140
   atccttctgt tgacttgtca ttgataacat ccagtcttcg tcaggattcc aaagaattat 4200
   agaagggatc cctaatacaa ggttttttat caagctggat aagagcatga tagtgggtag 4260
   tgccatcttg atgaagctca gaagcaacac caaggaagaa aataagaaaa ggtgtgagtt 4320
   tctcccagag aaactggaat aaatcatctc tttgagatga gcacttgggg taggtaagga 4380
   aaacatattt agattggagt ctgaagttct tgctagcaga aggcatgttg ttgtgactcc 4440
   gaggggttgc ctcaaactct atcttataac cggcgtggag gcatggaggc aagggcattt 4500
   tggtaattta agtagttagt ggaaaatgac gtcatttact taaagacgaa gtcttgcgac 4560
   aaggggggcc cacgccgaat tttaatatta ccggcgtggc cccaccttat cgcgagtgct 4620
   ttagcacgag cggtccagat ttaaagtaga aaagttcccg cccactaggg ttaaaggtgt 4680
   tcacactata aaagcatata cgatgtgatg gtatttgatg gagcgtatat tgtatcaggt 4740
   atttccgtcg gatacgaatt attcgtacgg ccggccacta gcgcgtcaat tgagcttggc 4800
   actggccgtc gttttacaac gtcgtgactg ggaaaaccct ggcgttaccc aacttaatcg 4860
   ccttgcagca catccccctt tcgccagctg gcgtaatagc gaagaggccc gcaccgatcg 4920
   cccttcccaa cagttgcgca gcctgaatgg cgaatgctag agcagcttga gcttggatca 4980
   gattgtcgtt tcccgccttc agtttaaac 5009
<210> 12
   <211> 5012
   <212> DNA
   <213> Artificial Sequence
<220>
   <223> Synthesized construct number 851
<400> 12
   ggcgcgccct agcagaaggc atgttgttgt gactccgagg ggttgcctca aactctatct 60
   tataaccggc gtggaggcat ggaggcaagg gcattttggt aatttaagta gttagtggaa 120
   aatgacgtca tttacttaaa gacgaagtct tgcgacaagg ggggcccacg ccgaatttta 180
   atattaccgg cgtggcccca ccttatcgcg agtgctttag cacgagcggt ccagatttaa 240
   agtagaaaag ttcccgccca ctagggttaa aggtgttcac actataaaag catatacgat 300
   gtgatggtat ttgataaagc gtatattgta tcaggtattt ccgtcggata cgaattattc 360
   gtacaagctt cttaagcctg caggtcaaca tggtggagca cgacacactt gtctactcca 420
   aaaatatcaa agatacagtc tcagaagacc aaagggcaat tgagactttt caacaaaggg 480
   taatatccgg aaacctcctc ggattccatt gcccagctat ctgtcacttt attgtgaaga 540
   tagtggaaaa ggaaggtggc tcctacaaat gccatcattg cgataaagga aaggccatcg 600
   ttgaagatgc ctctgccgac agtggtccca aagatggacc cccacccacg aggagcatcg 660
   tggaaaaaga agacgttcca accacgtctt caaagcaagt ggattgatgt gataacatgg 720
   tggagcacga cacacttgtc tactccaaaa atatcaaaga tacagtctca gaagaccaaa 780
   gggcaattga gacttttcaa caaagggtaa tatccggaaa cctcctcgga ttccattgcc 840
   cagctatctg tcactttatt gtgaagatag tggaaaagga aggtggctcc tacaaatgcc 900
   atcattgcga taaaggaaag gccatcgttg aagatgcctc tgccgacagt ggtcccaaag 960
   atggaccccc acccacgagg agcatcgtgg aaaaagaaga cgttccaacc acgtcttcaa 1020
   agcaagtgga ttgatgtgat atctccactg acgtaaggga tgacgcacaa tcccactatc 1080
   cttcgcaaga cccttcctct atataaggaa gttcatttca tttggagagg tattaaaatc 1140
   ttaataggtt ttgataaaag cgaacgtggg gaaacccgaa ccaaaccttc ttctaaactc 1200
   tctctcatct ctcttaaagc aaacttctct cttgtctttc ttgcgtgagc gatcttcaac 1260
   gttgtcagat cgtgcttcgg caccagtaca acgttttctt tcactgaagc gaaatcaaag 1320
   atctctttgt ggacacgtag tgcggcgcca ttaaataacg tgtacttgtc ctattcttgt 1380
   cggtgtggtc ttgggaaaag aaagcttgct ggaggctgct gttcagcccc atacattact 1440
   tgttacgatt ctgctgactt tcggcgggtg caatatctct acttctgctt gacgaggtat 1500
   tgttgcctgt acttctttct tcttcttctt gctgattggt tctataagaa atctagtatt 1560
   ttctttgaaa cagagttttc ccgtggtttt cgaacttgga gaaagattgt taagcttctg 1620
   tatattctgc ccaaatttgt cgggcccatg gcgaaaaacg ttgcgatttt cggcttattg 1680
   ttttctcttc ttgtgttggt tccttctcag atcttcgctc aaaaacttcc cggaaatgac 1740
   aacagcacgg caacgctgtg ccttgggcac catgcagtac caaacggaac gatagtgaaa 1800
   acaatcacga atgaccaaat tgaagttact aatgctactg agctggttca gagttcctca 1860
   acaggtgaaa tatgcgacag tcctcatcag atccttgatg gagaaaactg cacactaata 1920
   gatgctctat tgggagaccc tcagtgtgat ggcttccaaa ataagaaatg ggaccttttt 1980
   gttgaacgca gcaaagccta cagcaactgt tacccttatg atgtgccgga ttatgcctcc 2040
   cttaggtcac tagttgcctc atccggcaca ctggagttta acaatgaaag tttcaattgg 2100
   actggagtca ctcaaaacgg aacaagctct gcttgcataa ggagatctaa taacagtttc 2160
   tttagtagat tgaattggtt gacccactta aaattcaaat acccagcatt gaacgtgact 2220
   atgccaaaca atgaaaaatt tgacaaattg tacatttggg gggttcacca cccgggtacg 2280
   gacaatgacc aaatcttcct gtatgctcaa gcatcaggaa gaatcacagt ctctaccaaa 2340
   agaagccaac aaactgtaat cccgaatatc ggatctagac ccagagtaag gaatatcccc 2400
   agcagaataa gcatctattg gacaatagta aaaccgggag acatactttt gattaacagc 2460
   acagggaatc taattgctcc taggggttac ttcaaaatac gaagtgggaa aagctcaata 2520
   atgagatcag atgcacccat tggcaaatgc aattctgaat gcatcactcc aaacggaagc 2580
   attcccaatg acaaaccatt ccaaaatgta aacaggatca catacggggc ctgtcccaga 2640
   tatgttaagc aaaacactct gaaattggca acagggatgc gaaatgtacc agagaaacaa 2700
   actagaggca tatttggcgc aatcgcgggt ttcatagaaa atggttggga gggaatggtg 2760
   gatggttggt atggtttcag gcatcaaaat tctgagggaa taggacaagc agcagatctc 2820
   aaaagcactc aagcagcaat cgatcaaatc aatgggaagc tgaataggtt gatcgggaaa 2880
   accaacgaga aattccatca gattgaaaaa gagttctcag aagtcgaagg gagaatccag 2940
   gaccttgaga aatatgttga ggacaccaaa atagatctct ggtcatacaa cgcggagctt 3000
   cttgttgccc tggagaacca acatacaatt gatctaactg actcagaaat gaacaaactg 3060
   tttgaaaaaa caaagaagca actgagggaa aatgctgagg atatgggcaa tggttgtttc 3120
   aaaatatacc acaaatgtga caatgcctgc ataggatcaa tcagaaatgg aacttatgac 3180
   cacgatgtat acagagatga agcattaaac aaccggttcc agatcaaggg cgttgagctg 3240
   aagtcaggat acaaagattg gatactatgg atttcctttg ccatatcatg ttttttgctt 3300
   tgtgttgctt tgttggggtt catcatgtgg gcctgccaaa aaggcaacat taggtgcaac 3360
   atttgcattt gaaggcctat tttctttagt ttgaatttac tgttattcgg tgtgcatttc 3420
   tatgtttggt gagcggtttt ctgtgctcag agtgtgttta ttttatgtaa tttaatttct 3480
   ttgtgagctc ctgtttagca ggtcgtccct tcagcaagga cacaaaaaga ttttaatttt 3540
   attaaaaaaa aaaaaaaaaa agaccgggaa ttcgatatca agcttatcga cctgcagatc 3600
   gttcaaacat ttggcaataa agtttcttaa gattgaatcc tgttgccggt cttgcgatga 3660
   ttatcatata atttctgttg aattacgtta agcatgtaat aattaacatg taatgcatga 3720
   cgttatttat gagatgggtt tttatgatta gagtcccgca attatacatt taatacgcga 3780
   tagaaaacaa aatatagcgc gcaaactagg ataaattatc gcgcgcggtg tcatctatgt 3840
   tactagatct ctagaggatc cccgggtacc gagctcgaat tccgagtgta cttcaagtca 3900
   gttggaaatc aataaaatga ttattttatg aatatatttc attgtgcaag tagatagaaa 3960
   ttacatatgt tacataacac acgaaataaa caaaaaaaca caatccaaaa caaacacccc 4020
   aaacaaaata acactatata tatcctcgta tgaggagagg cacgttcagt gactcgacga 4080
   ttcccgagca aaaaaagtct ccccgtcaca catatagtgg gtgacgcaat tatcttcaaa 4140
   gtaatccttc tgttgacttg tcattgataa catccagtct tcgtcaggat tccaaagaat 4200
   tatagaaggg atccctaata caaggttttt tatcaagctg gataagagca tgatagtggg 4260
   tagtgccatc ttgatgaagc tcagaagcaa caccaaggaa gaaaataaga aaaggtgtga 4320
   gtttctccca gagaaactgg aataaatcat ctctttgaga tgagcacttg gggtaggtaa 4380
   ggaaaacata tttagattgg agtctgaagt tcttgctagc agaaggcatg ttgttgtgac 4440
   tccgaggggt tgcctcaaac tctatcttat aaccggcgtg gaggcatgga ggcaagggca 4500
   ttttggtaat ttaagtagtt agtggaaaat gacgtcattt acttaaagac gaagtcttgc 4560
   gacaaggggg gcccacgccg aattttaata ttaccggcgt ggccccacct tatcgcgagt 4620
   gctttagcac gagcggtcca gatttaaagt agaaaagttc ccgcccacta gggttaaagg 4680
   tgttcacact ataaaagcat atacgatgtg atggtatttg atggagcgta tattgtatca 4740
   ggtatttccg tcggatacga attattcgta cggccggcca ctagcgcgtc aattgagctt 4800
   ggcactggcc gtcgttttac aacgtcgtga ctgggaaaac cctggcgtta cccaacttaa 4860
   tcgccttgca gcacatcccc ctttcgccag ctggcgtaat agcgaagagg cccgcaccga 4920
   tcgcccttcc caacagttgc gcagcctgaa tggcgaatgc tagagcagct tgagcttgga 4980
   tcagattgtc gtttcccgcc ttcagtttaa ac 5012
<210> 13
   <211> 4994
   <212> DNA
   <213> Artificial Sequence
<220>
   <223> Synthesized construct No. 852
<400> 13
   ggcgcgccct agcagaaggc atgttgttgt gactccgagg ggttgcctca aactctatct 60
   tataaccggc gtggaggcat ggaggcaagg gcattttggt aatttaagta gttagtggaa 120
   aatgacgtca tttacttaaa gacgaagtct tgcgacaagg ggggcccacg ccgaatttta 180
   atattaccgg cgtggcccca ccttatcgcg agtgctttag cacgagcggt ccagatttaa 240
   agtagaaaag ttcccgccca ctagggttaa aggtgttcac actataaaag catatacgat 300
   gtgatggtat ttgataaagc gtatattgta tcaggtattt ccgtcggata cgaattattc 360
   gtacaagctt cttaagcctg caggtcaaca tggtggagca cgacacactt gtctactcca 420
   aaaatatcaa agatacagtc tcagaagacc aaagggcaat tgagactttt caacaaaggg 480
   taatatccgg aaacctcctc ggattccatt gcccagctat ctgtcacttt attgtgaaga 540
   tagtggaaaa ggaaggtggc tcctacaaat gccatcattg cgataaagga aaggccatcg 600
   ttgaagatgc ctctgccgac agtggtccca aagatggacc cccacccacg aggagcatcg 660
   tggaaaaaga agacgttcca accacgtctt caaagcaagt ggattgatgt gataacatgg 720
   tggagcacga cacacttgtc tactccaaaa atatcaaaga tacagtctca gaagaccaaa 780
   gggcaattga gacttttcaa caaagggtaa tatccggaaa cctcctcgga ttccattgcc 840
   cagctatctg tcactttatt gtgaagatag tggaaaagga aggtggctcc tacaaatgcc 900
   atcattgcga taaaggaaag gccatcgttg aagatgcctc tgccgacagt ggtcccaaag 960
   atggaccccc acccacgagg agcatcgtgg aaaaagaaga cgttccaacc acgtcttcaa 1020
   agcaagtgga ttgatgtgat atctccactg acgtaaggga tgacgcacaa tcccactatc 1080
   cttcgcaaga cccttcctct atataaggaa gttcatttca tttggagagg tattaaaatc 1140
   ttaataggtt ttgataaaag cgaacgtggg gaaacccgaa ccaaaccttc ttctaaactc 1200
   tctctcatct ctcttaaagc aaacttctct cttgtctttc ttgcgtgagc gatcttcaac 1260
   gttgtcagat cgtgcttcgg caccagtaca acgttttctt tcactgaagc gaaatcaaag 1320
   atctctttgt ggacacgtag tgcggcgcca ttaaataacg tgtacttgtc ctattcttgt 1380
   cggtgtggtc ttgggaaaag aaagcttgct ggaggctgct gttcagcccc atacattact 1440
   tgttacgatt ctgctgactt tcggcgggtg caatatctct acttctgctt gacgaggtat 1500
   tgttgcctgt acttctttct tcttcttctt gctgattggt tctataagaa atctagtatt 1560
   ttctttgaaa cagagttttc ccgtggtttt cgaacttgga gaaagattgt taagcttctg 1620
   tatattctgc ccaaatttgt cgggcccatg gagaaaatag tgcttcttct tgcaatagtc 1680
   agtcttgtta aaagtgatca gatttgcatt ggttaccatg caaacaattc aacagagcag 1740
   gttgacacaa tcatggaaaa gaacgttact gttacacatg cccaagacat actggaaaag 1800
   acacacaacg ggaagctctg cgatctagat ggagtgaagc ctctaatttt aagagattgt 1860
   agtgtagctg gatggctcct cgggaaccca atgtgtgacg aattcatcaa tgtaccggaa 1920
   tggtcttaca tagtggagaa ggccaatcca accaatgacc tctgttaccc agggagtttc 1980
   aacgactatg aagaactgaa acacctattg agcagaataa accattttga gaaaattcaa 2040
   atcatcccca aaagttcttg gtccgatcat gaagcctcat caggagttag ctcagcatgt 2100
   ccatacctgg gaagtccctc cttttttaga aatgtggtat ggcttatcaa aaagaacagt 2160
   acatacccaa caataaagaa aagctacaat aataccaacc aagaggatct tttggtactg 2220
   tggggaattc accatcctaa tgatgcggca gagcagacaa ggctatatca aaacccaacc 2280
   acctatattt ccattgggac atcaacacta aaccagagat tggtaccaaa aatagctact 2340
   agatccaaag taaacgggca aagtggaagg atggagttct tctggacaat tttaaaacct 2400
   aatgatgcaa tcaacttcga gagtaatgga aatttcattg ctccagaata tgcatacaaa 2460
   attgtcaaga aaggggactc agcaattatg aaaagtgaat tggaatatgg taactgcaac 2520
   accaagtgtc aaactccaat gggggcgata aactctagta tgccattcca caacatacac 2580
   cctctcacca tcggggaatg ccccaaatat gtgaaatcaa acagattagt ccttgcaaca 2640
   gggctcagaa atagccctca aagagagagc agaagaaaaa agagaggact atttggagct 2700
   atagcaggtt ttatagaggg aggatggcag ggaatggtag atggttggta tgggtaccac 2760
   catagcaatg agcaggggag tgggtacgct gcagacaaag aatccactca aaaggcaata 2820
   gatggagtca ccaataaggt caactcaatc attgacaaaa tgaacactca gtttgaggcc 2880
   gttggaaggg aatttaataa cttagaaagg agaatagaga atttaaacaa gaagatggaa 2940
   gacgggtttc tagatgtctg gacttataat gccgaacttc tggttctcat ggaaaatgag 3000
   agaactctag actttcatga ctcaaatgtt aagaacctct acgacaaggt ccgactacag 3060
   cttagggata atgcaaagga gctgggtaac ggttgtttcg agttctatca caaatgtgat 3120
   aatgaatgta tggaaagtat aagaaacgga acgtacaact atccgcagta ttcagaagaa 3180
   gcaagattaa aaagagagga aataagtggg gtaaaattgg aatcaatagg aacttaccaa 3240
   atactgtcaa tttattcaac agtggcgagt tccctagcac tggcaatcat gatggctggt 3300
   ctatctttat ggatgtgctc caatggatcg ttacaatgca gaatttgcat ttaaaggcct 3360
   attttcttta gtttgaattt actgttattc ggtgtgcatt tctatgtttg gtgagcggtt    3420
   ttctgtgctc agagtgtgtt tattttatgt aatttaattt ctttgtgagc tcctgtttag    3480
   caggtcgtcc cttcagcaag gacacaaaaa gattttaatt ttattaaaaa aaaaaaaaaa    3540
   aaagaccggg aattcgatat caagcttatc gacctgcaga tcgttcaaac atttggcaat    3600
   aaagtttctt aagattgaat cctgttgccg gtcttgcgat gattatcata taatttctgt    3660
   tgaattacgt taagcatgta ataattaaca tgtaatgcat gacgttattt atgagatggg    3720
   tttttatgat tagagtcccg caattataca tttaatacgc gatagaaaac aaaatatagc    3780
   gcgcaaacta ggataaatta tcgcgcgcgg tgtcatctat gttactagat ctctagagga    3840
   tccccgggta ccgagctcga attccgagtg tacttcaagt cagttggaaa tcaataaaat    3900
   gattatttta tgaatatatt tcattgtgca agtagataga aattacatat gttacataac    3960
   acacgaaata aacaaaaaaa cacaatccaa aacaaacacc ccaaacaaaa taacactata    4020
   tatatcctcg tatgaggaga ggcacgttca gtgactcgac gattcccgag caaaaaaagt    4080
   ctccccgtca cacatatagt gggtgacgca attatcttca aagtaatcct tctgttgact    4140
   tgtcattgat aacatccagt cttcgtcagg attccaaaga attatagaag ggatccctaa    4200
   tacaaggttt tttatcaagc tggataagag catgatagtg ggtagtgcca tcttgatgaa    4260
   gctcagaagc aacaccaagg aagaaaataa gaaaaggtgt gagtttctcc cagagaaact    4320
   ggaataaatc atctctttga gatgagcact tggggtaggt aaggaaaaca tatttagatt    4380
   ggagtctgaa gttcttgcta gcagaaggca tgttgttgtg actccgaggg gttgcctcaa    4440
   actctatctt ataaccggcg tggaggcatg gaggcaaggg cattttggta atttaagtag    4500
   ttagtggaaa atgacgtcat ttacttaaag acgaagtctt gcgacaaggg gggcccacgc    4560
   cgaattttaa tattaccggc gtggccccac cttatcgcga gtgctttagc acgagcggtc    4620
   cagatttaaa gtagaaaagt tcccgcccac tagggttaaa ggtgttcaca ctataaaagc    4680
   atatacgatg tgatggtatt tgatggagcg tatattgtat caggtatttc cgtcggatac    4740
   gaattattcg tacggccggc cactagcgcg tcaattgagc ttggcactgg ccgtcgtttt    4800
   acaacgtcgt gactgggaaa accctggcgt tacccaactt aatcgccttg cagcacatcc    4860
   ccctttcgcc agctggcgta atagcgaaga ggcccgcacc gatcgccctt cccaacagtt    4920
   gcgcagcctg aatggcgaat gctagagcag cttgagcttg gatcagattg tcgtttcccg    4980
   ccttcagttt aaac                                                      4994
<210> 14
   <211> 2030
   <212> DNA
   <213> Artificial Sequence
<220>
   <223> Synthesized construct number 848
<400> 14
   ctagcagaag gcatgttgtt gtgactccga ggggttgcct caaactctat cttataaccg     60
   gcgtggaggc atggaggcaa gggcattttg gtaatttaag tagttagtgg aaaatgacgt    120
   catttactta aagacgaagt cttgcgacaa gggggcccca cgccgaattt taatattacc    180
   ggcgtggccc caccttatcg cgagtgcttt agcacgagcg gtccagattt aaagtagaaa    240
   agttcccgcc cactagggtt aaaggtgttc acactataaa agcatatacg atgtgatggt    300
   atttgataaa gcgtatattg tatcaggtat ttccgtcgga tacgaattat tcgtacaagc    360
   ttcttaagcc tgcaggtcga ctctagagga tccccgggta ccgagctcga attccgagtg    420
   tacttcaagt cagttggaaa tcaataaaat gattatttta tgaatatatt tcattgtgca    480
   agtagataga aattacatat gttacataac acacgaaata aacaaaaaaa cacaatccaa    540
   aacaaacacc ccaaacaaaa taacactata tatatcctcg tatgaggaga ggcacgttca    600
   gtgactcgac gattcccgag caaaaaaagt ctccccgtca cacatatagt gggtgacgca    660
   attatcttca aagtaatcct tctgttgact tgtcattgat aacatccagt cttcgtcagg    720
   attgcaaaga attatagaag ggatcccacc ttttattttc ttcttttttc catatttagg    780
   gttgacagtg aaatcagact ggcaacctat taattgcttc cacaatggga cgaacttgaa    840
   ggggatgtcg tcgatgatat tataggtggc gtgttcatcg tagttggtga agtcgatggt    900
   cccgttccag tagttgtgtc gcccgagact tctagcccag gtggtctttc cggtacgagt    960
   tggtccgcag atgtagaggc tggggtgtct gaccccagtc cttccctcat cctggttaga   1020
   tcggccatcc actcaaggtc agattgtgct tgatcgtagg agacaggatg tatgaaagtg   1080
   taggcatcga tgcttacatg atataggtgc gtctctctcc agttgtgcag atcttcgtgg   1140
   cagcggagat ctgattctgt gaagggcgac acgtactgct caggttgtgg aggaaataat   1200
   ttgttggctg aatattccag ccattgaagc tttgttgccc attcatgagg gaattcttct   1260
   ttgatcatgt caagatactc ctccttagac gttgcagtct ggataatagt tcgccatcgt   1320
   gcgtcagatt tgcgaggaga gaccttatga tctcggaaat ctcctctggt tttaatatct   1380
   ccgtcctttg atatgtaatc aaggacttgt ttagagtttc tagctggctg gatattaggg   1440
   tgatttcctt caaaatcgaa aaaagaagga tccctaatac aaggtttttt atcaagctgg   1500
   ataagagcat gatagtgggt agtgccatct tgatgaagct cagaagcaac accaaggaag   1560
   aaaataagaa aaggtgtgag tttctcccag agaaactgga ataaatcatc tctttgagat   1620
   gagcacttgg ggtaggtaag gaaaacatat ttagattgga gtctgaagtt cttgctagca   1680
   gaaggcatgt tgttgtgact ccgaggggtt gcctcaaact ctatcttata accggcgtgg   1740
   aggcatggag gcaagggcat tttggtaatt taagtagtta gtggaaaatg acgtcattta      1800
   cttaaagacg aagtcttgcg acaagggggg cccacgccga attttaatat taccggcgtg      1860
   gccccacctt atcgcgagtg ctttagcacg agcggtccag atttaaagta gaaaagttcc      1920
   cgcccactag ggttaaaggt gttcacacta taaaagcata tacgatgtga tggtatttga      1980
   tggagcgtat attgtatcag gtatttccgt cggatacgaa ttattcgtac                 2030
<210> 15
   <211> 5800
   <212> DNA
   <213> Artificial Sequence
<220>
   <223> Synthesized construct number 475
<400> 15
   ggcgcgccct agcagaaggc atgttgttgt gactccgagg ggttgcctca aactctatct      60
   tataaccggc gtggaggcat ggaggcaagg gcattttggt aatttaagta gttagtggaa     120
   aatgacgtca tttacttaaa gacgaagtct tgcgacaagg ggggcccacg ccgaatttta     180
   atattaccgg cgtggcccca ccttatcgcg agtgctttag cacgagcggt ccagatttaa     240
   agtagaaaag ttcccgccca ctagggttaa aggtgttcac actataaaag catatacgat     300
   gtgatggtat ttgataaagc gtatattgta tcaggtattt ccgtcggata cgaattattc     360
   gtacaagctt cttaagcctg caggtcaaca tggtggagca cgacacactt gtctactcca     420
   aaaatatcaa agatacagtc tcagaagacc aaagggcaat tgagactttt caacaaaggg     480
   taatatccgg aaacctcctc ggattccatt gcccagctat ctgtcacttt attgtgaaga     540
   tagtggaaaa ggaaggtggc tcctacaaat gccatcattg cgataaagga aaggccatcg     600
   ttgaagatgc ctctgccgac agtggtccca aagatggacc cccacccacg aggagcatcg     660
   tggaaaaaga agacgttcca accacgtctt caaagcaagt ggattgatgt gataacatgg     720
   tggagcacga cacacttgtc tactccaaaa atatcaaaga tacagtctca gaagaccaaa     780
   gggcaattga gacttttcaa caaagggtaa tatccggaaa cctcctcgga ttccattgcc     840
   cagctatctg tcactttatt gtgaagatag tggaaaagga aggtggctcc tacaaatgcc     900
   atcattgcga taaaggaaag gccatcgttg aagatgcctc tgccgacagt ggtcccaaag     960
   atggaccccc acccacgagg agcatcgtgg aaaaagaaga cgttccaacc acgtcttcaa    1020
   agcaagtgga ttgatgtgat atctccactg acgtaaggga tgacgcacaa tcccactatc    1080
   cttcgcaaga cccttcctct atataaggaa gttcatttca tttggagagg tattaaaatc    1140
   ttaataggtt ttgataaaag cgaacgtggg gaaacccgaa ccaaaccttc ttctaaactc    1200
   tctctcatct ctcttaaagc aaacttctct cttgtctttc ttgcgtgagc gatcttcaac    1260
   gttgtcagat cgtgcttcgg caccagtaca acgttttctt tcactgaagc gaaatcaaag    1320
   atctctttgt ggacacgtag tgcggcgcca ttaaataacg tgtacttgtc ctattcttgt    1380
   cggtgtggtc ttgggaaaag aaagcttgct ggaggctgct gttcagcccc atacattact    1440
   tgttacgatt ctgctgactt tcggcgggtg caatatctct acttctgctt gacgaggtat    1500
   tgttgcctgt acttctttct tcttcttctt gctgattggt tctataagaa atctagtatt    1560
   ttctttgaaa cagagttttc ccgtggtttt cgaacttgga gaaagattgt taagcttctg    1620
   tatattctgc ccaaatttgt cgggcccatg gcgaaaaacg ttgcgatttt cggcttattg    1680
   ttttctcttc ttgtgttggt tccttctcag atcttcgctg atcgaatctg cactggaata    1740
   acatcttcaa actcacctca tgtggtcaaa acagccactc aaggggaggt caatgtgact    1800
   ggtgtgatac cactaacaac aacaccaaca aaatcttatt ttgcaaatct caaaggaaca    1860
   aggaccagag ggaaactatg cccagactgt ctcaactgca cagatctgga tgtggctttg    1920
   ggcagaccaa tgtgtgtggg gaccacacct tcggcgaagg cttcaatact ccacgaagtc    1980
   aaacctgtta catccgggtg ctttcctata atgcacgaca gaacaaaaat caggcaacta    2040
   cccaatcttc tcagaggata tgaaaatatc aggctatcaa cccaaaacgt catcgatgcg    2100
   gaaaaggcac caggaggacc ctacagactt ggaacctcag gatcttgccc taacgctacc    2160
   agtaagagcg gatttttcgc aacaatggct tgggctgtcc caaaggacaa caacaaaaat    2220
   gcaacgaacc cactaacagt agaagtacca tacatttgta cagaagggga agaccaaatc    2280
   actgtttggg ggttccattc agataacaaa acccaaatga agaacctcta tggagactca    2340
   aatcctcaaa agttcacctc atctgctaat ggagtaacca cacactatgt ttctcagatt    2400
   ggcagcttcc cagatcaaac agaagacgga ggactaccac aaagcggcag gattgttgtt    2460
   gattacatga tgcaaaaacc tgggaaaaca ggaacaattg tctaccaaag aggtgttttg    2520
   ttgcctcaaa aggtgtggtg cgcgagtggc aggagcaaag taataaaagg gtccttgcct    2580
   ttaattggtg aagcagattg ccttcatgaa aaatacggtg gattaaacaa aagcaagcct    2640
   tactacacag gagaacatgc aaaagccata ggaaattgcc caatatgggt gaaaacacct    2700
   ttgaagctcg ccaatggaac caaatataga cctcctgcaa aactattaaa ggaaaggggt    2760
   ttcttcggag ctattgctgg tttcctagaa ggaggatggg aaggaatgat tgcaggctgg    2820
   cacggataca catctcacgg agcacatgga gtggcagtgg cggcggacct taagagtacg    2880
   caagaagcta taaacaagat aacaaaaaat ctcaattctt tgagtgagct agaagtaaag    2940
   aatcttcaaa gactaagtgg tgccatggat gaactccaca acgaaatact cgagctggat    3000
   gagaaagtgg atgatctcag agctgacact ataagctcgc aaatagaact tgcagtcttg    3060
   ctttccaacg aaggaataat aaacagtgaa gatgagcatc tattggcact tgagagaaaa 3120
   ctaaagaaaa tgctgggtcc ctctgctgta gagataggaa atggatgctt cgaaaccaaa 3180
   cacaagtgca accagacctg cttagacagg atagctgctg gcacctttaa tgcaggagaa 3240
   ttttctctcc ccacttttga ttcactgaac attactgctg catctttaaa tgatgatgga 3300
   ttggataacc atactatact gctctattac tcaactgctg cttctagttt ggctgtaaca 3360
   ttgatgctag ctatttttat tgtttatatg gtctccagag acaacgtttc atgctccatc 3420
   tgtctataaa ggcctatttt ctttagtttg aatttactgt tattcggtgt gcatttctat 3480
   gtttggtgag cggttttctg tgctcagagt gtgtttattt tatgtaattt aatttctttg 3540
   tgagctcctg tttagcaggt cgtcccttca gcaaggacac aaaaagattt taattttatt 3600
   aaaaaaaaaa aaaaaaaaga ccgggaattc gatatcaagc ttatcgacct gcagatcgtt 3660
   caaacatttg gcaataaagt ttcttaagat tgaatcctgt tgccggtctt gcgatgatta 3720
   tcatataatt tctgttgaat tacgttaagc atgtaataat taacatgtaa tgcatgacgt 3780
   tatttatgag atgggttttt atgattagag tcccgcaatt atacatttaa tacgcgatag 3840
   aaaacaaaat atagcgcgca aactaggata aattatcgcg cgcggtgtca tctatgttac 3900
   tagatctcta gagtctcaag cttggcgcgg ggtaccgagc tcgaattccg agtgtacttc 3960
   aagtcagttg gaaatcaata aaatgattat tttatgaata tatttcattg tgcaagtaga 4020
   tagaaattac atatgttaca taacacacga aataaacaaa aaaacacaat ccaaaacaaa 4080
   caccccaaac aaaataacac tatatatatc ctcgtatgag gagaggcacg ttcagtgact 4140
   cgacgattcc cgagcaaaaa aagtctcccc gtcacacata tagtgggtga cgcaattatc 4200
   ttcaaagtaa tccttctgtt gacttgtcat tgataacatc cagtcttcgt caggattgca 4260
   aagaattata gaagggatcc caccttttat tttcttcttt tttccatatt tagggttgac 4320
   agtgaaatca gactggcaac ctattaattg cttccacaat gggacgaact tgaaggggat 4380
   gtcgtcgatg atattatagg tggcgtgttc atcgtagttg gtgaagtcga tggtcccgtt 4440
   ccagtagttg tgtcgcccga gacttctagc ccaggtggtc tttccggtac gagttggtcc 4500
   gcagatgtag aggctggggt gtctgacccc agtccttccc tcatcctggt tagatcggcc 4560
   atccactcaa ggtcagattg tgcttgatcg taggagacag gatgtatgaa agtgtaggca 4620
   tcgatgctta catgatatag gtgcgtctct ctccagttgt gcagatcttc gtggcagcgg 4680
   agatctgatt ctgtgaaggg cgacacgtac tgctcaggtt gtggaggaaa taatttgttg 4740
   gctgaatatt ccagccattg aagctttgtt gcccattcat gagggaattc ttctttgatc 4800
   atgtcaagat actcctcctt agacgttgca gtctggataa tagttcgcca tcgtgcgtca 4860
   gatttgcgag gagagacctt atgatctcgg aaatctcctc tggttttaat atctccgtcc 4920
   tttgatatgt aatcaaggac ttgtttagag tttctagctg gctggatatt agggtgattt     4980
   ccttcaaaat cgaaaaaaga aggatcccta atacaaggtt ttttatcaag ctggataaga     5040
   gcatgatagt gggtagtgcc atcttgatga agctcagaag caacaccaag gaagaaaata     5100
   agaaaaggtg tgagtttctc ccagagaaac tggaataaat catctctttg agatgagcac     5160
   ttggggtagg taaggaaaac atatttagat tggagtctga agttcttgct agcagaaggc     5220
   atgttgttgt gactccgagg ggttgcctca aactctatct tataaccggc gtggaggcat     5280
   ggaggcaagg gcattttggt aatttaagta gttagtggaa aatgacgtca tttacttaaa     5340
   gacgaagtct tgcgacaagg ggggcccacg ccgaatttta atattaccgg cgtggcccca     5400
   ccttatcgcg agtgctttag cacgagcggt ccagatttaa agtagaaaag ttcccgccca     5460
   ctagggttaa aggtgttcac actataaaag catatacgat gtgatggtat ttgatggagc     5520
   gtatattgta tcaggtattt ccgtcggata cgaattattc gtacggccgg ccactagtaa     5580
   ttgagcttgg cactggccgt cgttttacaa cgtcgtgact gggaaaaccc tggcgttacc     5640
   caacttaatc gccttgcagc acatccccct ttcgccagct ggcgtaatag cgaagaggcc     5700
   cgcaccgatc gcccttccca acagttgcgc agcctgaatg gcgaatgcta gagcagcttg     5760
   agcttggatc agattgtcgt ttcccgcctt cagtttaaac                           5800
<210> 16
   <211> 5740
   <212> DNA
   <213> Artificial Sequence
<220>
   <223> Synthesized construct number 471
<400> 16
   ggcgcgccct agcagaaggc atgttgttgt gactccgagg ggttgcctca aactctatct        60
   tataaccggc gtggaggcat ggaggcaagg gcattttggt aatttaagta gttagtggaa       120
   aatgacgtca tttacttaaa gacgaagtct tgcgacaagg ggggcccacg ccgaatttta       180
   atattaccgg cgtggcccca ccttatcgcg agtgctttag cacgagcggt ccagatttaa       240
   agtagaaaag ttcccgccca ctagggttaa aggtgttcac actataaaag catatacgat       300
   gtgatggtat ttgataaagc gtatattgta tcaggtattt ccgtcggata cgaattattc       360
   gtacaagctt cttaagcctg caggtcaaca tggtggagca cgacacactt gtctactcca       420
   aaaatatcaa agatacagtc tcagaagacc aaagggcaat tgagactttt caacaaaggg       480
   taatatccgg aaacctcctc ggattccatt gcccagctat ctgtcacttt attgtgaaga       540
   tagtggaaaa ggaaggtggc tcctacaaat gccatcattg cgataaagga aaggccatcg       600
   ttgaagatgc ctctgccgac agtggtccca aagatggacc cccacccacg aggagcatcg       660
   tggaaaaaga agacgttcca accacgtctt caaagcaagt ggattgatgt gataacatgg      720
   tggagcacga cacacttgtc tactccaaaa atatcaaaga tacagtctca gaagaccaaa      780
   gggcaattga gacttttcaa caaagggtaa tatccggaaa cctcctcgga ttccattgcc      840
   cagctatctg tcactttatt gtgaagatag tggaaaagga aggtggctcc tacaaatgcc      900
   atcattgcga taaaggaaag gccatcgttg aagatgcctc tgccgacagt ggtcccaaag      960
   atggaccccc acccacgagg agcatcgtgg aaaaagaaga cgttccaacc acgtcttcaa     1020
   agcaagtgga ttgatgtgat atctccactg acgtaaggga tgacgcacaa tcccactatc     1080
   cttcgcaaga cccttcctct atataaggaa gttcatttca tttggagagg tattaaaatc     1140
   ttaataggtt ttgataaaag cgaacgtggg gaaacccgaa ccaaaccttc ttctaaactc     1200
   tctctcatct ctcttaaagc aaacttctct cttgtctttc ttgcgtgagc gatcttcaac     1260
   gttgtcagat cgtgcttcgg caccagtaca acgttttctt tcactgaagc gaaatcaaag     1320
   atctctttgt ggacacgtag tgcggcgcca ttaaataacg tgtacttgtc ctattcttgt     1380
   cggtgtggtc ttgggaaaag aaagcttgct ggaggctgct gttcagcccc atacattact     1440
   tgttacgatt ctgctgactt tcggcgggtg caatatctct acttctgctt gacgaggtat     1500
   tgttgcctgt acttctttct tcttcttctt gctgattggt tctataagaa atctagtatt     1560
   ttctttgaaa cagagttttc ccgtggtttt cgaacttgga gaaagattgt taagcttctg     1620
   tatattctgc ccaaatttgt cgggcccatg gcgaaaaacg ttgcgatttt cggcttattg     1680
   ttttctcttc ttgtgttggt tccttctcag atcttcgctg acacattatg tataggttat     1740
   catgcgaaca attcaacaga cactgtagac acagtactag aaaagaatgt aacagtaaca     1800
   cactctgtta accttctaga agacaagcat aacgggaaac tatgcaaact aagaggggta     1860
   gccccattgc atttgggtaa atgtaacatt gctggctgga tcctgggaaa tccagagtgt     1920
   gaatcactct ccacagcaag ctcatggtcc tacattgtgg aaacacctag ttcagacaat     1980
   ggaacgtgtt acccaggaga tttcatcgat tatgaggagc taagagagca attaagctca     2040
   gtgtcatcat ttgaaaggtt tgagatattc cccaagacaa gttcatggcc caatcatgac     2100
   tcgaacaaag gtgtaacggc agcatgtcct catgctggag caaaaagctt ctacaaaaat     2160
   ttaatatggc tagttaaaaa aggaaattca tacccaaagc tcagcaaatc ctacattaat     2220
   gataaaggga aagaagtcct cgtgctatgg ggcattcacc atccatctac tagtgctgac     2280
   caacaaagtc tctatcagaa tgcagataca tatgtttttg tggggtcatc aagatacagc     2340
   aagaagttca agccggaaat agcaataaga cccaaagtga gggatcaaga agggagaatg     2400
   aactattact ggacactagt agagccggga gacaaaataa cattcgaagc aactggaaat     2460
   ctagtggtac cgagatatgc attcgcaatg gaaagaaatg ctggatctgg tattatcatt 2520
   tcagatacac cagtccacga ttgcaataca acttgtcaaa cacccaaggg tgctataaac 2580
   accagcctcc catttcagaa tatacatccg atcacaattg gaaaatgtcc aaaatatgta 2640
   aaaagcacaa aattgagact ggccacagga ttgaggaata tcccgtctat tcaatctaga 2700
   ggactatttg gggccattgc cggtttcatt gaaggggggt ggacagggat ggtagatgga 2760
   tggtacggtt atcaccatca aaatgagcag gggtcaggat atgcagccga cctgaagagc 2820
   acacagaatg ccattgacga gattactaac aaagtaaatt ctgttattga aaagatgaat 2880
   acacagttca cagcagtagg taaagagttc aaccacctgg aaaaaagaat agagaattta 2940
   aataaaaaag ttgatgatgg tttcctggac atttggactt acaatgccga actgttggtt 3000
   ctattggaaa atgaaagaac tttggactac cacgattcaa atgtgaagaa cttatatgaa 3060
   aaggtaagaa gccagctaaa aaacaatgcc aaggaaattg gaaacggctg ctttgaattt 3120
   taccacaaat gcgataacac gtgcatggaa agtgtcaaaa atgggactta tgactaccca 3180
   aaatactcag aggaagcaaa attaaacaga gaagaaatag atggggtaaa gctggaatca 3240
   acaaggattt accagatttt ggcgatctat tcaactgtcg ccagttcatt ggtactggta 3300
   gtctccctgg gggcaatcag tttctggatg tgctctaatg ggtctctaca gtgtagaata 3360
   tgtatttaaa ggcctatttt ctttagtttg aatttactgt tattcggtgt gcatttctat 3420
   gtttggtgag cggttttctg tgctcagagt gtgtttattt tatgtaattt aatttctttg 3480
   tgagctcctg tttagcaggt cgtcccttca gcaaggacac aaaaagattt taattttatt 3540
   aaaaaaaaaa aaaaaaaaga ccgggaattc gatatcaagc ttatcgacct gcagatcgtt 3600
   caaacatttg gcaataaagt ttcttaagat tgaatcctgt tgccggtctt gcgatgatta 3660
   tcatataatt tctgttgaat tacgttaagc atgtaataat taacatgtaa tgcatgacgt 3720
   tatttatgag atgggttttt atgattagag tcccgcaatt atacatttaa tacgcgatag 3780
   aaaacaaaat atagcgcgca aactaggata aattatcgcg cgcggtgtca tctatgttac 3840
   tagatctcta gagtctcaag cttggcgcgg ggtaccgagc tcgaattccg agtgtacttc 3900
   aagtcagttg gaaatcaata aaatgattat tttatgaata tatttcattg tgcaagtaga 3960
   tagaaattac atatgttaca taacacacga aataaacaaa aaaacacaat ccaaaacaaa 4020
   caccccaaac aaaataacac tatatatatc ctcgtatgag gagaggcacg ttcagtgact 4080
   cgacgattcc cgagcaaaaa aagtctcccc gtcacacata tagtgggtga cgcaattatc 4140
   ttcaaagtaa tccttctgtt gacttgtcat tgataacatc cagtcttcgt caggattgca 4200
   aagaattata gaaggatccc caccttttat tttcttcttt tttccatatt tagggttgac 4260
   agtgaaatca gactggcaac ctattaattg cttccacaat gggacgaact tgaaggggat 4320
   gtcgtcgatg atattatagg tggcgtgttc atcgtagttg gtgaagtcga tggtcccgtt 4380
   ccagtagttg tgtcgcccga gacttctagc ccaggtggtc tttccggtac gagttggtcc 4440
   gcagatgtag aggctggggt gtctgacccc agtccttccc tcatcctggt tagatcggcc 4500
   atccactcaa ggtcagattg tgcttgatcg taggagacag gatgtatgaa agtgtaggca 4560
   tcgatgctta catgatatag gtgcgtctct ctccagttgt gcagatcttc gtggcagcgg 4620
   agatctgatt ctgtgaaggg cgacacgtac tgctcaggtt gtggaggaaa taatttgttg 4680
   gctgaatatt ccagccattg aagctttgtt gcccattcat gagggaattc ttctttgatc 4740
   atgtcaagat actcctcctt agacgttgca gtctggataa tagttcgcca tcgtgcgtca 4800
   gatttgcgag gagagacctt atgatctcgg aaatctcctc tggttttaat atctccgtcc 4860
   tttgatatgt aatcaaggac ttgtttagag tttctagctg gctggatatt agggtgattt 4920
   ccttcaaaat cgaaaaaaga aggatcccta atacaaggtt ttttatcaag ctggataaga 4980
   gcatgatagt gggtagtgcc atcttgatga agctcagaag caacaccaag gaagaaaata 5040
   agaaaaggtg tgagtttctc ccagagaaac tggaataaat catctctttg agatgagcac 5100
   ttggggtagg taaggaaaac atatttagat tggagtctga agttcttgct agcagaaggc 5160
   atgttgttgt gactccgagg ggttgcctca aactctatct tataaccggc gtggaggcat 5220
   ggaggcaagg gcattttggt aatttaagta gttagtggaa aatgacgtca tttacttaaa 5280
   gacgaagtct tgcgacaagg ggggcccacg ccgaatttta atattaccgg cgtggcccca 5340
   ccttatcgcg agtgctttag cacgagcggt ccagatttaa agtagaaaag ttcccgccca 5400
   ctagggttaa aggtgttcac actataaaag catatacgat gtgatggtat ttgatggagc 5460
   gtatattgta tcaggtattt ccgtcggata cgaattattc gtacggccgg ccactagtaa 5520
   ttgagcttgg cactggccgt cgttttacaa cgtcgtgact gggaaaaccc tggcgttacc 5580
   caacttaatc gccttgcagc acatccccct ttcgccagct ggcgtaatag cgaagaggcc 5640
   cgcaccgatc gcccttccca acagttgcgc agcctgaatg gcgaatgcta gagcagcttg 5700
   agcttggatc agattgtcgt ttcccgcctt cagtttaaac 5740
<210> 17
   <211> 5743
   <212> DNA
   <213> Artificial Sequence
<220>
   <223> Synthesized construct number 473
<400> 17
   ggcgcgccct agcagaaggc atgttgttgt gactccgagg ggttgcctca aactctatct    60
   tataaccggc gtggaggcat ggaggcaagg gcattttggt aatttaagta gttagtggaa   120
   aatgacgtca tttacttaaa gacgaagtct tgcgacaagg ggggcccacg ccgaatttta 180
   atattaccgg cgtggcccca ccttatcgcg agtgctttag cacgagcggt ccagatttaa 240
   agtagaaaag ttcccgccca ctagggttaa aggtgttcac actataaaag catatacgat 300
   gtgatggtat ttgataaagc gtatattgta tcaggtattt ccgtcggata cgaattattc 360
   gtacaagctt cttaagcctg caggtcaaca tggtggagca cgacacactt gtctactcca 420
   aaaatatcaa agatacagtc tcagaagacc aaagggcaat tgagactttt caacaaaggg 480
   taatatccgg aaacctcctc ggattccatt gcccagctat ctgtcacttt attgtgaaga 540
   tagtggaaaa ggaaggtggc tcctacaaat gccatcattg cgataaagga aaggccatcg 600
   ttgaagatgc ctctgccgac agtggtccca aagatggacc cccacccacg aggagcatcg 660
   tggaaaaaga agacgttcca accacgtctt caaagcaagt ggattgatgt gataacatgg 720
   tggagcacga cacacttgtc tactccaaaa atatcaaaga tacagtctca gaagaccaaa 780
   gggcaattga gacttttcaa caaagggtaa tatccggaaa cctcctcgga ttccattgcc 840
   cagctatctg tcactttatt gtgaagatag tggaaaagga aggtggctcc tacaaatgcc 900
   atcattgcga taaaggaaag gccatcgttg aagatgcctc tgccgacagt ggtcccaaag 960
   atggaccccc acccacgagg agcatcgtgg aaaaagaaga cgttccaacc acgtcttcaa 1020
   agcaagtgga ttgatgtgat atctccactg acgtaaggga tgacgcacaa tcccactatc 1080
   cttcgcaaga cccttcctct atataaggaa gttcatttca tttggagagg tattaaaatc 1140
   ttaataggtt ttgataaaag cgaacgtggg gaaacccgaa ccaaaccttc ttctaaactc 1200
   tctctcatct ctcttaaagc aaacttctct cttgtctttc ttgcgtgagc gatcttcaac 1260
   gttgtcagat cgtgcttcgg caccagtaca acgttttctt tcactgaagc gaaatcaaag 1320
   atctctttgt ggacacgtag tgcggcgcca ttaaataacg tgtacttgtc ctattcttgt 1380
   cggtgtggtc ttgggaaaag aaagcttgct ggaggctgct gttcagcccc atacattact 1440
   tgttacgatt ctgctgactt tcggcgggtg caatatctct acttctgctt gacgaggtat 1500
   tgttgcctgt acttctttct tcttcttctt gctgattggt tctataagaa atctagtatt 1560
   ttctttgaaa cagagttttc ccgtggtttt cgaacttgga gaaagattgt taagcttctg 1620
   tatattctgc ccaaatttgt cgggcccatg gcgaaaaacg ttgcgatttt cggcttattg 1680
   ttttctcttc ttgtgttggt tccttctcag atcttcgctc aaaaacttcc cggaaatgac 1740
   aacagcacgg caacgctgtg ccttgggcac catgcagtac caaacggaac gatagtgaaa 1800
   acaatcacga atgaccaaat tgaagttact aatgctactg agctggttca gagttcctca 1860
   acaggtgaaa tatgcgacag tcctcatcag atccttgatg gagaaaactg cacactaata 1920
   gatgctctat tgggagaccc tcagtgtgat ggcttccaaa ataagaaatg ggaccttttt 1980
   gttgaacgca gcaaagccta cagcaactgt tacccttatg atgtgccgga ttatgcctcc 2040
   cttaggtcac tagttgcctc atccggcaca ctggagttta acaatgaaag tttcaattgg 2100
   actggagtca ctcaaaacgg aacaagctct gcttgcataa ggagatctaa taacagtttc 2160
   tttagtagat tgaattggtt gacccactta aaattcaaat acccagcatt gaacgtgact 2220
   atgccaaaca atgaaaaatt tgacaaattg tacatttggg gggttcacca cccgggtacg 2280
   gacaatgacc aaatcttcct gtatgctcaa gcatcaggaa gaatcacagt ctctaccaaa 2340
   agaagccaac aaactgtaat cccgaatatc ggatctagac ccagagtaag gaatatcccc 2400
   agcagaataa gcatctattg gacaatagta aaaccgggag acatactttt gattaacagc 2460
   acagggaatc taattgctcc taggggttac ttcaaaatac gaagtgggaa aagctcaata 2520
   atgagatcag atgcacccat tggcaaatgc aattctgaat gcatcactcc aaacggaagc 2580
   attcccaatg acaaaccatt ccaaaatgta aacaggatca catacggggc ctgtcccaga 2640
   tatgttaagc aaaacactct gaaattggca acagggatgc gaaatgtacc agagaaacaa 2700
   actagaggca tatttggcgc aatcgcgggt ttcatagaaa atggttggga gggaatggtg 2760
   gatggttggt atggtttcag gcatcaaaat tctgagggaa taggacaagc agcagatctc 2820
   aaaagcactc aagcagcaat cgatcaaatc aatgggaagc tgaataggtt gatcgggaaa 2880
   accaacgaga aattccatca gattgaaaaa gagttctcag aagtcgaagg gagaatccag 2940
   gaccttgaga aatatgttga ggacaccaaa atagatctct ggtcatacaa cgcggagctt 3000
   cttgttgccc tggagaacca acatacaatt gatctaactg actcagaaat gaacaaactg 3060
   tttgaaaaaa caaagaagca actgagggaa aatgctgagg atatgggcaa tggttgtttc 3120
   aaaatatacc acaaatgtga caatgcctgc ataggatcaa tcagaaatgg aacttatgac 3180
   cacgatgtat acagagatga agcattaaac aaccggttcc agatcaaggg cgttgagctg 3240
   aagtcaggat acaaagattg gatactatgg atttcctttg ccatatcatg ttttttgctt 3300
   tgtgttgctt tgttggggtt catcatgtgg gcctgccaaa aaggcaacat taggtgcaac 3360
   atttgcattt gaaggcctat tttctttagt ttgaatttac tgttattcgg tgtgcatttc 3420
   tatgtttggt gagcggtttt ctgtgctcag agtgtgttta ttttatgtaa tttaatttct 3480
   ttgtgagctc ctgtttagca ggtcgtccct tcagcaagga cacaaaaaga ttttaatttt 3540
   attaaaaaaa aaaaaaaaaa agaccgggaa ttcgatatca agcttatcga cctgcagatc 3600
   gttcaaacat ttggcaataa agtttcttaa gattgaatcc tgttgccggt cttgcgatga 3660
   ttatcatata atttctgttg aattacgtta agcatgtaat aattaacatg taatgcatga 3720
   cgttatttat gagatgggtt tttatgatta gagtcccgca attatacatt taatacgcga 3780
   tagaaaacaa aatatagcgc gcaaactagg ataaattatc gcgcgcggtg tcatctatgt 3840
   tactagatct ctagagtctc aagcttggcg cggggtaccg agctcgaatt ccgagtgtac 3900
   ttcaagtcag ttggaaatca ataaaatgat tattttatga atatatttca ttgtgcaagt 3960
   agatagaaat tacatatgtt acataacaca cgaaataaac aaaaaaacac aatccaaaac 4020
   aaacacccca aacaaaataa cactatatat atcctcgtat gaggagaggc acgttcagtg 4080
   actcgacgat tcccgagcaa aaaaagtctc cccgtcacac atatagtggg tgacgcaatt 4140
   atcttcaaag taatccttct gttgacttgt cattgataac atccagtctt cgtcaggatt 4200
   gcaaagaatt atagaaggga tcccaccttt tattttcttc ttttttccat atttagggtt 4260
   gacagtgaaa tcagactggc aacctattaa ttgcttccac aatgggacga acttgaaggg 4320
   gatgtcgtcg atgatattat aggtggcgtg ttcatcgtag ttggtgaagt cgatggtccc 4380
   gttccagtag ttgtgtcgcc cgagacttct agcccaggtg gtctttccgg tacgagttgg 4440
   tccgcagatg tagaggctgg ggtgtctgac cccagtcctt ccctcatcct ggttagatcg 4500
   gccatccact caaggtcaga ttgtgcttga tcgtaggaga caggatgtat gaaagtgtag 4560
   gcatcgatgc ttacatgata taggtgcgtc tctctccagt tgtgcagatc ttcgtggcag 4620
   cggagatctg attctgtgaa gggcgacacg tactgctcag gttgtggagg aaataatttg 4680
   ttggctgaat attccagcca ttgaagcttt gttgcccatt catgagggaa ttcttctttg 4740
   atcatgtcaa gatactcctc cttagacgtt gcagtctgga taatagttcg ccatcgtgcg 4800
   tcagatttgc gaggagagac cttatgatct cggaaatctc ctctggtttt aatatctccg 4860
   tcctttgata tgtaatcaag gacttgttta gagtttctag ctggctggat attagggtga 4920
   tttccttcaa aatcgaaaaa agaaggatcc ctaatacaag gttttttatc aagctggata 4980
   agagcatgat agtgggtagt gccatcttga tgaagctcag aagcaacacc aaggaagaaa 5040
   ataagaaaag gtgtgagttt ctcccagaga aactggaata aatcatctct ttgagatgag 5100
   cacttggggt aggtaaggaa aacatattta gattggagtc tgaagttctt gctagcagaa 5160
   ggcatgttgt tgtgactccg aggggttgcc tcaaactcta tcttataacc ggcgtggagg 5220
   catggaggca agggcatttt ggtaatttaa gtagttagtg gaaaatgacg tcatttactt 5280
   aaagacgaag tcttgcgaca aggggggccc acgccgaatt ttaatattac cggcgtggcc 5340
   ccaccttatc gcgagtgctt tagcacgagc ggtccagatt taaagtagaa aagttcccgc 5400
   ccactagggt taaaggtgtt cacactataa aagcatatac gatgtgatgg tatttgatgg 5460
   agcgtatatt gtatcaggta tttccgtcgg atacgaatta ttcgtacggc cggccactag 5520
   taattgagct tggcactggc cgtcgtttta caacgtcgtg actgggaaaa ccctggcgtt 5580
   acccaactta atcgccttgc agcacatccc cctttcgcca gctggcgtaa tagcgaagag     5640
   gcccgcaccg atcgcccttc ccaacagttg cgcagcctga atggcgaatg ctagagcagc     5700
   ttgagcttgg atcagattgt cgtttcccgc cttcagttta aac                       5743
<210> 18
   <211> 5725
   <212> DNA
   <213> Artificial Sequence
<220>
   <223> Synthesized construct number 474
<400> 18
<210> 19
   <211> 25
   <212> DNA
   <213> Artificial Sequence
<220>
   <223> Synthesized primer Plasto-443c
<400> 19
   gtattagtaa ttagaatttg gtgtc 25
<210> 20
   <211> 34
   <212> DNA
   <213> Artificial Sequence
<220>
   <223> Synthesized primer supPl9-plasto.r
<400> 20
   ccttgtatag ctcgttccat tttctctcaa gatg 34
<210> 21
   <211> 20
   <212> DNA
   <213> Artificial sequence
<220>
   <223> Synthesized primer supP19-1c
<400> 21
   atggaacgag ctatacaagg 20
<210> 22
   <211> 32
   <212> DNA
   <213> Artificial Sequence
<220>
   <223> Synthesized primer SupP19-SacI.r
<400> 22
   agtcgagctc ttactcgctt tctttttcga ag 32
<210> 23
   <211> 509
   <212> DNA
   <213> Cowpea Mosaic Virus
<400> 23
   tattaaaatc ttaataggtt ttgataaaag cgaacgtggg gaaacccgaa ccaaaccttc      60
   ttctaaactc tctctcatct ctcttaaagc aaacttctct cttgtctttc ttgcgtgagc     120
   gatcttcaac gttgtcagat cgtgcttcgg caccagtaca acgttttctt tcactgaagc     180
   gaaatcaaag atctctttgt ggacacgtag tgcggcgcca ttaaataacg tgtacttgtc     240
   ctattcttgt cggtgtggtc ttgggaaaag aaagcttgct ggaggctgct gttcagcccc     300
   atacattact tgttacgatt ctgctgactt tcggcgggtg caatatctct acttctgctt     360
   gacgaggtat tgttgcctgt acttctttct tcttcttctt gctgattggt tctataagaa     420
   atctagtatt ttctttgaaa cagagttttc ccgtggtttt cgaacttgga gaaagattgt     480
   taagcttctg tatattctgc ccaaatttg                                       509

## Description

The present invention relates to the expression of proteins of interest in plants. The present invention also provides methods and compositions for the production of proteins of interest in plants.

Influenza is the leading cause of death in humans due to a respiratory virus. Common symptoms include fever, sore throat, shortness of breath, and muscle soreness, among others. During flu season, influenza viruses infect 10-20% of the population worldwide, leading to 250-500,000 deaths annually. Influenza pandemics are usually caused by highly transmittable and virulent influenza viruses, and can lead to elevated levels of illness and death globally. The emergence of new influenza A subtypes resulted in 4 major pandemics in the 20th century resulting in significant mortality and economic disruption. There is increasing concern that the virus may become highly infectious for humans. The major problem for human health is the fact that influenza viruses are antigenically unstable, that is, they mutate rapidly.

Influenza viruses are classified into types A, B, or C, based on the nucleoproteins and matrix protein antigens present. Influenza type A viruses may be further divided into subtypes according to the combination of hemagglutinin (HA) and neuraminidase (NA) surface glycoproteins presented. HA governs the ability of the virus to bind to and penetrate the host cell. NA removes terminal sialic acid residues from glycan chains on host cell and viral surface proteins, which prevents viral aggregation and facilitates virus mobility.

The current method of combating influenza in humans is by annual vaccination. Each year, the World Health Organization selects certain viral strains for inclusion in the annual influenza vaccine, which is produced in fertilized eggs. However, the number of vaccine doses produced each year is not sufficient to vaccinate the world's population. Furthermore, this production method faces a number of possible drawbacks including the possibility of contamination due to the use of whole virus, variable yields depending on the virus strain, extensive planning requirements for obtaining eggs, contamination risks due to chemicals used in purification, and long production times. Moreover, persons hypersensitive to egg proteins may not be eligible for receiving vaccines produced via this method.

Alternative production methods have been investigated. For example, influenza virus has been produced in mammalian cell cultures of, for example, in MDCK or PERC.6 cells, or the like. Another approach is reverse genetics, in which viruses are produced by cell transformation with viral genes. These methods, however, also requires the use of whole virus as well as elaborate methods and specific culture environments.

In order to protect the world population from influenza and to stave off future pandemics, vaccine manufacturers will need to develop effective, rapid methods producing vaccine doses. The current use of fertilized eggs to produce vaccines is insufficient and involves a lengthy process. Recombinant technologies offer promising approaches to the production of influenza antigens. However, the production of hemagglutinin has been limited to membrane-associated protein, which involves complex extraction processes with low yields, or to poorly-immunogenic soluble proteins.

Plants offer great potential as production systems for recombinant proteins. One approach to producing foreign proteins in plants is to generate stable transgenic plant lines. However this is a time consuming and labor intensive process. An alternative to transgenic plants is the use of plant virus-based expression vectors. Plant virus-based vectors allow for the rapid, high level, transient expression of proteins in plants.

Many different plant viruses have been modified to function as expression vectors. For example, Cowpea Mosaic Virus (CPMV) has been utilized for producing a variety of proteins (see, for example, WO2007/135480; WO2009/087391; Sainsbury F. et al., 2008, Plant Physiology; 148: 121-1218; Sainsbury F. et al., 2008, Plant Biotechnology Journal; 6: 82-92; Sainsbury F. et al., 2009, Plant Biotechnology Journal; 7: 682-693).

A geminivirus-derived, plant-based system has been proposed for the production of virus-like particles (Huang et al., 2009, Biotechnology and Bioengineering, 103: 706-714). Co-delivery of bean yellow dwarf virus (BeYDV)-derived vector and Rep/RepA-supplying vector by agroinfiltration of *Nicotiana benthamiana* leaves resulted in replicon amplification and protein production.

Plant-based expression systems preferably have a number of properties such as, for example, containing convenient cloning sites for genes of interest, can easily infect plants in a cost-effective manner, and/or can cause efficient local/systemic infection of inoculated plants. In addition, the infection should provide a good yield of useful protein material. Despite the advances made in modifying viruses to serve as expression systems in plants there is still room for improvement.

The present invention relates to the expression of proteins of interest in plants. The present invention also provides methods and compositions for the production of proteins of interest in plants. Also provided are nucleic acids and expression systems to produce proteins of interest in plants. The expression systems comprise a comovirus-based expression cassette and a geminivirus-based amplification element. Plant cells, plant tissues, whole plants, nucleic acids comprising gene constructs encoding the protein of interest, and methods of expressing proteins of interest in plants are also provided.

The present disclosure also provides an expression system for expressing hemagglutinin protein in plants.

The present invention further provides a plant expression system (A) comprising a first nucleic acid sequence comprising a regulatory region, operatively linked with a one or more than one comovirus enhancer, a nucleotide sequence of interest, and one or more than one geminivirus amplification element, and a second nucleic acid encoding a geminivirus replicase. The regulatory region may be selected from a plastocyanin promoter, a CaMV 35S promoter, a 2x CaMV35S promoter, a CAS promoter, a RbcS promoter, a Ubi promoter, or an actin promoter. Furthermore the one or more than one comovirus enhancer may be a comovirus 5' UTR, for example, a Cowpea Mosaic Virus (CPMV) 5'UTR, or the CPMV 5'UTR may comprise the nucleotide sequence of SEQ ID NO:23. Furthermore, the one or more than one comovirus enhancer may comprises a comovirus 3' UTR, for example, a Cowpea Mosaic Virus 3' UTR, and a plastocyanin 3' UTR. In addition, the one or more than one geminivirus amplification element may be selected from a Bean Yellow Dwarf Virus long intergenic region (BeYDV LIR), and a BeYDV short intergenic region (BeYDV SIR).

The present invention also provides the plant expression system (A) as described above, wherein the first nucleic acid sequence and the second nucleic acid sequence may be located on the same nucleic acid molecule, or they may be located on different DNA molecules.

The plant expression system (A) as defined above may further comprise a third nucleic acid sequence, the third nucleic acid sequence encoding a suppressor of silencing. The suppressor of silencing may be selected from the group HcPro and p19. The third nucleic acid sequence and the first nucleic acid sequence may be located on the same molecule, or the third nucleic acid sequence and the first nucleic acid sequence may be located on different molecules. The plant expression system may also comprises a forth nucleic acid sequence, the forth nucleic acid sequence encoding a chaperone protein. The chaperone protein may be selected from the group Hsp40 and Hsp70. The forth nucleic acid sequence and the first nucleic acid sequence may be located on the same molecule, or the forth nucleic acid sequence and the first nucleic acid sequence may be located on different DNA molecules.

The present invention provides the plant expression system (A) as defined above, wherein the nucleotide sequence of interest encodes an influenza hemagglutinin. The influenza hemagglutinin may comprises a native signal peptide sequence, or a non-native signal peptide. Furthermore, the influenza hemagglutinin is selected from influenza type B hemagglutinin and influenza type A subtype H3 hemagglutinin.

The present invention also pertains to a plant expression system (B) comprising a nucleic acid sequence comprising a regulatory sequence operatively linked to one or more than one comovirus enhancer; a nucleotide sequence of interest; one or more than one geminivirus amplification element, and a nucleic acid sequence encoding a geminivirus replicase protein. The regulatory region may be selected from a plastocyanin promoter, a CaMV 35S promoter, a 2x CaMV35S promoter, a CAS promoter, a RbcS promoter, a Ubi promoter, or an actin promoter. Furthermore the one or more than one comovirus enhancer may be a comovirus 5' UTR, for example, a Cowpea Mosaic Virus (CPMV) 5'UTR, or the CPMV 5'UTR may comprise the nucleotide sequence of SEQ ID NO:23. Furthermore, the one or more than one comovirus enhancer may comprises a comovirus 3' UTR, for example, a Cowpea Mosaic Virus 3' UTR, and a plastocyanin 3' UTR. In addition, the one or more than one geminivirus amplification element may be selected from a Bean Yellow Dwarf Virus long intergenic region (BeYDV LIR), and a BeYDV short intergenic region (BeYDV SIR).

The plant expression system (B) as defined above may further comprise a second nucleic acid sequence, the second nucleic acid sequence encoding a suppressor of silencing. The suppressor of silencing may be selected from the group HcPro and p19. The second nucleic acid sequence and the nucleic acid sequence (comprising the regulatory sequence operatively linked to one or more than one comovirus enhancer; the nucleotide sequence of interest; one or more than one geminivirus amplification element, and the nucleic acid sequence encoding a geminivirus replicase protein) may be located on the same molecule, or the second nucleic acid sequence and the nucleic acid sequence may be located on different molecules. The plant expression system may also comprises a third nucleic acid sequence, the third nucleic acid sequence encoding a chaperone protein. The chaperone protein may be selected from the group Hsp40 and Hsp70. The third nucleic acid sequence and the nucleic acid sequence (comprising the regulatory sequence operatively linked to one or more than one comovirus enhancer; the nucleotide sequence of interest; one or more than one geminivirus amplification element, and the nucleic acid sequence encoding a geminivirus replicase protein) may be located on the same molecule, or the third nucleic acid sequence and the nucleic acid sequence may be located on different DNA molecules.

The present invention provides the plant expression system (B) as defined above, wherein the nucleotide sequence of interest encodes an influenza hemagglutinin. The influenza hemagglutinin may comprises a native signal peptide sequence, or a non-native signal peptide. Furthermore, the influenza hemagglutinin is selected from influenza type B hemagglutinin and influenza type A subtype H3 hemagglutinin.

The present invention also provides a method (A) of producing an influenza virus like particle (VLP) in a plant or in a portion of a plant, the method comprising,
- introducing into the plant or in the portion of a plant the plant expression system comprising a first nucleic acid sequence comprising a regulatory region, operatively linked with a one or more than one comovirus enhancer, a nucleotide sequence of interest, the nucleotide sequence of interest encoding an influenza hemagglutinin, and one or more than one geminivirus amplification element,
- a second nucleic acid encoding a geminivirus replicase, and
- incubating the plant or the portion of a plant under conditions that permit expression of the nucleotide sequence encoding the influenza hemagglutinin, thereby producing the influenza VLP.
The present invention also pertains to the method (A) as described above, wherein in the step of introducing, the first nucleic acid sequence and the second nucleic acid sequence may be located on the same molecule, or the first nucleic acid sequence and the second nucleic acid sequence maybe located on different molecules. If the first nucleic acid sequence and the second nucleic acid sequence are located on different molecules, then the different molecules may be introduced in the plant or in the portion of a plant at the same time or the different molecules may be introduced in the plant or in the portion of a plant at different times, for example by successive infiltration.
The present invention also provides the method (A) as described above , wherein in the step of incubating, the expression of the nucleotide sequence encoding the influenza hemagglutinin is a transient expression. Furthermore, the method may include a step of harvesting the plant or the portion of a plant, thereby obtaining a harvested plant or portion of a plant containing the influenza VLP. The method may further comprising a step of isolating the influenza VLP from the harvested plant or portion of a plant containing the influenza VLP, thereby obtaining an isolated influenza VLP.
The present invention provides the method (A) as descried above, wherein in the step of introducing, the plant expression system further comprises a third nucleic acid sequence, the third nucleic acid sequence encoding a suppressor of silencing expression. The third nucleic acid sequence and the first nucleic acid sequence may be located on the same molecule, or the third nucleic acid sequence and the first nucleic acid sequence may be located on different molecules. If the third nucleic acid sequence and the first nucleic acid sequence are located on different molecules, then the different molecules may be introduced in the plant or in the portion of a plant at the same time. Furthermore, within the step of introducing, the plant expression system may further comprises a fourth nucleic acid sequence, the forth nucleic acid sequence encoding a chaperone protein. The fourth nucleic acid sequence and the first nucleic acid sequence may be located on the same molecule, or the fourth nucleic acid sequence and the first nucleic acid sequence may be located on different molecules. If the fourth nucleic acid sequence and the first nucleic acid sequence are located on different molecules, then the different molecules may be introduced in the plant or in the portion of a plant at the same time.
The present invention also provides a method (B) of producing a protein of interest in a plant or in a portion of a plant, the method comprising,
- introducing in the plant or in the portion of a plant the plant expression system comprising a first nucleic acid sequence comprising a regulatory region, operatively linked with a one or more than one comovirus enhancer, a nucleotide sequence of interest, and one or more than one geminivirus amplification element, and a second nucleic acid encoding a geminivirus replicase, and
- incubating the plant or the portion of a plant under conditions that permit expression of the nucleotide sequence encoding the protein of interest, thereby producing the protein of interest.
The present invention also pertains to the method (B) as described above, wherein in the step of introducing, the first nucleic acid sequence and the second nucleic acid sequence may be located on the same molecule, or the first nucleic acid sequence and the second nucleic acid sequence maybe located on different molecules. If the first nucleic acid sequence and the second nucleic acid sequence are located on different molecules, then the different molecules may be introduced in the plant or in the portion of a plant at the same time.
The present invention also provides the method (B) as described above , wherein in the step of incubating, the expression of the nucleotide sequence encoding the influenza hemagglutinin is a transient expression. Furthermore, the method may include a step of harvesting the plant or the portion of a plant, thereby obtaining a harvested plant or portion of a plant containing the protein of interest. The method may further comprising a step of isolating the protein of interest from the harvested plant or portion of a plant containing the protein of interest, thereby obtaining an isolated protein of interest.
The present invention provides the method (B) as descried above, wherein in the step of introducing, the plant expression system further comprises a third nucleic acid sequence, the third nucleic acid sequence encoding a suppressor of silencing expression. The third nucleic acid sequence and the first nucleic acid sequence may be located on the same molecule, or the third nucleic acid sequence and the first nucleic acid sequence may be located on different molecules. If the third nucleic acid sequence and the first nucleic acid sequence are located on different molecules, then the different molecules may be introduced in the plant or in the portion of a plant at the same time. Furthermore, within the step of introducing, the plant expression system may further comprises a fourth nucleic acid sequence, the forth nucleic acid sequence encoding a chaperone protein. The fourth nucleic acid sequence and the first nucleic acid sequence may be located on the same molecule, or the fourth nucleic acid sequence and the first nucleic acid sequence may be located on different molecules. If the fourth nucleic acid sequence and the first nucleic acid sequence are located on different molecules, then the different molecules may be introduced in the plant or in the portion of a plant at the same time.
This summary does not necessarily describe all features of the invention. Other aspects, features and advantages of the invention will be apparent to those of ordinary skill in the art upon review of the following description of specific embodiments of the invention.

### BRIEF DESCRIPTION OF THE DRAWINGS

These and other features of the invention will become more apparent from the following description in which reference is made to the appended drawings wherein:

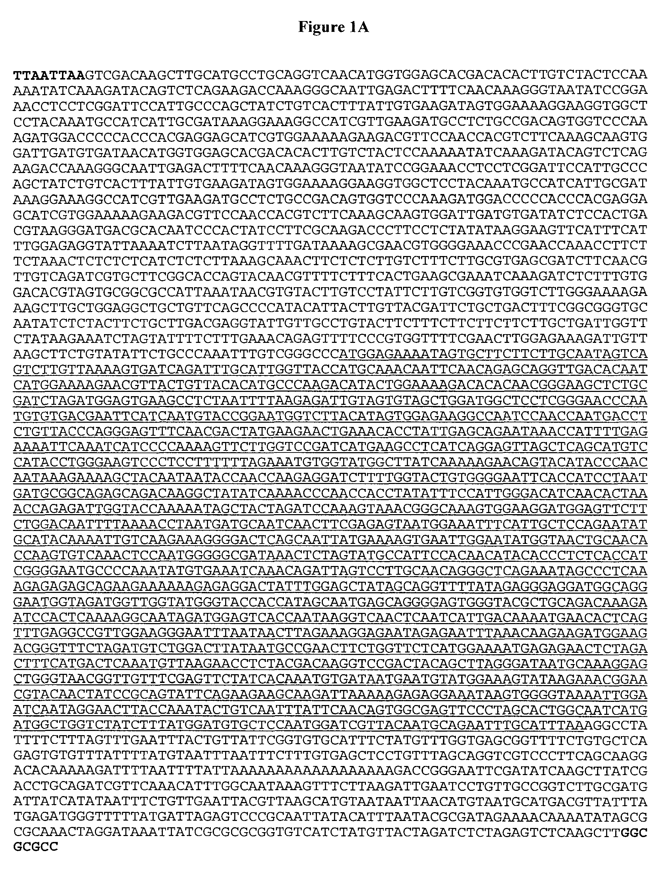

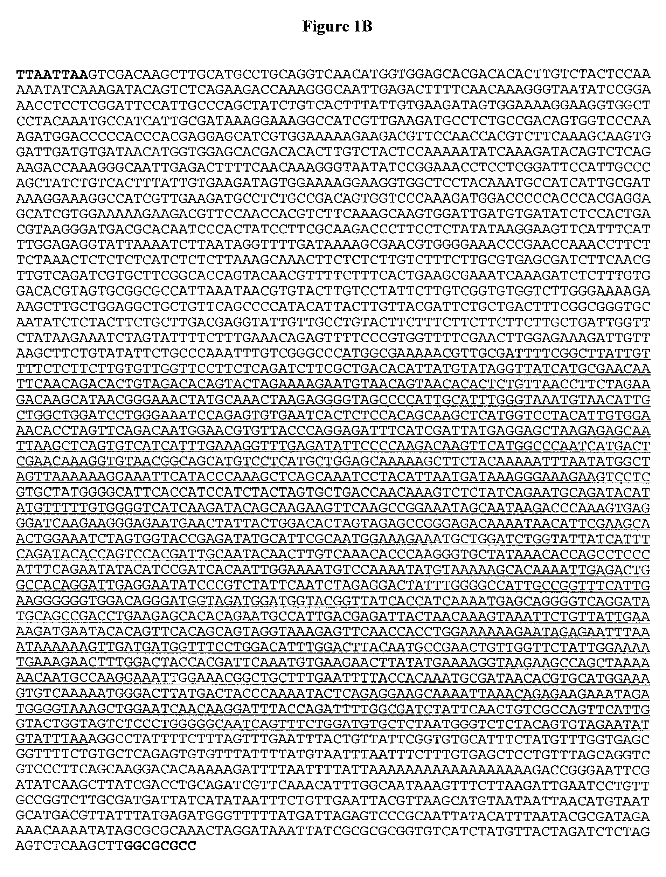

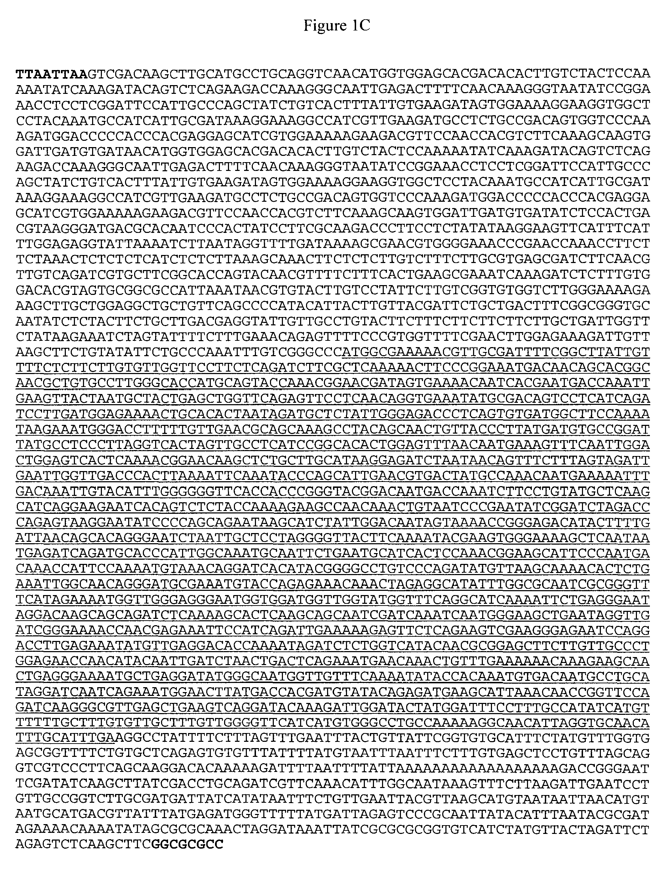

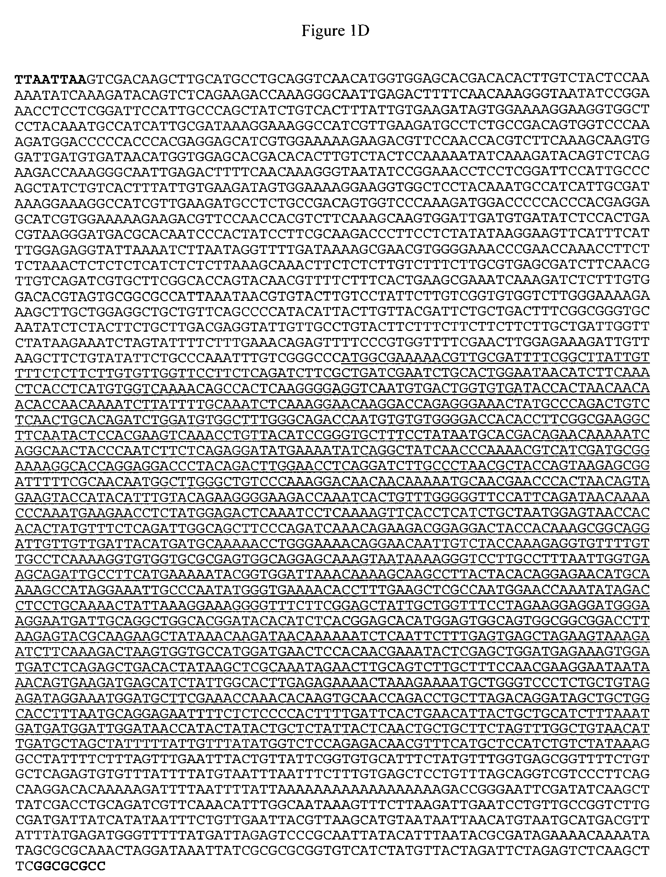

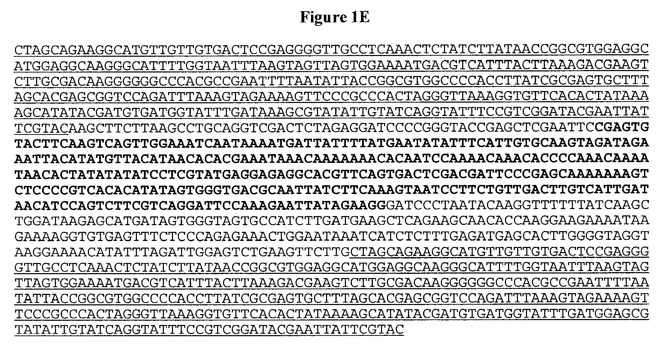

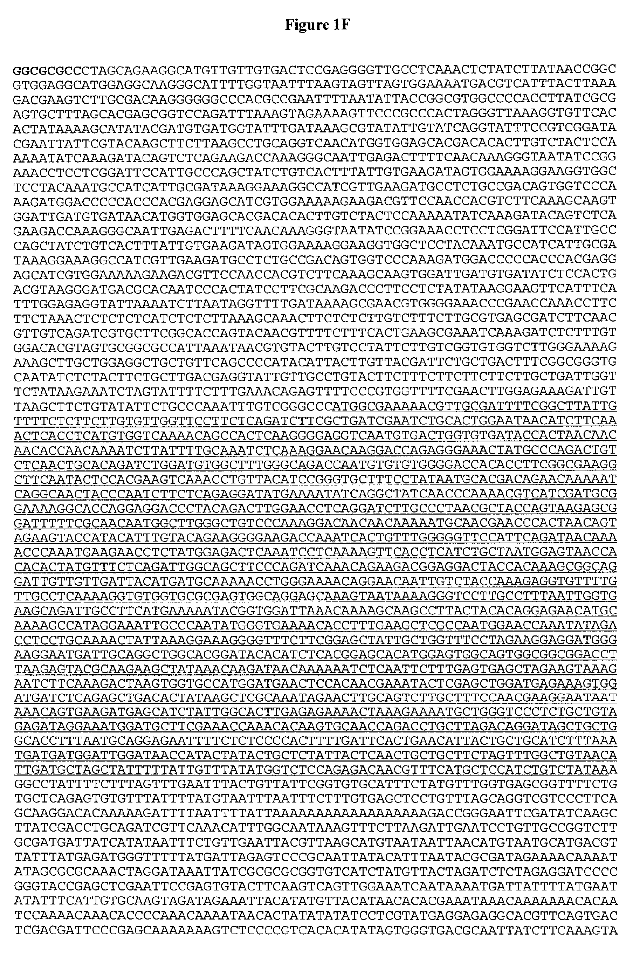

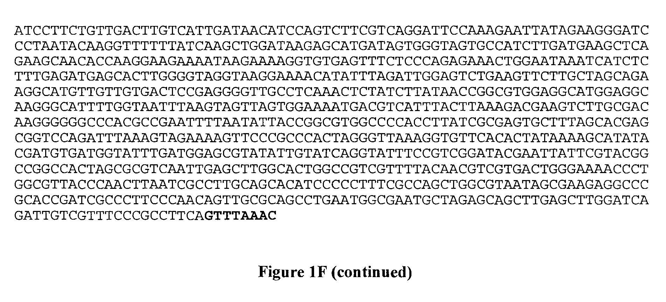

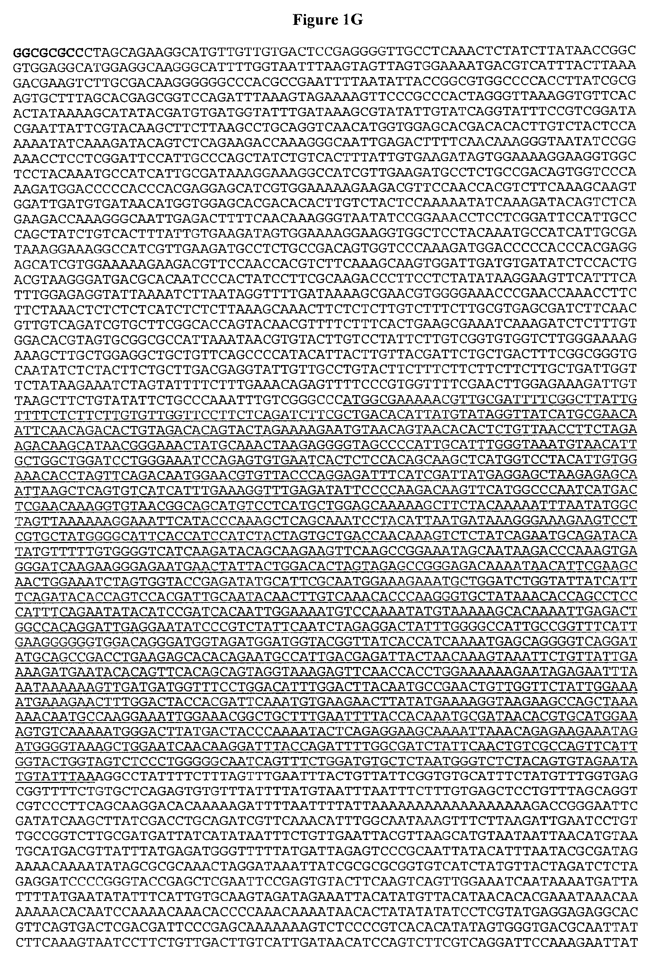

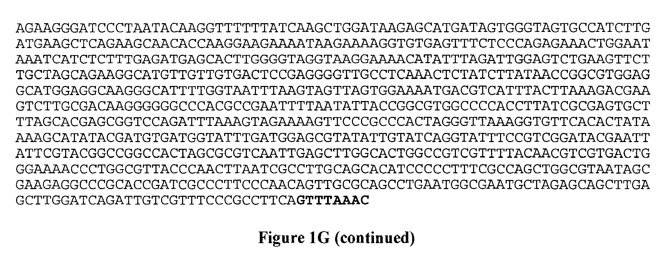

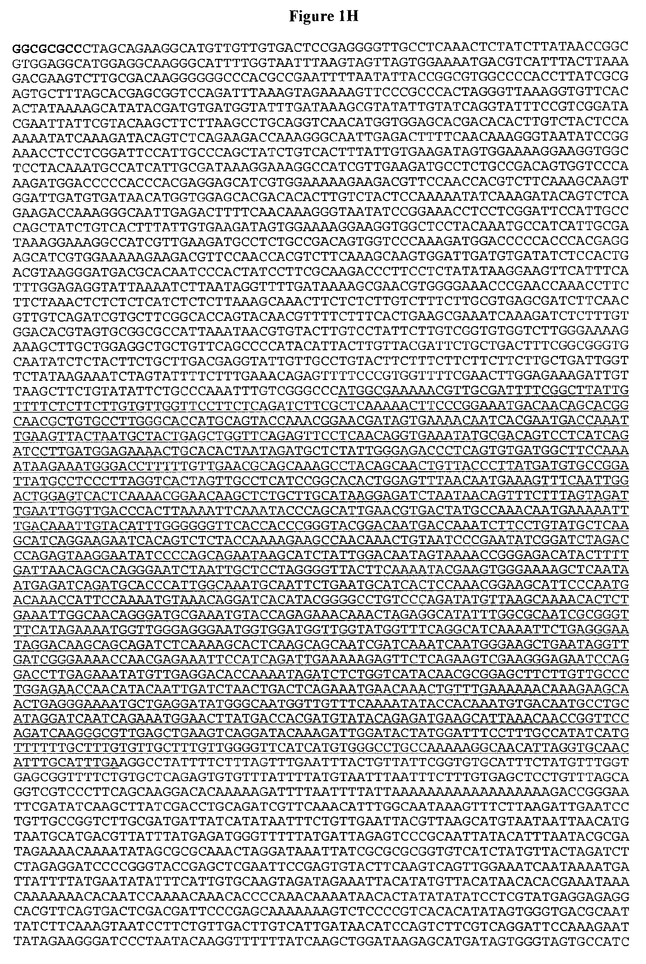

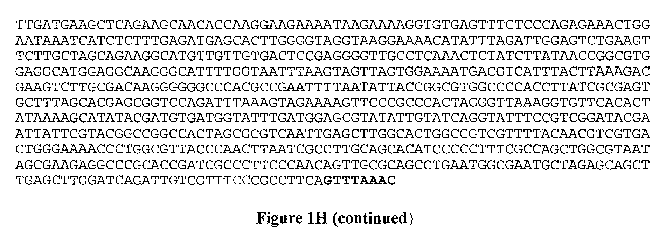

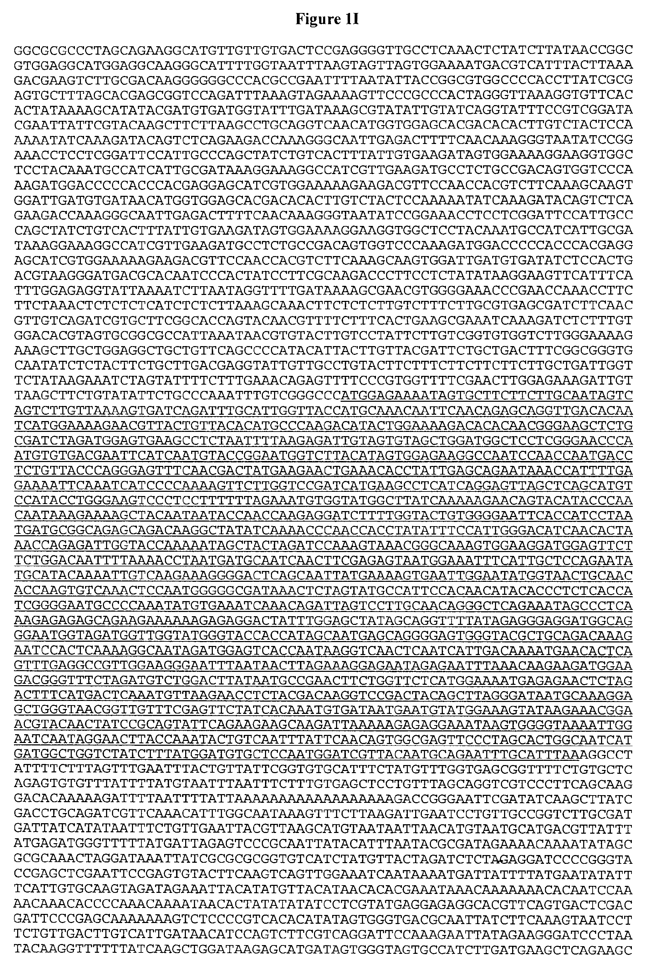

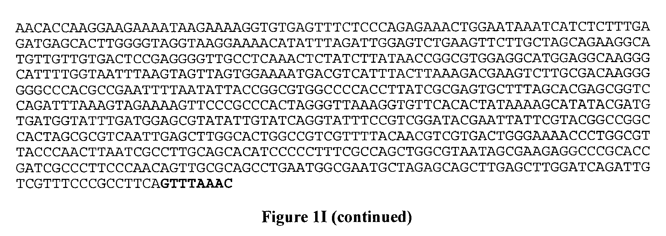

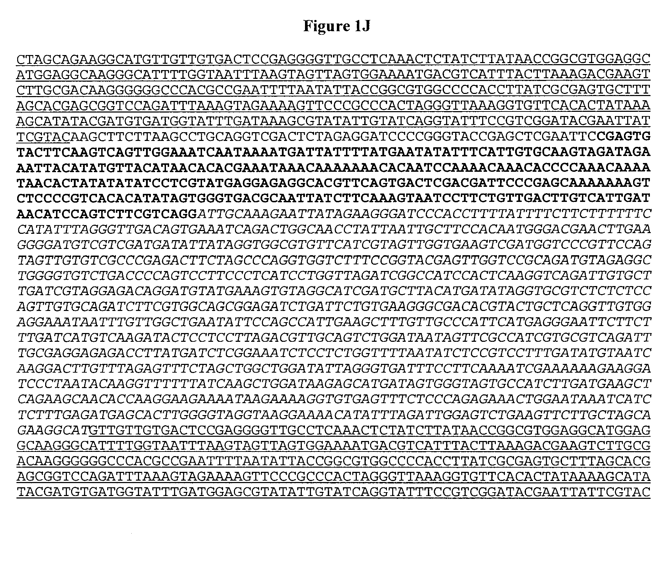

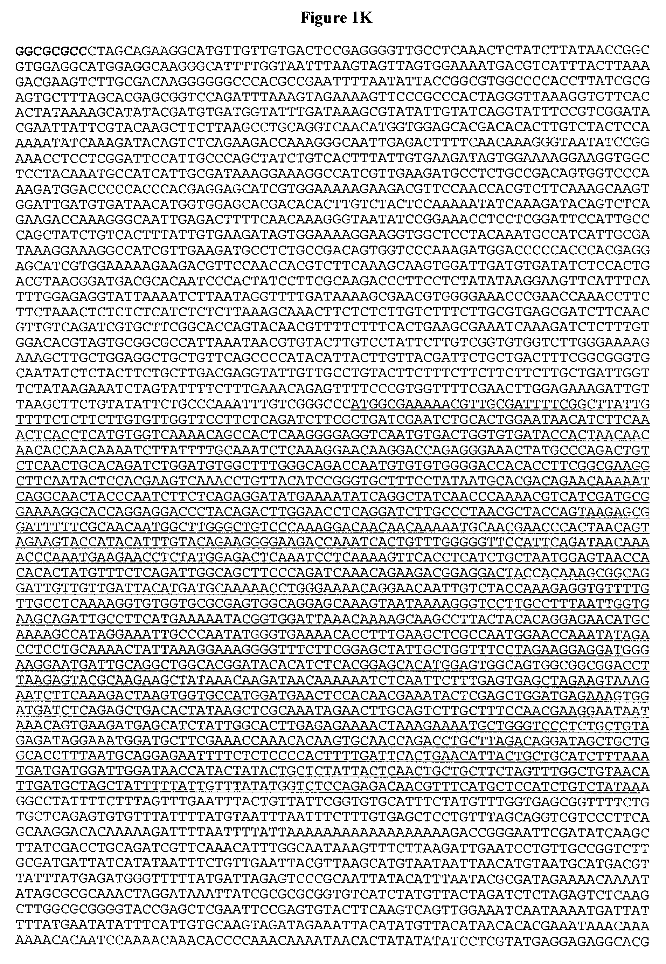

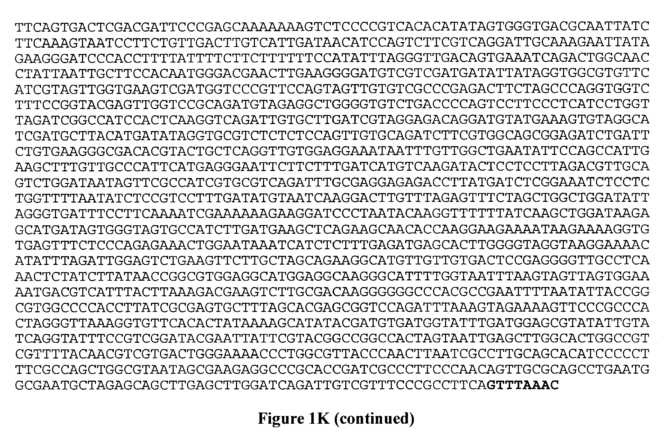

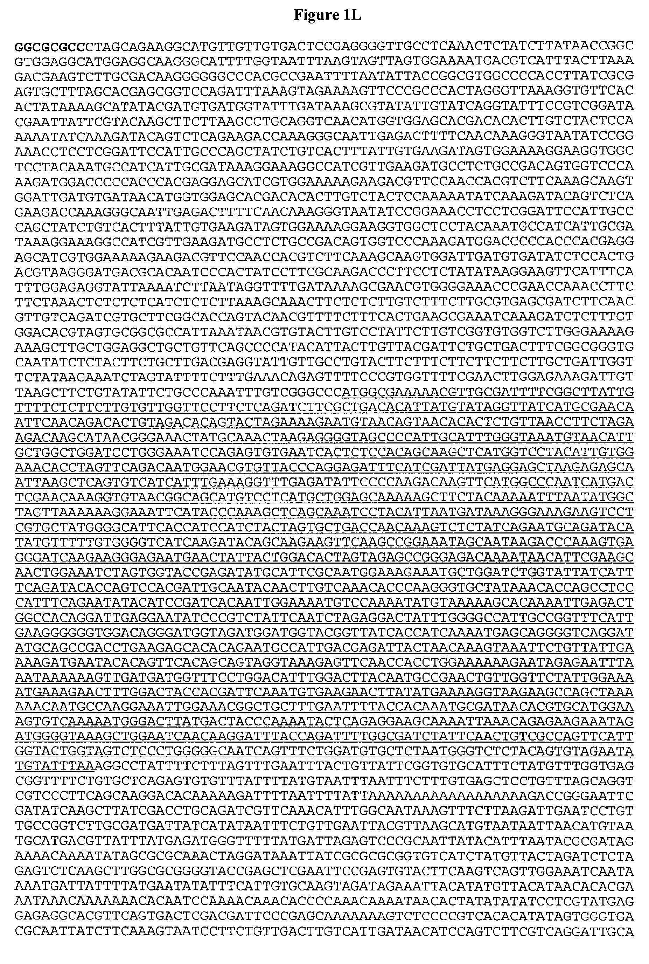

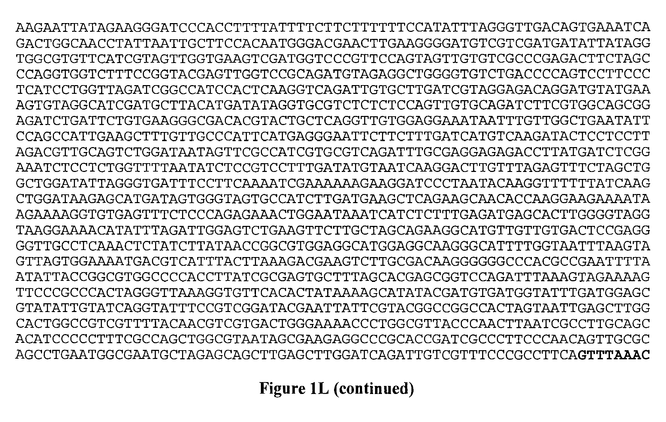

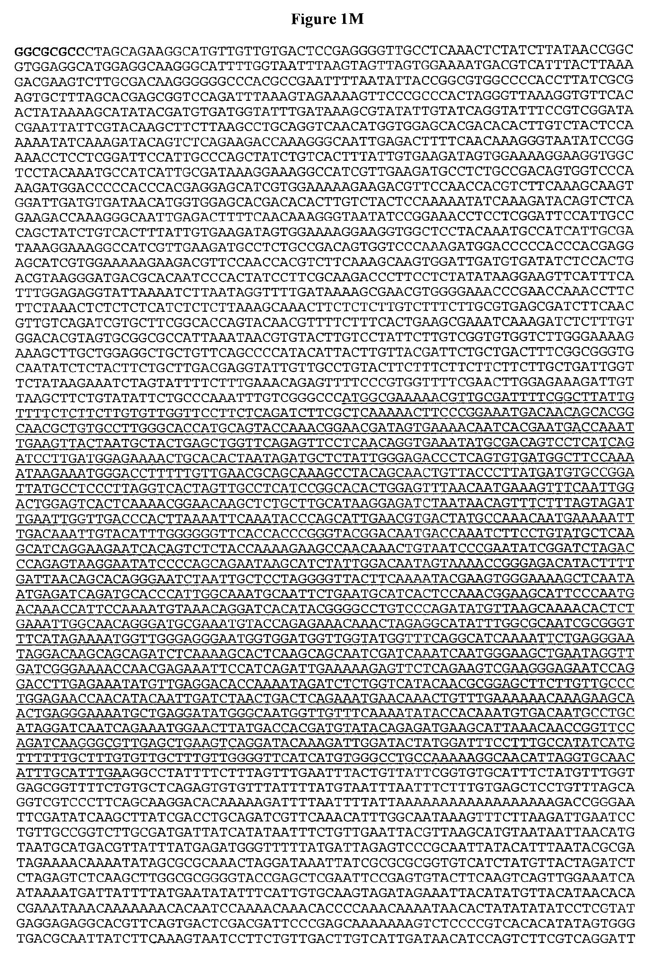

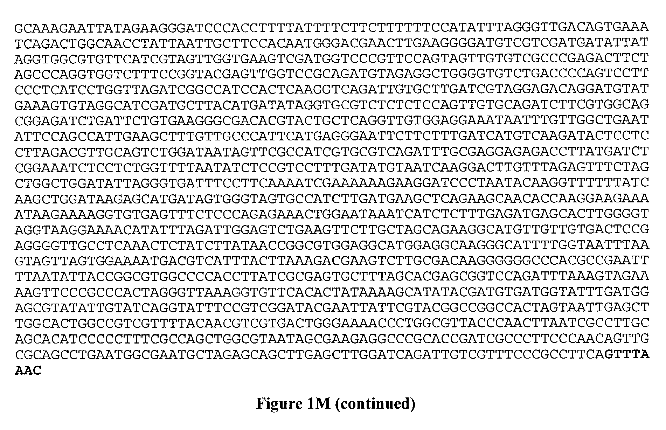

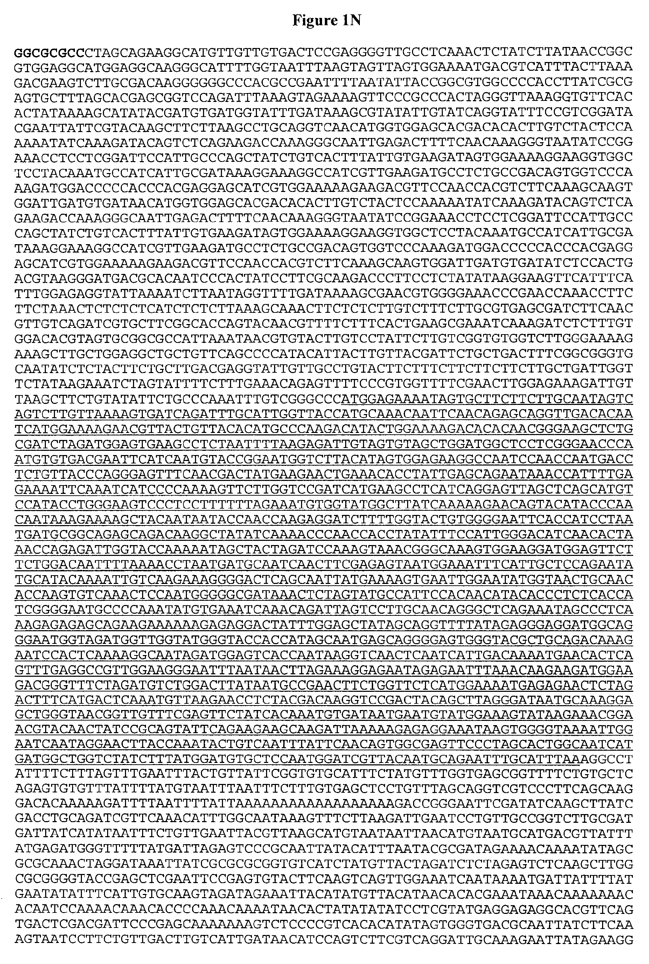

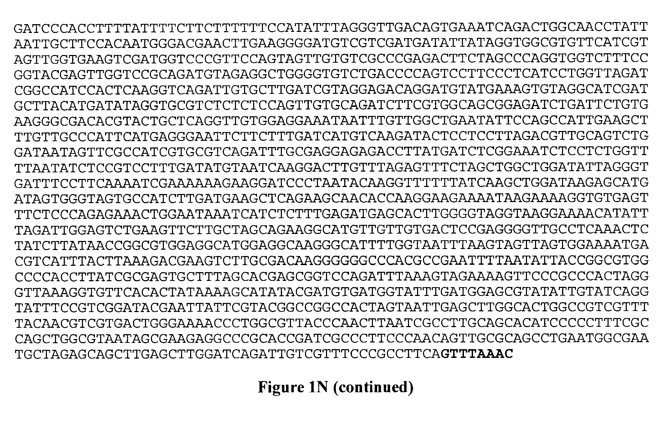

**Figure 1** shows nucleotide sequences of several constructs described in the present invention. **Figure 1A** shows the nucleotide sequence of expression cassette 972 (SEQ ID NO:1) comprising a sequence from PacI site (bold; upstreamof the promoter) to AscI site (bold immediately downstream of the NOS terminator), and includes wild type HA0 of H5 A/Indonesia/5/2005 (underlined). **Figure 1B** shows the nucleotide sequence of expression cassette 560 (SEQ ID NO:2) comprising a sequence from PacI site (bold; upstream of the promoter) to AscI site (bold; immediately downstream of the NOS terminator). PDI signal peptide (PDISP)-HAO of H1 A/California/4/2009 (underlined). **Figure 1C** shows the nucleotide sequence of expression cassette 971 (SEQ ID NO:3), comprising a sequence from PacI site (bold; upstream of the promoter) to AscI site (bold; immediately downstream of the NOS terminator). PDISP-HA0 of H3 A/Brisbane/10/2007 (underlined). **Figure ID** shows the nucleotide sequence of expression cassette 973 (SEQ ID NO:4), comprising a sequence from PacI site (bold; upstream of the promoter) to AscI site (bold; immediately downstream of the NOS terminator). PDISP-HA0 of B/Florida/4/2006 (underlined). **Figure IE** shows the nucleotide sequence of LIR-MCS-SIR+LIR (SEQ ID NO:9). LIR (long intergenic region)- MCS (Multiple cloning site)- short intergenic region (SIR)+LIR sequence of 849 construct. **Figure 1F** shows the nucleotide sequence of expression cassette 853 (SEQ ID NO:10), comprising a sequence from AscI site (bold; upstream of the LIR) to PmeI site (bold; downstream of the SIR+LIR). PDISP-HA0 of B/Florida/4/2006 (underlined). **Figure 1G** shows the nucleotide sequence of expression cassette 561 (SEQ ID NO:11) comprising a sequence from AscI site (bold; upstream of the LIR) to PmeI site (bold; downstream of the SIR+LIR). PDISP-HA0 of H1 A/California/4/2009 (underlined). **Figure 1H** shows the nucleotide sequence of expression cassette 851 (SEQ ID NO:12) comprising a sequence from AscI site (upstream of the LIR) to PmeI site (downstream of the SIR+LIR). PDISP-HA0 of H3 A/Brisbane/10/2007 (underlined). **Figure 1I** shows the nucleotide sequence of expression cassette 852 (SEQ ID NO: 13) comprising a sequence from AscI site (upstream of the LIR) to PmeI site (downstream of the SIR+LIR). Native Sp with HA0 from H5 A/Indonesia/05/2005 (underlined). **Figure 1J** shows the nucleotide sequence (SEQ ID NO:14) of LIR-MCS-SIR-*Replicase*-LIR sequence of 848 construct. **Figure 1K** shows the nucleotide sequence of expression cassette 475 (SEQ ID NO:15) comprising nucleotides from AscI site (bold; upstream of the LIR) to PmeI site (bold; downstream of the SIR-C1/C2-LIR). PDISP-HA0 of B/Florida/4/2006 (underlined). **Figure 1L** shows the nucleotide sequence of expression cassette 471 (SEQ ID NO:16) comprising nucleotides from AscI site (bold; upstream of the LIR) to PmeI site (bold; downstream of the SIR-C1/C2-LIR). PDISP-HA0 of H1 A/California/4/2009 (underlined). **Figure 1M** shows the nucleotide sequence of expression cassette 473 (SEQ ID NO:17) comprising nucleotides from AscI site (bold; upstream of the LIR) to PmeI site (bold; downstream of the SIR-C1/C2-LIR). PDISP-HA0 of H3 A/Brisbane/10/2007 (underlined). **Figure 1N** shows the nucleotide sequence of expression cassette 474 (SEQ ID NO:18) from AscI site (bold; upstream of the LIR) to PmeI site (bold; downstream of the SIR-C1/C2-LIR). Native Sp with HA0 from H5 A/Indonesia/05/2005 (underlined).

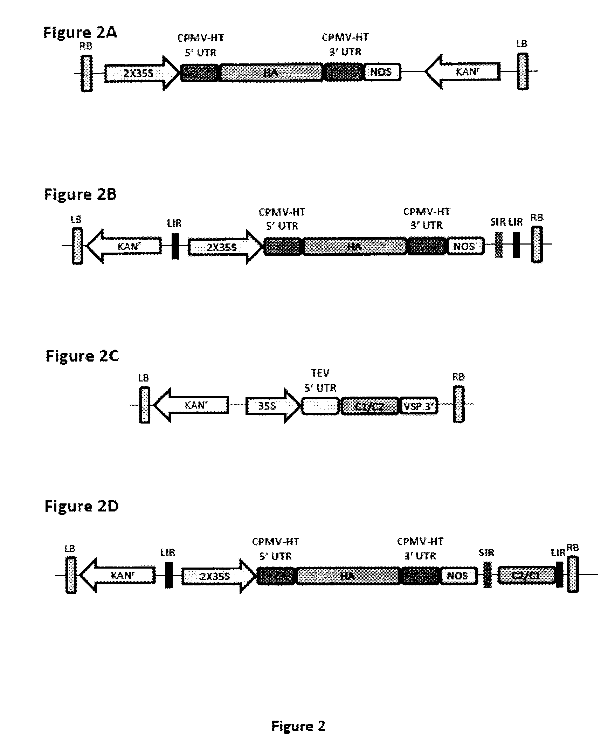

**Figure 2** shows schematic representation of several constructs that can be used for transient expression of HA in *Nicotiana benthamiana.* **Figure 2A** shows a schematic construct for CPMV-HT-based constructs for HA expression (constructs number 971, 972, 973 and 560). **Figure 2B** shows a schematic construct CPMV-HT + BeYDV-based construct for HA expression (constructs number 851, 852, 853 and 561). **Figure 2C** shows a schematic construct for BeYDV replicase expression construct (construct number 834). **Figure 2D** shows a schematic construct for CPMV-HT + BeYDV-based construct comprising the replicase gene (C1/C2) under the control of LIR for HA expression (constructs number 471, 473, 474 and 475).

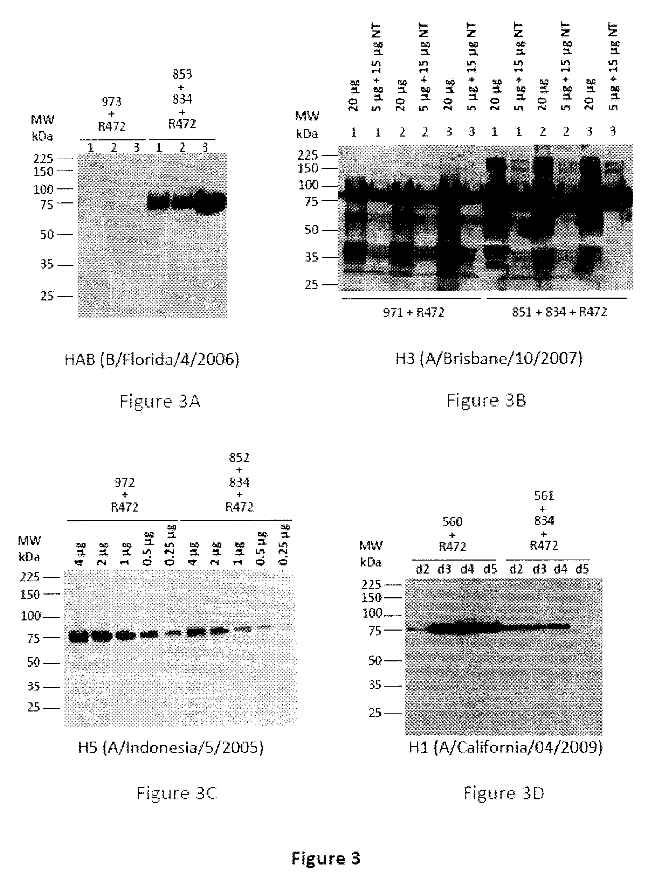

**Figure 3** shows a comparison of transient HA accumulation using CPMV-HT-based expression system alone or a CPMV-HT-based expression system in conjunction with the BeYDV amplification system. **Figure 3A** shows a comparison of HAB (from strain B/Florida/4/2006) accumulation in plants using a CPMV-HT-based expression cassette (construct number 973; see Figures 1D and 2A), or in conjunction with a BeYDV amplification system (constructs number 853 and 834 (providing expression of viral replicase); see Figures 1F and 2B). Three plants were analyzed for each construct (1, 2 and 3). Twenty micrograms of proteins were loaded for each extract analyzed. **Figure 3B** shows a comparison of H3 (from strain A/Brisbane/10/2007) accumulation in plants using a CPMV-HT-based expression cassette alone (construct number 971; see Figures 1C and 2A) or in conjunction with a BeYDV amplification system (construct number 851; see Figures 1H and 2B). Three plants were analyzed for each construct (1, 2 and 3).Twenty micrograms or five micrograms of proteins from transformed plants were loaded as indicated. Fifteen micrograms of proteins from non-transformed plants were added to the 5 µg loads. **Figure 3C** shows a comparison of H5 (from strain A/Indonesia/05/05) accumulation in plants using a CPMV-HT-based expression cassette alone (construct number 972; see Figures 1A and Figure 2A) or in conjunction with a BeYDV amplification system (construct number 852 see Figures 1I and 2B). Leaves of three plants were pooled prior to extraction. The amount of proteins from transformed plants is indicated for each lane. To ensure equal loading, the extracts from transformed plants were completed to 4 µg with protein extract from non-transformed plants. **Figure 3D** shows a comparison of H1 (from strain A/California/04/2009) accumulation in plants using a CPMV-HT-based expression cassette alone (construct number 560; see Figures 1B and 2A) or in conjunction with a BeYDV amplification system (construct number 561; see Figures 1G and 2B). For each construct, three plants were harvested on days 2, 3, 4 and 5 post infiltration (respectively indicated as d2, d3, d4 and d5). Leaves of three plants were pooled prior to extraction. Five micrograms of proteins from transformed plants were mixed with 15 µg of proteins from non-transformed plants prior to loading.

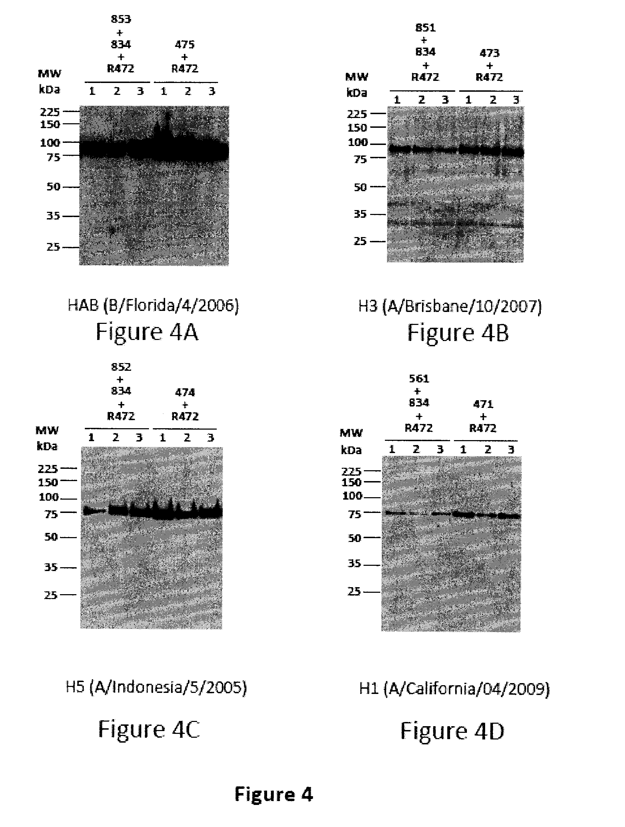

**Figure 4** shows a comparison of transient HA accumulation in plants using several CPMV-HT-based expression cassettes in conjunction with a BeYDV amplification system and a replicase provided on a separate plasmid, or on the same plasmid under the control of the LIR. **Figure 4A** shows a comparison of HA (from strain B/Florida/4/2006) accumulation in plants using the CPMV-HT-based expression cassette in conjunction with a BeYDV amplification system (construct number 853; see Figures 1F and 2B; and Plastocyanin-P19; construct number 472) with a replicase gene on a separate plasmid, or on the same plasmid under the control of the LIR (construct number 475; see Figures 1K and 2D; and Plastocyanin-P19; construct number 472). Three plants were analyzed for each construct (1, 2 and 3). Twenty micrograms of proteins were loaded for each extract analyzed. **Figure 4B** shows a comparison of H3 (from strain A/Brisbane/10/2007) accumulation in plants using the CPMV-HT-based expression cassette in conjunction with the BeYDV amplification system (construct number 851; see Figures 1H and 2B; and Plastocyanin-P19; construct number 472) with the replicase gene on a separate plasmid, or on the same plasmid under the control of the LIR (construct number 473; see Figures 1M and 2D; and Plastocyanin-P19; construct number 472). Three plants were analyzed for each construct (1,2 and 3). Half a microgram of proteins from transformed plants was mixed with 10 µg of proteins from non-transformed plants prior to loading. **Figure 4C** shows a comparison of H5 (from strain A/Indonesia/05/05) accumulation in plants using the CPMV-HT-based expression cassette in conjunction with the BeYDV amplification system (construct number 852; see Figures 1I and 2B; and Plastocyanin-P19; construct number 472) with the replicase gene on a separate plasmid, or on the same plasmid under the control of the LIR (construct number 474; see Figures 1N and 2D; and Plastocyanin-P19; construct number 472). Three plants were analyzed for each construct (1, 2 and 3). Half a microgram of proteins from transformed plants was mixed with 10 µg of proteins from non-transformed plants prior to loading. **Figure 4D** shows a comparison of H1 (from strain A/California/04/2009) accumulation in plants using the CPMV-HT-based expression cassette in conjunction with the BeYDV amplification system (construct number 561; see Figure 1G and 2B; and Plastocyanin-P19; construct number 472) with the replicase gene on a separate plasmid, or on the same plasmid under the control of the LIR (construct number 471; see Figure 1L and 2D; and Plastocyanin-P19; construct number 472). Three plants were analyzed for each construct (1, 2 and 3). Two and a half micrograms of proteins from transformed plants were mixed with 10 µg of proteins from non-transformed plants prior to loading.

### DETAILED DESCRIPTION

The present invention relates to nucleic acids and expression systems to produce proteins of interest in plants. The expression systems comprise a comovirus-based expression cassette and a geminivirus-based amplification element. Plant cells, plant tissues, whole plants, inoculum, nucleic acids (e.g. gene constructs), comprising the proteins of interest, and methods of expressing protein of interest in plants are also provided.

The present invention further provides a plant expression system comprising a first nucleic acid sequence comprising a regulatory region, operatively linked with a one or more than one comovirus enhancer, a nucleotide sequence of interest, and one or more than one geminivirus amplification element, and a second nucleic acid encoding a geminivirus replicase. The first nucleic acid sequence and the second nucleic acid sequence may be located on the same nucleic acid molecule, or they may be located on different DNA molecules. For example, the plant expression system may comprise a nucleic acid sequence comprising a regulatory sequence operatively linked to one or more than one comovirus enhancer; a nucleotide sequence of interest; one or more than one geminivirus amplification element, and a nucleic acid sequence encoding a geminivirus replicase protein.

The present invention also provides a nucleic acid comprising a promoter (regulatory region) sequence, a comovirus regulatory region, one or more sequences encoding one or more proteins of interest and a geminivirus amplification element, provided on the same or a different nucleic acid. The nucleic acid may further comprising a sequence encoding a geminivirus replicase, a comovirus 3' untranlsated region (UTR) or a plastocyanin 3' UTR, or a combination of a geminivirus replicase and a comovirus 3' UTR or a plastocyanin 3' UTR.

In the description that follows, a number of terms are used extensively, the following definitions are provided to facilitate understanding of various aspects of the invention. Use of examples in the specification, including examples of terms, is for illustrative purposes only and is not intended to limit the scope and meaning of the embodiments of the invention herein.

The terms "polypeptide", peptide" and "protein" are used interchangeably herein to refer to a polymer of amino acid residues. The terms apply to naturally occurring amino acid polymers as well as amino acid polymers in which one or more amino acid residues is a non-naturally occurring amino acid, e.g., an amino acid analog. As used herein, the terms encompass amino acid chains of any length, including full length proteins for example antigens, wherein the amino acid residues are linked by covalent peptide bonds.

As used herein, the term "derivative" in the context of a polypeptide or protein, e.g. an antibody, refers to a polypeptide or protein that comprises an amino acid sequence which has been altered by the introduction of amino acid residue substitutions, deletions or additions. The term "derivative" as used herein also refers to a polypeptide or protein which has been modified, i.e., by the covalent attachment of any type of molecule to the antibody. For example, a polypeptide or protein may be modified, e.g., by glycosylation, acetylation, pegylation, phosphorylation, amidation, derivatization by known protecting/blocking groups, proteolytic cleavage, linkage to a cellular ligand or other protein, etc. Derivatives, polypeptides or proteins may be produced by chemical modifications using techniques known to those of skill in the art, including, but not limited to specific chemical cleavage, acetylation, formylation, metabolic synthesis of tunicamycin, etc. In some embodiments a derivative, a polypeptide or protein possesses a similar or identical function as the polypeptide or protein from which it was derived.

The expression system as described herein comprises an expression cassette based on a bipartite virus, or virus with a bipartite genome. For example, the present bipartite viruses may be of the Comoviridae family. Genera of the Comoviridae family include Comovirus, Nepovirus, Fabavirus, Cheravirus and Sadwavirus. Comoviruses include Cowpea mosaic virus (CPMV), Cowpea severe mosaic virus (CPSMV), Squash mosaic virus (SqMV), Red clover mottle virus (RCMV), Bean pod mottle virus (BPMV), Turnip ringspot virus (TuRSV), Broad bean true mosaic virus (BBtMV), Broad bean stain virus (BBSV), Radish mosaic virus (RaMV). Examples of comoviruse RNA-2 sequences comprising enhancer elements that may be useful for various aspects of the invention include, but are not limited to: CPMV RNA-2 (GenBank Accession No. NC_003550), RCMV RNA-2 (GenBank Accession No. NC_003738), BPMV RNA-2 (GenBank Accession No. NC_003495), CPSMV RNA-2 (GenBank Accession No.NC_003544), SqMV RNA-2 (GenBank Accession No.NC_003800), TuRSV RNA-2 (GenBank Accession No. NC_013219.1). BBtMV RNA-2 (GenBank Accession No. GU810904), BBSV RNA2 (GenBank Accession No. FJ028650), RaMV (GenBank Accession No. NC_003800)

Segments of the bipartite comoviral RNA genome are referred to as RNA-1 and RNA-2. RNA-1 encodes the proteins involved in replication while RNA-2 encodes the proteins necessary for cell-to-cell movement and the two capsid proteins. Any suitable comovirus-based cassette may be used including CPMV, CPSMV, SqMV, RCMV, or BPMV, for example, the expression cassette may be based on CPMV.

"Expression cassette" refers to a nucleotide sequence comprising a nucleic acid of interest under the control of, and operably (or operatively) linked to, an appropriate promoter or other regulatory elements for transcription of the nucleic acid of interest in a host cell.

It has been shown that transformation of *Nicotiana benthamiana* with full-length, replication-competent cDNA copies of both genomic RNAs of CPMV can result in a productive infection (Liu et al., 2004, Virology 323, 37-48). Examples of CPMV-based expression cassettes are described in WO2007/135480; WO2009/087391; and Sainsbury F. et al., (2008, Plant Physiology; 148: 1212-1218; Sainsbury F. et al., (2008, Plant Biotechnology Journal; 6: 82-92; Sainsbury F. et al., 2009, Plant Biotechnology Journal; 7: 682-693). As an example, which is not to be considered limiting, the untranslated regions (UTRs) obtained from the genomic RNA 2 of the cowpea mosaic virus (CMV) in which the two first translation initiation codons found in the 5'leader sequence have been deleted, may be used as described in WO 2009/087391. When combined to the CaMV 35S promoter and the nopaline synthase (NOS) terminator, the modified CPMV UTRs enhanced translation of the flanking coding region. The CPMV-based expression system was named CPMV-HT (hyperanslatable).

As described herein, an expression enhancer sequence, which sequence is derived from (or shares homology with) the RNA-2 genome segment of a bipartite RNA virus, such as a comovirus, in which a target initiation site has been mutated, may be used for expressing a nucleic acid sequence of interest. The present invention further provides processes for increasing the expression, or translational enhancing activity, of a sequence derived from an RNA-2 genome segment of a bipartite virus, which processes comprise mutating a target initiation site therein.

"Enhancer" sequences (or enhancer elements), include sequences derived from (or sharing homology with) the RNA-2 genome segment of a bipartite RNA virus, such as a comovirus, in which a target initiation site has been mutated. Such sequences can enhance downstream expression of a heterologous ORF to which they are attached. Without limitation, it is believed that such sequences when present in transcribed RNA, can enhance translation of a heterologous ORF to which they are attached.

A "target initiation site" as referred to herein and in the context of an enhancer sequence, is the initiation site (start codon) of a wild- type RNA-2 genome segment of a bipartite virus, for example a comovirus, from which the enhancer sequence in question is derived. The target initiation site serves as the initiation site for the production (translation) of the 105K protein, which is the longer of two carboxy coterminal proteins encoded by the wild-type RNA-2 genome segment. Production of the 105K protein, is initiated at the initiation site at position 161 in the wild-type CPMV RNA-2 genome segment. Thus, a target initiation site in an enhancer sequence derived from the CPMV RNA-2 genome segment may be the initiation site at position 161 in the wild-type CPMV RNA-2. Mutations around the start codon at position 161 may have the same, or similar, effect as mutating the start codon at position 161 itself by, for example, disrupting the context around the start codon may mean that the start codon is by-passed more frequently.

The enhancer sequence may comprise an indirectly mutated target initiation site produced by mutating one or more nucleotides upstream and/or downstream of the target initiation site, but retaining the wild-type target initiation site. The effect of mutating the upstream, downstream, or both an upstream and downstream nucleotides, is the same, or similar, to the effect observed when the target initiation site itself is mutated.

As target initiation sites serve as the initiation site for the production of the longer of two carboxy coterminal proteins encoded by a wild-type RNA-2 genome segment, it follows that target initiation sites are in-frame (in phase) with a second initiation site on the same wild-type RNA-2 genome segment, which serves as the initiation site for the production of the shorter of two carboxy coterminal proteins encoded by the wild-type RNA-2. Two initiation sites are in-frame if they are in the same triplet reading frame. For example, the target initiation site in an enhancer sequence derived from the wild-type CPMV RNA- 2 genome segment (the initiation site at position 161), is in frame with the initiation site at position 512, which serves as the initiation site for the production of the shorter of the two carboxy coterminal proteins encoded by CPMV RNA-2 (the 95K protein) in the wild-type CPMV RNA-2 genome segment.

Thus, a target initiation site that is located upstream (5') of a second initiation site in the wild-type RNA-2 genome segment from which the enhancer sequence is derived, may serve as the initiation site for the production of the shorter of two carboxy coterminal polyproteins encoded by the wild-type RNA-2 genome segment. In addition, a target initiation site may also be located downstream (3') of a third initiation site in the wild-type RNA-2 genome from which the enhancer sequence is derived. In CPMV, the target initiation site at position 161, is located upstream of a second initiation site at position 512 which serves as the initiation site for the production of the 95K protein. A third initiation site at position 115 is also present. A target initiation site in an enhancer sequence derived from the RNA-2 genome segment of a bipartite virus is therefore the first of two initiation sites for the production of two carboxy coterminal proteins encoded by the wild-type RNA-2. 'First' in this context refers to the initiation site located closer to the 5' end of the wild-type RNA-2 genome segment.

More than one initiation site in the sequence may be mutated, if desired. For example the 'third' initiation site at (or corresponding to) position 115 may also be deleted or altered. It has been shown that removal of AUG 115 in addition to the removal of AUG 161 further enhances expression (Sainsbury and Lomonossoff, 2008, Plant Physiology; 148: 1212-1218). For example, the enhancer sequences of the present invention may be based on modified sequences from the RNA-2 genome segments of bipartite RNA viruses.

The sequences of the RNA-2 genome segments of these comoviruses and several specific strains are available from the NCBI database under the accession numbers listed in brackets: cowpea mosaic virus RNA-2 (NC_003550), cowpea severe mosaic virus RNA-2 (NC_003544), squash mosaic virus RNA-2 (NC_003800), squash mosaic virus strain Kimble RNA-2 (AF059533), squash mosaic virus strain Arizona RNA-2 (AF059532), red clover mottle virus RNA-2 (NC_003738), bean pod mottle virus RNA-2 (NC_003495), bean pod mottle virus strain K-Hopkins1 RNA-2 (AF394609), bean pod mottle virus strain K-Hancock1 RNA-2 (AF394607), Andean potato mottle virus (APMoV: L16239) and Radish mosaic virus (RaMV; AB295644). There are also partial RNA-2 sequences available from bean rugose mosaic virus (BRMV; AF263548) and a tentative member of the genus Comovirus, turnip ringspot virus (EF191015). Numerous sequences from the other genera in the family Comoviridae are also available. To date, all comoviruses which have been investigated have been shown to have two alternative start codons for the expression of two carboxy coterminal polyproteins form their RNA-2 genome segments. In particular, the RNA-2 genome segments of CPMV, CPSMV, BPMV, SqMV and RCMV are known to comprise two alternative start codons for the expression of two carboxy coterminal polyproteins.

Target initiation sites in other comoviruses, which are equivalent to the initiation site at position 161 in the wild-type RNA-2 segment of CPMV can therefore be identified by methods as known in the art. For example, a sequence of interest may be aligned, using standard methods of sequence alignment such as BLAST, or by manual alignment, with that of CMPV to determine the location of a corresponding, equivalent initiation site.

As described herein, the enhancer sequence may comprise for example, nucleotides 1 to 512, or 1-509 of the CPMV RNA-2 genome segment, wherein the target initiation site at position 161 has been mutated. Alternatively, the enhancer sequence may comprise an equivalent sequence from another comovirus, wherein the target initiation site equivalent to the start codon at position 161 of CPMV has been mutated. The target initiation site may be mutated by substitution, deletion or insertion. For example, the target initiation site may be mutated by a point mutation. The CPMV enhancer may comprise SEQ NO: 23 as set forth below (mutated ATGs - ATG 115 and 161 - are underlined):.

The present enhancer sequence may have at least 99%, 98%, 97%, 96%, 95%, 90%, 85%, 80%, 75%, 70%, 65%, 60%, 55%, or 50% identity to the CPMV RNA-2 sequence, or any amount therebetween, wherein the target initiation site corresponding to position 161 of the wild-type CPMV RNA-2 genome segment has been mutated by substitution, deletion or insertion. For example the enhancer sequence may have from about 80% to about 99%, from about 90% to about 99%, or about 95% to about 99%, identity to the CPMV RNA-2 sequence, or any amount therebetween, wherein the target initiation site corresponding to position 161 of the wild-type CPMV RNA-2 genome segment has been mutated by substitution, deletion or insertion.

The terms "percent similarity", "percent identity" and "percent homology" when referring to a particular sequence are used for example as set forth in the University of Wisconsin GCG software program. Methods of alignment of sequences for comparison are well-known in the art. Optimal alignment of sequences for comparison can be conducted, using for example the algorithm of Smith & Waterman, (1981, Adv. Appl. Math. 2:482), by the alignment algorithm of Needleman & Wunsch, (1970, J. Mol. Biol. 48:443), by the search for similarity method of Pearson & Lipman, (1988, Proc. Nat'l. Acad. Sci. USA 85:2444), by computerized implementations of these algorithms (for example: GAP, BESTFIT, FASTA, and TFASTA in the Wisconsin Genetics Software Package, Genetics Computer Group (GCG), 575 Science Dr., Madison, Wis.), or by manual alignment and visual inspection (see, e.g., Current Protocols in Molecular Biology, Ausubel et al., eds. 1995 supplement).

An example of an algorithm suitable for determining percent sequence identity and sequence similarity are the BLAST and BLAST 2.0 algorithms, which are described in Altschul et al., (1977, Nuc. Acids Res. 25:3389-3402) and Altschul et al., (1990, J. Mol. Biol. 215:403-410 ), respectively. BLAST and BLAST 2.0 are used, with the parameters described herein, to determine percent sequence identity for the nucleic acids and proteins of the invention. For example the BLASTN program (for nucleotide sequences) may use as defaults a wordlength (W) of 11, an expectation (E) of 10, M=5, N=-4 and a comparison of both strands. For amino acid sequences, the BLASTP program may use as defaults a wordlength of 3, and expectation (E) of 10, and the BLOSUM62 scoring matrix (see Henikoff & Henikoff, 1989, Proc. Natl. Acad. Sci. USA 89:10915) alignments (B) of 50, expectation (E) of 10, M=5, N=-4, and a comparison of both strands. Software for performing BLAST analyses is publicly available through the National Center for Biotechnology Information (see URL: ncbi.nlm.nih.gov/).

Enhancer sequences may also hybridise, under stringent conditions, with the complementary sequence of the CPMV RNA-2, with the proviso that the target initiation site corresponding to position 161 in the wild-type CPMV RNA-2 genome segment has been mutated.

Hybridization under stringent hybridization conditions is known in the art (see for example Current Protocols in Molecular Biology, Ausubel et al., eds. 1995 and supplements; Maniatis et al., in Molecular Cloning (A Laboratory Manual), Cold Spring Harbor Laboratory, 1982; Sambrook and Russell, in Molecular Cloning: A Laboratory Manual, 3rd edition 2001). An example of one such stringent hybridization conditions may be about 16-20 hours hybridization in 4 X SSC at 65°C, followed by washing in 0.1 X SSC at 65°C for an hour, or 2 washes in 0.1 X SSC at 65°C each for 20 or 30 minutes. Alternatively, an exemplary stringent hybridization condition could be overnight (16-20 hours) in 50% formamide, 4 X SSC at 42°C, followed by washing in 0.1 X SSC at 65°C for an hour, or 2 washes in 0.1 X SSC at 65°C each for 20 or 30 minutes, or overnight (16-20 hours), or hybridization in Church aqueous phosphate buffer (7% SDS; 0.5M NaPO₄ buffer pH 7.2; 10 mM EDTA) at 65°C, with 2 washes either at 50°C in 0.1 X SSC, 0.1% SDS for 20 or 30 minutes each, or 2 washes at 65°C in 2 X SSC, 0.1% SDS for 20 or 30 minutes each.

A target initiation site in an enhancer sequence of the invention may be mutated by deletion, insertion or substitution, such that it no longer functions as a translation initiation site. For example, a point mutation may be made at the position of the target initiation site in the enhancer sequence. Alternatively, the target initiation site in the enhancer sequence may be deleted either partially or in its entirety. For example, a deletion spanning the target initiation site in the enhancer sequence may be made. Deletions spanning the initiation site may be about 5, 10, 15, 20, 25, 30, 35, 40, 45, or 50 nucleotides in length, when compared with the sequence of the wild-type RNA-2 genome segment from which the enhancer sequence is derived, with the proviso that the deletion span the initiation site.

Without wishing to be bound by theory, mutation of the start codon at position 161 in CPMV is thought to lead to inactivation of a translational suppressor, which results in enhanced initiation of translation from start codons located downstream of the inactivated translational suppressor. Thus, the enhancer sequence for use as described herein may be derived from an RNA-2 genome segment of a bipartite virus, wherein the enhancer sequence comprises an inactivated translational suppressor sequence.

A translational suppressor sequence may be a sequence in the wild-type RNA-2 genome segment of the bipartite virus (e.g. a comovirus) from which the enhancer sequence in question is derived, which comprises, or consists of, the initiation site for the production (translation) of the longer of two carboxy coterminal proteins encoded by the wild-type RNA-2 genome segment. Translational suppressor sequences in enhancer sequences derived from the CPMV RNA-2 genome segment, are sequences comprising, or consisting of, the target initiation site described above. Thus, translational suppressor sequences comprise, or consist of, a target initiation site as defined above, and may be inactivated by mutagenesis as described above.

The present disclosure provides an isolated nucleic acid comprising an expression enhancer sequence as described above. "Nucleic acid" or a "nucleic acid molecule" as used herein refers to any DNA or RNA molecule, either single or double stranded and, if single stranded, the molecule of its complementary sequence in either linear or circular form. In discussing nucleic acid molecules, a sequence or structure of a particular nucleic acid molecule may be described herein according to the normal convention of providing the sequence in the 5' to 3' direction. With reference to nucleic acids of the invention, the term "isolated nucleic acid" is sometimes used. This term, when applied to DNA, refers to a DNA molecule that is separated from sequences with which it is immediately contiguous in the naturally occurring genome of the organism in which it originated. For example, an "isolated nucleic acid" may comprise a DNA molecule inserted into a vector, such as a plasmid or virus vector, or integrated into the genomic DNA of a prokaryotic or eukaryotic cell or host organism. When applied to RNA, the term "isolated nucleic acid" refers primarily to an RNA molecule encoded by an isolated DNA molecule as defined above. Alternatively, the term may refer to an RNA molecule that has been sufficiently separated from other nucleic acids with which it would be associated in its natural state (i.e., in cells or tissues). An "isolated nucleic acid" (either DNA or RNA) may further represent a molecule produced directly by biological or synthetic means and separated from other components present during its production.

The nucleic acid may thus comprise a portion, or fragment, of the RNA-2 genome segment of the bipartite RNA virus from which the enhancer is derived. For example, the nucleic acid may not comprise at least a portion of the coding region of the RNA-2 genome segment from which it is derived. The coding region may be the region of the RNA-2 genome segment encoding the shorter of two carboxy coterminal proteins. The nucleic acid may comprise the portion of an RNA-2 genome segment of a bipartite virus extending from the 5' end of the wild-type RNA-2 genome segment to the initiation site from which production (translation) of the shorter of two carboxy coterminal proteins encoded by the wild-type RNA-2 genome segment is initiated.

As described herein, there is provided one or more expression systems comprising an enhancer sequence as described above. The expression system may also comprise a nucleic acid of interest encoding a protein of interest inserted downstream of the enhancer sequence.
By "gene of interest", "nucleotide (or nucleic acid) sequence of interest", or "coding region of interest", it is meant any gene, nucleotide sequence, or coding region (these terms are used interchangeably) that is to be expressed within a host organism, for example a plant, and may produce a protein of interest. Such a nucleotide sequence of interest may include, but is not limited to, a gene or coding region whose product is an industrial enzyme, a protein supplement, a nutraceutical, a value-added product, or a fragment thereof for feed, food, or both feed and food use. A nucleotide sequence, or coding region of interest may also include a gene that encodes a pharmaceutically active protein, for example growth factors, growth regulators, antibodies, antigens, and fragments thereof, or their derivatives useful for immunization or vaccination and the like. Such proteins include, but are not limited to, interleukins, for example one or more than one of IL-1 to IL-24, IL-26 and IL-27, cytokines, Erythropoietin (EPO), insulin, G-CSF, GM-CSF, hPG-CSF, M-CSF or combinations thereof, interferons, for example, interferon-alpha, interferon-beta, interferon-gama, blood clotting factors, for example, Factor VIII, Factor IX, or tPA hGH, receptors, receptor agonists, antibodies, neuropolypeptides, insulin, vaccines, growth factors for example but not limited to epidermal growth factor, keratinocyte growth factor, transformation growth factor, growth regulators, antigens, autoantigens, fragments thereof, or combinations thereof.
The protein of interest may also include an influenza hemagglutinin (HA; see WO 2009/009876). HA is a homotrimeric membrane type I glycoprotein, generally comprising a signal peptide, an HA1 domain, and an HA2 domain comprising a membrane-spanning anchor site at the C-terminus and a small cytoplasmic tail. Nucleotide sequences encoding HA are well known and are available (see, for example, the BioDefense and Public Health Database (now Influenza Research Database; Squires et al., 2008 Nucleic Acids Research 36:D497-D503) at URL: biohealthbase.org/GSearch/home.do?decorator=Influenza; or the databases maintained by the National Center for Biotechnology Information (see URL: ncbi.nlm.nih.gov).

Disclosed are HA proteins from a type A influenza, a type B influenza, or a subtype of type A influenza HA selected from the group of H1, H2, H3, H4, H5, H6, H7, H8, H9, H10, H11, H12, H13, H14, H15, and H16. The HA may be from a type A influenza, selected from the group H1, H2, H3, H5, H6, H7 and H9. Fragments of the HAs listed above may also be considered a protein of interest. Furthermore, domains from an HA type or subtype listed above may be combined to produce chimeric HA's (see for example WO2009/076778) Examples of subtypes comprising HA proteins include A/New Caledonia/20/99 (H1N1), A/Indonesia/5/2006 (H5N1), A/chicken/New York/1995, A/herring gull/DE/677/88 (H2N8), A/Texas/32/2003, A/mallard/MN/33/00, A/duck/Shanghai/1/2000, A/northern pintail/TX/828189/02, A/Turkey/Ontario/6118/68(H8N4), A/shoveler/Iran/G54/03, A/chicken/Germany/N/1949(H10N7), A/duck/England/56(H11N6), A/duck/Alberta/60/76(H12N5), A/Gull/Maryland/704/77(H13N6), A/Mallard/Gurjev/263/82, A/duck/Australia/341/83 (H15N8), A/black-headed gull/Sweden/5/99(H16N3), B/Lee/40, C/Johannesburg/66, A/PuertoRico/8/34 (H1N1), A/Brisbane/59/2007 (H1N1), A/Solomon Islands 3/2006 (H1N1), A/Brisbane 10/2007 (H3N2), A/Wisconsin/67/2005 (H3N2), B/Malaysia/2506/2004, B/Florida/4/2006, A/Singapore/1/57 (H2N2), A/Anhui/1/2005 (H5N1), A/Vietnam/1194/2004 (HSN1), A/Teal/HongKong/W312/97 (H6N1), A/Equine/Prague/56 (H7N7), A/HongKong/1073/99 (H9N2)).
The HA protein may be an H1, H2, H3, H5, H6, H7 or H9 subtype. For example, the H1 protein may be from the A/New Caledonia/20/99 (H1N1), A/PuertoRico/8/34 (H1N1), A/Brisbane/59/2007 (H1N1), A/Solomon Islands 3/2006 (H1N1), A/California/04/2009 (H1N1) or A/California/07/2009 (H1N1) strain. The H3 protein may also be from the A/Brisbane 10/2007 (H3N2), A/Wisconsin/67/2005 (H3N2) or A/Perth/16/2009 (H3N2) strain. In a further aspect of the invention, the H2 protein may be from the A/Singapore/1/57 (H2N2) strain. The H5 protein may be from the A/Anhui/1/2005 (H5N1), A/Vietnam/1194/2004 (H5N1), or A/Indonesia/5/2005 strain. In an aspect of the invention, the H6 protein may be from the A/Teal/HongKong/W312/97 (H6N1) strain. The H7 protein may be from the A/Equine/Prague/56 (H7N7) strain. In an aspect of the invention, the H9 protein is from the A/HongKong/1073/99 (H9N2) strain. In a further aspect of the invention, the HA protein may be from an influenza virus may be a type B virus, including B/Malaysia/2506/2004, B/Florida/4/2006 or B/Brisbane/60/08. Examples of amino acid sequences of the HA proteins from H1, H2, H3, H5, H6, H7, H9 or B subtypes include sequences as described in WO 2009/009876, WO 2009/076778, WO 2010/003225). The influenza virus HA protein may be H5 Indonesia.

The HA may comprise a native, or a non-native signal peptide; the non-native signal peptide may be of plant origin. For example, the signal peptide may be a protein disulfide isomerase signal peptide. The native signal peptide may correspond to that of the hemagglutinin being expressed, or may correspond to a second hemagglutinin.
Disclosed are nucleic acid molecules comprising sequences encoding an HA protein. The nucleic acid molecules may further comprise one or more regulatory regions operatively linked to the sequence encoding an HA protein. The nucleic acid molecules may comprise a sequence encoding an H1, H2, H3, H4, H5, H6, H7, H8, H9, H10, H11, H12, H13, H14, H15, H16 or HA from type B influenza. For example, the HA protein encoded by the nucleic acid molecule may be an H1, H2, H3, H5, H6, H7, H9 subtype an HA from type B. The H1 protein encoded by the nucleic acid molecule may be from the A/New Caledonia/20/99 (H1N1), A/PuertoRico/8/34 (H1N1), A/Brisbane/59/2007 (H1N1), A/Solomon Islands 3/2006 (H1N1), A/California/04/2009 (H1N1) or A/California/07/2009 (H1N1) strain. The H3 protein encoded by the nucleic acid molecule may be from the A/Brisbane 10/2007 (H3N2), A/Wisconsin/67/2005 (H3N2) or A/Perth/16/2009 (H3N2) strain. The H2 protein encoded by the nucleic acid molecule may be from the A/Singapore/1/57 (H2N2) strain. The H5 protein encoded by the nucleic acid molecule may also be from the A/Anhui/1/2005 (H5N1), A/Vietnam/1194/2004 (H5N1), or A/Indonesia/5/2005 strain. The H6 protein encoded by the nucleic acid molecule may be from the A/Teal/HongKong/W312/97 (H6N1) strain. The H7 protein encoded by the nucleic acid molecule may also be from the A/Equine/Prague/56 (H7N7) strain. Additionally, the H9 protein encoded by the nucleic acid molecule may be from the A/HongKong/1073/99 (H9N2) strain. The HA protein from B type encoded by the nucleic acid may be from the B/Florida/4/2006, B/Malaysia/2506/2004 or B/Brisbane/60/08 strain. Examples of sequences of nucleic acid molecules encoding such HA proteins from H1, H2, H3, H5, H6, H7, H9 subtypes or B type include sequences as described in WO 2009/009876, WO 2009/076778, WO 2010/003225. The nucleic acid sequence may encode the influenza virus HA protein H5 Indonesia.
If the nucleic acid sequence of interest encodes a product that is directly or indirectly toxic to the plant, then such toxicity may be reduced by selectively expressing the nucleotide sequence of interest within a desired tissue or at a desired stage of plant development.
The coding region of interest or the nucleotide sequence of interest may be expressed in any suitable plant host which is either transformed or comprises the nucleotide sequences, or nucleic acid molecules, or genetic constructs, or vectors of the present invention. Examples of suitable hosts include, but are not limited to, *Arabidopsis,* agricultural crops including for example canola, *Brassica* spp., maize, *Nicotiana* spp., (tobacco) for example, *Nicotiana benthamiana,* alfalfa, potato, sweet potato *(Ipomoea batatars*), ginseng, pea, oat, rice, soybean, wheat, barley, sunflower, cotton, corn, rye *(Secale cereale),* sorghum (*Sorghum bicolor, Sorghum vulgare*), safflower (*Carthamus tinctorius*).

Therefore as described herein, there is provided an expression system comprising: (a) an enhancer sequence as described above; and (b) a nucleotide sequence encoding influenza type B hemagglutinin or influenza type A subtype H3 hemagglutinin, wherein the nucleotide sequence is located downstream of the enhancer sequence.
By "regulatory region" "regulatory element" or "promoter" it is meant a portion of nucleic acid typically, but not always, upstream of the protein coding region of a gene, which may be comprised of either DNA or RNA, or both DNA and RNA. When a regulatory region is active, and in operative association, or operatively linked, with a gene of interest, this may result in expression of the gene of interest. A regulatory element may be capable of mediating organ specificity, or controlling developmental or temporal gene activation. A "regulatory region" includes promoter elements, core promoter elements exhibiting a basal promoter activity, elements that are inducible in response to an external stimulus, elements that mediate promoter activity such as negative regulatory elements or transcriptional enhancers. "Regulatory region", as used herein, also includes elements that are active following transcription, for example, regulatory elements that modulate gene expression such as translational and transcriptional enhancers, translational and transcriptional repressors, upstream activating sequences, and mRNA instability determinants. Several of these latter elements may be located proximal to the coding region.
In the context of this disclosure, the term "regulatory element" or "regulatory region" typically refers to a sequence of DNA, usually, but not always, upstream (5') to the coding sequence of a structural gene, which controls the expression of the coding region by providing the recognition for RNA polymerase and/or other factors required for transcription to start at a particular site. However, it is to be understood that other nucleotide sequences, located within introns, or 3' of the sequence may also contribute to the regulation of expression of a coding region of interest. An example of a regulatory element that provides for the recognition for RNA polymerase or other transcriptional factors to ensure initiation at a particular site is a promoter element. Most, but not all, eukaryotic promoter elements contain a TATA box, a conserved nucleic acid sequence comprised of adenosine and thymidine nucleotide base pairs usually situated approximately 25 base pairs upstream of a transcriptional start site. A promoter element may comprise a basal promoter element, responsible for the initiation of transcription, as well as other regulatory elements (as listed above) that modify gene expression.
There are several types of regulatory regions, including those that are developmentally regulated, inducible or constitutive. A regulatory region that is developmentally regulated, or controls the differential expression of a gene under its control, is activated within certain organs or tissues of an organ at specific times during the development of that organ or tissue. However, some regulatory regions that are developmentally regulated may preferentially be active within certain organs or tissues at specific developmental stages, they may also be active in a developmentally regulated manner, or at a basal level in other organs or tissues within the plant as well. Examples of tissue-specific regulatory regions, for example see-specific a regulatory region, include the napin promoter, and the cruciferin promoter (Rask et al., 1998, J. Plant Physiol. 152: 595-599; Bilodeau et al., 1994, Plant Cell 14: 125-130). An example of a leaf-specific promoter includes the plastocyanin promoter (see US 7,125,978).
An inducible regulatory region is one that is capable of directly or indirectly activating transcription of one or more DNA sequences or genes in response to an inducer. In the absence of an inducer the DNA sequences or genes will not be transcribed. Typically the protein factor that binds specifically to an inducible regulatory region to activate transcription may be present in an inactive form, which is then directly or indirectly converted to the active form by the inducer. However, the protein factor may also be absent. The inducer can be a chemical agent such as a protein, metabolite, growth regulator, herbicide or phenolic compound or a physiological stress imposed directly by heat, cold, salt, or toxic elements or indirectly through the action of a pathogen or disease agent such as a virus. A plant cell containing an inducible regulatory region may be exposed to an inducer by externally applying the inducer to the cell or plant such as by spraying, watering, heating or similar methods. Inducible regulatory elements may be derived from either plant or non-plant genes (e.g. Gatz, C. and Lenk, I.R.P., 1998, Trends Plant Sci. 3, 352-358). Examples, of potential inducible promoters include, but not limited to, tetracycline-inducible promoter (Gatz, C.,1997, Ann. Rev. Plant Physiol. Plant Mol. Biol. 48, 89-108), steroid inducible promoter (Aoyama, T. and Chua, N.H.,1997, Plant J. 2, 397-404) and ethanol-inducible promoter (Salter, M.G., et al, 1998, Plant Journal 16, 127-132; Caddick, M.X., et al, 1998, Nature Biotech. 16, 177-180) cytokinin inducible IB6 and CKI1 genes (Brandstatter, I. and Kieber, J.J.,1998, Plant Cell 10, 1009-1019; Kakimoto, T., 1996, Science 274, 982-985) and the auxin inducible element, DR5 (Ulmasov, T., et al., 1997, Plant Cell 9, 1963-1971).
A constitutive regulatory region directs the expression of a gene throughout the various parts of a plant and continuously throughout plant development. Examples of known constitutive regulatory elements include promoters associated with the CaMV 35S transcript. (p35S; Odell et al., 1985, Nature, 313: 810-812), the rice actin 1 (Zhang et al, 1991, Plant Cell, 3: 1155-1165), actin 2 (An et al., 1996, Plant J., 10: 107-121), or tms 2 (U.S. 5,428,147), and triosephosphate isomerase 1 (Xu et. al., 1994, Plant Physiol. 106: 459-467) genes, the maize ubiquitin 1 gene (Cornejo et al, 1993, Plant Mol. Biol. 29: 637-646), the *Arabidopsis* ubiquitin 1 and 6 genes (Holtorf et al, 1995, Plant Mol. Biol. 29: 637-646), the tobacco translational initiation factor 4A gene (Mandel et al, 1995 Plant Mol. Biol. 29: 995-1004). the Cassava Vein Mosaic Virus promoter, pCAS, (Verdaguer et al., 1996); the promoter of the small subunit of ribulose biphosphate carboxylase, pRbcS: (Outchkourov et al., 2003), the pUbi (for monocots and dicots ).
As described herein, promoters comprising enhancer sequences with demonstrated efficiency in leaf expression, have been found to be effective in transient expression. Without wishing to be bound by theory, attachment of upstream regulatory elements of a photosynthetic gene by attachment to the nuclear matrix may mediate strong expression. For example up to -784 from the translation start site of the pea plastocyanin gene may be used mediate strong reporter gene expression.
The term "constitutive" as used herein does not necessarily indicate that a gene under control of the constitutive regulatory region is expressed at the same level in all cell types, but that the gene is expressed in a wide range of cell types even though variation in abundance is often observed.
By "operatively linked" it is meant that the particular sequences, for example a regulatory element and a coding region of interest, interact either directly or indirectly to carry out an intended function, such as mediation or modulation of gene expression. The interaction of operatively linked sequences may, for example, be mediated by proteins that interact with the operatively linked sequences.
The one or more than one nucleotide sequence of the present invention may be expressed in any suitable plant host that is transformed by the nucleotide sequence, or constructs, or vectors of the present invention. Examples of suitable hosts include, but are not limited to, *Arabidopsis,* agricultural crops including for example canola, *Brassica* spp., maize, *Nicotiana* spp., (tobacco) for example, *Nicotiana benthamiana,* alfalfa, potato, sweet potato *(Ipomoea batatars*), ginseng, pea, oat, rice, soybean, wheat, barley, sunflower, cotton, corn, rye *(Secale cereale),* sorghum (*Sorghum bicolor, Sorghum vulgare*), safflower (*Carthamus tinctorius*).
The one or more constructs of the present invention can further comprise a 3' untranslated region. A 3' untranslated region refers to that portion of a gene comprising a DNA segment that contains a polyadenylation signal and any other regulatory signals capable of effecting mRNA processing or gene expression. The polyadenylation signal is usually characterized by effecting the addition of polyadenylic acid tracks to the 3' end of the mRNA precursor. Polyadenylation signals are commonly recognized by the presence of homology to the canonical form 5' AATAAA-3' although variations are not uncommon. One or more of the chimeric genetic constructs of the present invention can also include further enhancers, either translation or transcription enhancers, as may be required. These enhancer regions are well known to persons skilled in the art, and can include the ATG initiation codon and adjacent sequences. The initiation codon must be in phase with the reading frame of the coding sequence to ensure translation of the entire sequence.

Non-limiting examples of suitable 3' regions are the 3' transcribed non-translated regions containing a polyadenylation signal of *Agrobacterium* tumor inducing (Ti) plasmid genes, such as the nopaline synthase (Nos gene) and plant genes such as the soybean storage protein genes, the small subunit of the ribulose-1, 5-bisphosphate carboxylase gene (ssRUBISCO; US 4,962,028), the promoter used in regulating plastocyanin expression (Pwee and Gray 1993). The termination (terminator) sequence may also be a termination sequence derived from the RNA-2 genome segment of a bipartite RNA virus, e.g. a comovirus. For example, the termination sequence may be derived from the same bipartite RNA virus from which the enhancer sequence is derived. The termination sequence may comprise a stop codon. Termination sequence may also be followed by polyadenylation signals.

Expression systems as described herein may be operably linked to promoter and terminator sequences. Thus, an expression systems may further comprise a termination sequence and the gene encoding a protein of interest may be located between the enhancer sequence and the termination sequence, i.e. downstream (3') of the enhancer sequence and upstream (5') of the termination sequence. Thus the disclosure further provides an expression cassette comprising: (i) a promoter, operably linked to (ii) an enhancer sequence as described above (iii) a nucleotide sequence of interest, and (iv) a terminator sequence.

Expression cassettes, expression constructs and expression systems of the invention may also comprise an untranslated region (UTR). The UTR may be located upstream of a terminator sequence present in the expression cassette (gene expression construct or gene expression system). Where the expression cassette (gene expression construct or gene expression system) comprises a nucleic acid encoding a protein of interest, the UTR may be located downstream of the nucleic acid of interest (3'UTR). Thus, the UTR may be located between a nucleic acid encoding a protein of interest and a terminator sequence. The UTR may be derived from a bipartite RNA virus, e.g. from the RNA-2 genome segment of a bipartite RNA virus. The UTR may be the 3' UTR of the same RNA-2 genome segment from which the enhancer sequence present in the expression cassette (expression construct or gene expression system) is derived. Preferably, the UTR is the 3' UTR of a comoviral RNA-2 genome segment, e.g. the 3' UTR of the CPMV RNA-2 genome segment.

The expression constructs as described above may be present in a vector. The vector may comprise border sequences which permit the transfer and integration of the expression cassette into the genome of the organism or host. The construct may be a plant binary vector, for example a binary transformation vector based on pPZP (Hajdukiewicz, et al. 1994). Other example constructs include pBin19 (see Frisch, D. A., L. W. Harris-Haller, et al. 1995, Plant Molecular Biology 27: 405-409).
If desired, the constructs of this invention may be further manipulated to include selectable markers. However, this may not be required. Useful selectable markers include enzymes that provide for resistance to chemicals such as an antibiotic for example, gentamycin, hygromycin, kanamycin, or herbicides such as phosphinothrycin, glyphosate, chlorosulfuron, and the like. Similarly, enzymes providing for production of a compound identifiable by colour change such as GUS (beta-glucuronidase), or luminescence, such as luciferase or GFP, may be used.

An example of a vector that may be used as described herein is a pEAQ vector which permits direct cloning by use of a polylinker between the 5' leader and 3' UTRs of an expression cassette. This vector may also include a translational enhancer as described herein, positioned on a T-DNA which also contains a suppressor of gene silencing and NPTII. The polylinker may also encode one or two sets of 6 x Histidine residues to allow the inclusion of N- or C-terminal His-tags to the protein of interest to facilitate protein purification.

Post-transcriptional gene silencing (PTGS) may be involved in limiting expression of transgenes in plants, and co-expression of a suppressor of silencing from the potato virus Y (HcPro) may be used to counteract the specific degradation of transgene mRNAs (Brigneti et al., 1998, EMBO J. 17, 6739-6746). Alternate suppressors of silencing are well known in the art and may be used as described herein (Chiba et al., 2006, Virology 346:7-14), for example but not limited to, TEV-p1/HC-Pro (Tobacco etch virus-p1/HC-Pro), BYV -p21, p19 of Tomato bushy stunt virus (TBSV p19; the construction of p19 is described in described in WO 2010/0003225), capsid protein of Tomato crinkle virus (TCV -CP), 2b of Cucumber mosaic virus; CMV-2b), p25 of Potato virus X (PVX-p25), p11 of Potato virus M (PVM-p11), p11 of Potato virus S (PVS-p11), p16 of Blueberry scorch virus, (BScV -p16), p23 of Citrus tristeza virus (CTV-p23), p24 of Grapevine leafroll-associated virus-2, (GLRaV-2 p24), p10 of Grapevine virus A, (GVA-p10), p14 of Grapevine virus B (GVB-p14), p10 of Heracleum latent virus (HLV-p10), or p16 of Garlic common latent virus (GCLV-p16).

Therefore, one or more suppressors of silencing, for example, but not limited to, HcPro, TEV -p1/HC-Pro, BYV-p21, TBSV p19, TCV-CP, CMV-2b, PVX-p25, rgscam, B2 protein from FHV, the small coat protein of CPMV, and coat protein from TCV, PVM-p11, PVS-p11, BScV-p16, CTV-p23, GLRaV-2 p24, GBV-p14, HLV-p10, GCLV-p16, or GVA-p10 may be co-expressed along with the comovirus-based expression cassette, geminivirus-derived amplification element, and the nucleic acid sequence encoding the protein of interest to further ensure high levels of protein production within a plant.

The expression systems may also comprise amplification elements from a geminivirus for example, an amplification element from the bean yellow dwarf virus (BeYDV). BeYDV belongs to the Mastreviruses genus adapted to dicotyledonous plants. BeYDV is monopartite having a single-strand circular DNA genome and can replicate to very high copy numbers by a rolling circle mechanism. BeYDV-derived DNA replicon vector systems have been used for rapid high-yield protein production in plants.

As used herein, the phrase "amplification elements" refers to a nucleic acid segment comprising at least a portion of one ore more long intergenic regions (or long intergenic repeat; LIR) of a geminivirus genome. As used herein, "long intergenic region", "long intergenic repeat", or "LIR", refers to a region of a long intergenic region that contains a rep binding site capable of mediating excision and replication by a geminivirus Rep protein. In some aspects, the nucleic acid segment comprising one or more LIRs, may further comprises a short intergenic region (or small intergenic region; SIR) of a geminivirus genome. As used herein, "short intergenic region", "small intergenic region" or "SIR", refers to the complementary strand (the short IR (SIR) of a Mastreviruses). Any suitable geminivirus-derived amplification element may be used herein. See, for example, WO2000/20557; WO2010/025285; Zhang X. et al. (2005, Biotechnology and Bioengineering, Vol. 93, 271-279), Huang Z. et al. (2009, Biotechnology and Bioengineering, Vol. 103, 706-714), Huang Z. et al.(2009, Biotechnology and Bioengineering, Vol. 106, 9-17). As shown if Figures 2B and 2D, if more than one LIR is used in the construct, for example two LIRs, then the promoter, CMPV-HT regions and the nucleic acid sequence of interest and the terminator are bracketed by each of the two LIRs. Furthermore, the amplification element might for example originate from the sequence as disclosed in Halley-Stott et al. (2007) Archives of Virology 152: 1237-1240, deposited under Gen Bank accession number DQ458791. The nucleic acid segment comprising LIRs and SIRs may be determined by aligning the desired sequence with the sequences provided herein for BeYDV. For example, the nucleic acid segment comprising LIRs of the sequence, Gen Bank DQ458791, comprise nucleotides 2401 to 2566 and 1 to 128.The nucleic acid segment comprising SIRs are nucleotides 1154 to 1212.

As described herein, co-delivery of bean yellow dwarf virus (BeYDV)-derived vector and a Rep/RepA-supplying vector, by agroinfiltration of *Nicotiana benthamiana* leaves results in efficient replicon amplification and robust protein production.

A comovirus-based expression cassette and a geminivirus-derived amplification element may be comprised in respective, first and second vectors, or the component parts may be included in one vector. If two vectors are used, the first and second vectors may be introduced into a plant cell simultaneously or separately.

A viral replicase may also be included in the expression system as described herein to increase expression of the nucleic acid of interest. An non-limiting example of a replicase is a BeYDV replicase (pREP110) encoding BeYDV Rep and RepA (C2/C1; Huang et al., 2009, Biotechnol. Bioeng. 103, 706-714). Another non-limiting example of a replicase is disclosed in Halley-Stott et al. (2007, Archives of Virology 152: 1237-1240), and deposited under Gen Bank accession number DQ458791, . The nucleic acid segment comprising C1:C2 gene comprises nucleotides 1310 to 2400.

By "co-expressed" it is meant that two or more than two nucleotide sequences are expressed at about the same time within the plant, and within the same tissue of the plant. However, the nucleotide sequences need not be expressed at exactly the same time. Rather, the two or more nucleotide sequences are expressed in a manner such that the encoded products have a chance to interact. The two or more than two nucleotide sequences can be co-expressed using a transient expression system, where the two or more sequences are introduced within the plant at about the same time under conditions that both sequences are expressed. Alternatively, a platform plant comprising one of the nucleotide sequences may be transformed in a stable manner, with an additional sequence encoding the protein of interest introduced into the platform plant in a transient manner.
The constructs of the present invention can be introduced into plant cells using Ti plasmids, Ri plasmids, plant virus vectors, direct DNA transformation, micro-injection, electroporation, etc. For reviews of such techniques see for example Weissbach and Weissbach, Methods for Plant Molecular Biology, Academy Press, New York VIII, pp. 421-463 (1988); Geierson and Corey, Plant Molecular Biology, 2d Ed. (1988); and Miki and Iyer, Fundamentals of Gene Transfer in Plants. In Plant Metabolism, 2d Ed. DT. Dennis, DH Turpin, DD Lefebrve, DB Layzell (eds), Addison Wesly, Langmans Ltd. London, pp. 561-579 (1997). Other methods include direct DNA uptake, the use of liposomes, electroporation, for example using protoplasts, micro-injection, microprojectiles or whiskers, and vacuum infiltration. See, for example, Bilang, et al. (1991, Gene 100: 247-250), Scheid et al. (1991, Mol. Gen. Genet. 228: 104-112), Guerche et al. (1987, Plant Science 52: 111-116), Neuhause et al. (1987, Theor. Appl Genet. 75: 30-36), Klein et al., (2987, Nature 327: 70-73); Freeman et al. (1984, Plant Cell Physiol. 29: 1353), Howell et al. (1980, Science 208: 1265), Horsch et al. (1985, Science 227: 1229-1231), DeBlock et al., (1989, Plant Physiology 91: 694-701), Methods for Plant Molecular Biology (Weissbach and Weissbach, eds., Academic Press Inc., 1988), Methods in Plant Molecular Biology (Schuler and Zielinski, eds., Academic Press Inc., 1989), WO 92/09696, WO 94/00583, EP 331083, EP 175966, Liu and Lomonossoff (2002, J Virol Meth, 105:343-348), EP 290395; WO 8706614; U.S. Pat. Nos. 4,945,050; 5,036,006; and 5,100,792, U.S. patent application Ser. Nos. 08/438,666, filed May 10, 1995, and 07/951,715, filed Sep. 25, 1992.
Transient expression methods may be used to express the constructs of the present invention (see D'Aoust et al., 2009, Methods in Molecular biology, Vol 483, pages41-50; Liu and Lomonossoff, 2002, Journal of Virological Methods, 105:343-348). Alternatively, a vacuum-based transient expression method, as described by Kapila et al., (1997, Plant Sci. 122, 101-108), or WO 00/063400, WO 00/037663 may be used. These methods may include, for example, but are not limited to, a method of Agro-inoculation or Agro-infiltration, syringe infiltration, however, other transient methods may also be used as noted above. With Agro-inoculation, Agro-infiltration, or syringe infiltration, a mixture of *Agrobacteria* comprising the desired nucleic acid enter the intercellular spaces of a tissue, for example the leaves, aerial portion of the plant (including stem, leaves and flower), other portion of the plant (stem, root, flower), or the whole plant. After crossing the epidermis the *Agrobacteria* infect and transfer t-DNA copies into the cells. The t-DNA is episomally transcribed and the mRNA translated, leading to the production of the protein of interest in infected cells, however, the passage of t-DNA inside the nucleus is transient.

Also considered part of this invention are transgenic plants, plant cells or seeds containing the chimeric gene construct of the present invention that may be used as a platform plant suitable for transient protein expression described herein. Methods of regenerating whole plants from plant cells are also known in the art (for example see Guerineau and Mullineaux (1993, Plant transformation and expression vectors. In: Plant Molecular Biology Labfax (Croy RRD ed) Oxford, BIOS Scientific Publishers, pp 121-148). In general, transformed plant cells are cultured in an appropriate medium, which may contain selective agents such as antibiotics, where selectable markers are used to facilitate identification of transformed plant cells. Once callus forms, shoot formation can be encouraged by employing the appropriate plant hormones in accordance with known methods and the shoots transferred to rooting medium for regeneration of plants. The plants may then be used to establish repetitive generations, either from seeds or using vegetative propagation techniques. Transgenic plants can also be generated without using tissue culture. Methods for stable transformation, and regeneration of these organisms are established in the art and known to one of skill in the art. Available techniques are reviewed in Vasil et al., (Cell Culture and Somatic Cell Genetics of Plants, VoI I, II and III, Laboratory Procedures and Their Applications, Academic Press, 1984), and Weissbach and Weissbach, (Methods for Plant Molecular Biology, Academic Press, 1989). The method of obtaining transformed and regenerated plants is not critical to the present invention.

If plants, plant portion, or plant cells are to be transformed or co-transformed by two or more nucleic acid constructs, the nucleic acid construct may be introduced into the *Agrobacterium* in a single transfection event the nucleic acids are pooled, and the bacterial cells transfected as described. Alternately, the constructs may be introduced serially. In this case, a first construct is introduced to the *Agrobacterium* as described, the cells grown under selective conditions (e.g. in the presence of an antibiotic) where only the singly transformed bacteria can grow. Following this first selection step, a second nucleic acid construct is introduced to the *Agrobacterum* as described, and the cells grown under doubly-selective conditions, where only the doubly-transformed bacteria can grow. The doubly-transformed bacteria may then be used to transform a plant, plant portion or plant cell as described herein, or may be subjected to a further transformation step to accommodate a third nucleic acid construct..

The present disclosure further provides a transgenic plant comprising the present expression system, wherein the heterologous nucleic acid of interest in the cassette is expressed at an enhanced level when compared to other analogous expression systems that lack one or more components of the expression system as described herein.

The present disclosure further comprises a method for generating a protein of interest, comprising the steps of providing a plant ,or plant part, that expresses the expression system as described herein, harvesting, at least, a tissue in which the protein of interest has been expressed and optionally, isolating the protein of interest from the tissue.

Thus in various aspects, and without limitation, the invention provides:
- one or more expression systems comprising a comovirus-based expression cassette, one or more BeYDV amplification elements, a promoter, a nucleic acid encoding a protein of interest, or a polylinker, and optionally a terminator.
- methods of expressing a protein of interest, in a host organism such as a plant using one or more expression systems or vectors as described herein.

**Table 1: lists of sequences**

| SEQ ID NO: | Description | Figure No |
|---|---|---|
| 1 | 972 - wild type HA0 of H5 A/Indonesia/5/2005 | 1A |
| 2 | 560 - PDISP-HA0 of H1 A/California/4/2009 | 1B |
| 3 | 971 - PDISP-HA0 of H3 A/Brisbane/10/2007 | 1C |
| 4 | 973 - PDISP-HA0 of B/Florida/4/2006 | 1D |
| 5 | primer - MfeI-MluI-AscI-LIR.c | - |
| 6 | primer - SbfI-AflII-HindIII-LIR.r | - |
| 7 | primer - SacI-EcoRI-SIR.c | - |
| 8 | primer - SpeI-FseI-LIR.r | - |
| 9 | 849 - LIR-MCS-SIR+LIR | 1E |
| 10 | 853 - LIR - PDISP-HA0 of B/Florida/4/2006 - SIR+LIR | 1F |
| 11 | 561 - LIR - PDISP-HA0 of H1 A/California/4/2009 - SIR+LIR | 1G |
| 12 | 851 - LIR - PDISP-HA0 of H3 A/Brisbane/10/2007 - SIR+LIR | 1H |
| 13 | 852 - LIR - Native Sp with HA0 from H5 A/Indonesia/05/2005 - SIR+LIR | 1I |
| 14 | 848 - LIR-MCS-SIR-replicase-LIR | 1J |
| 15 | 475 - LIR- PDISP-HA0 of B/Florida/4/2006 - SIR-C1/C2-LIR | 1K |
| 16 | 471 - LIR - PDISP-HA0 of H1 A/California/4/2009 - SIR-C1/C2-LIR | 1L |
| 17 | 473 - LIR - PDISP-HA0 of H3 A/Brisbane/10/2007 - SIR-C1/C2-LIR | 1M |
| 18 | 474 - LIR - Native Sp with HA0 from H5 A/Indonesia/05/2005 - SIR-C1/C2-LIR | 1N |
| 19 | Primer - Plasto-443c | - |
| 20 | Primer - supP19-plasto.r | - |
| 21 | Primer - supP19-1c | - |
| 22 | Primer - SupP19-SacI.r | |
| 23 | CPMV Enhancer Sequence | - |

The present invention will be further illustrated in the following examples. However it is to be understood that these examples are for illustrative purposes only, and should not be used to limit the scope of the present invention in any manner.

### Assembly of expression cassettes

Constructs that may be used for the production of VLPs are described in WO 2009/009876, WO 2009/076778 and WO2010/003225, and U.S. Provisional Application No. 61/220,161 (filed June 24, 2009. Non- limiting examples of constructs may also include those listed in Table 2. Assembly of these constructs is described in WO 2009/009876, WO 2009/076778, WO2010/003225 and US 61/220,161. However other constructs comprising known HAs, including but not limited to, those provided in Table 2, and combined with similar or different regulatory elements and promoters, may also be used for the production of VLPs as described herein.

**Table 2: Examples of constructs used for hemagglutinin production.**

| **Cassette number** | **Corresponding HA** | **HA abbreviation** |
|---|---|---|
| 540 | SpPDI-H1 from strain A/New Caledonia/20/99 (H1N1) | H1/NC |
| 560 | SpPDI-H1 A/California/4/2009 in 2X35S/CPMV-*HT* expression cassette | H1/Cal WT |
| 580 | SpPDI-H1 A/New Caledonia/20/99 in 2x35S/CPMV-*HT* expression cassette | H1/NC |
| 660 | H5 from strain A/Indonesia/5/2005 (H5N1) | H1/Indo |
| 663 | H5 A/Indonesia/5/2005 | H1/Indo |
| 685 | H5 A/Indonesia/5/2005 in CPMV-*HT* expression cassette | H1/Indo |
| 686 | SpPDI-H5 A/Indonesia/5/2005 in CPMV-*HT* expression cassette | H1/Indo |
| 690 | H1 A/Brisbane/59/07 receptor-binding (RB) domain in H5 A/Indonesia/5/05 backbone | H1/Bris |
| 691 | H1 A/Brisbane/59/07 esterase and receptor-binding domains (E1-RB-E2) in H5 A/Indonesia/5/05 backbone | H1/Bris |
| 696 | H5 A/Indonesia/5/05 receptor-binding (RB) domain in H1 A/New Caledonia/20/99 backbone | H1/Indo |
| 732 | H1 A/Brisbane/59/2007 in CPMV-*HT* expression cassette | H1/Bris |
| 733 | SpPDI-H1 A/Brisbane/59/2007 in CPMV-*HT* expression cassette | H1/Bris |
| 734 | H1 A/Brisbane/59/07 receptor-binding (RB) domain in H5 A/Indonesia/5/05 backbone in CPMV-HT expression cassette | H1/Bris |
| 735 | H3 A/Brisbane/10/2007 in CPMV-*HT* expression cassette | H3/Bris |
| 736 | SpPDI-H3 A/Brisbane/10/2007 in CPMV-*HT* expression cassette | H3/Bris |
| 737 | Assembly of chimeric SpPDI-H3 A/Brisbane/10/2007 (ectodomain) + H5 A/Indonesia/5/2005 (TmD + Cyto tail) in CPMV-*HT* expression cassette | H3/Bris-H5/Indo chimera |
| 738 | HA B/Florida/4/2006 in CPMV-*HT* expression cassette | B/Flo |
| 739 | SpPDI-HA B/Florida/4/2006 in CPMV-*HT* expression cassette | B/Flo |
| 745 | SpPDI-HA B/Florida/4/2006 (ectodomain) + H5 A/Indonesia/5/2005 (TmD + Cyto tail) in CPMV-*HT* expression cassette | B/Flo |
| 747 | SpPDI-HA B/Florida/4/2006+ H5 A/Indonesia/5/2005 (TmD + Cyto tail) in 2X35S-CPMV-*HT* expression cassette | B/Flo |
| 774 | HA of A/Brisbane/59/2007 (H1N1) | H1/Bris |
| 775 | HA of A/Solomon Islands 3/2006 (H1N1) | H1/Solomon |
| 776 | HA of A/Brisbane 10/2007 (H3N2) | H3/Bris |
| 777 | HA of A/Wisconsin/67/2005 (H3N2) | H3/Wisc |
| 778 | HA of B/Malaysia/2506/2004 | B/Malaysia |
| 779 | HA of B/Florida/4/2006 | B/Flo |
| 780 | HA of A/Singapore/1/57 (H2N2) | H2/Sing |
| 781 | HA of A/Anhui/1/2005 (H5N1) | H5/Anhui |
| 782 | HA of A/Vietnam/1194/2004 (H5N1) | H5/Vietnam |
| 783 | HA of A/Teal/HongKong/W312/97 (H6N1) | H6/HongKong |
| 784 | HA of A/Equine/Prague/56 (H7N7) | H7/Prague |
| 785 | HA of A/HongKong/1073/99 (H9N2) | H9/HongKong |
| 787 | H1 A/Brisbane/59/2007 | H1/Bris |
| 790 | H3 A/Brisbane/10/2007 | H3/Bris |
| 798 | HA B/Florida/4/2006 | B/Flo |

### EXAMPLES

### Materials and methods - Molecular cloning

### CaMV 2X35S-CPMV HT-based expression cassettes

2X35S-CPMV HT -Native Sp-HA0 H5 A/Indonesia/5/2005 (construct number 972; SEQ ID NO:1; Figure 1A). The assembly of construct number 972, comprising the coding region for HA0 from H5 A/Indonesia/5/2005 with its native signal peptide under the control of CaMV 2X35S-CPMV *HT* expression cassette was described previously (WO2010/003225). The sequence of the resulting expression cassette is presented in Figure 1A (SEQ ID NO:1).

2X35S-CPMV HT -SpPDI-HA0 H1 A/California/4/2009 (construct number 560; SEQ ID NO:1 2; Figure 1B). The assembly of construct number 560, comprising the nucleotide sequence encoding alfalfa PDI signal peptide (SpPDI) fused to HA0 from H1 A/California/4/2009 under the control of CaMV 2X35S-CPMV-*HT* was described previously (WO2010/003225). The sequence of the resulting expression cassette is presented in Figure 1B (SEQ ID NO:2).

2X35S-CPMV HT-SpPDI-HA0 H3 A/Brisbane/10/2007 (construct number 971; SEQ ID NO:3; Figure 1C). A sequence encoding alfalfa PDI signal peptide fused to HA0 from H3 A/Brisbane/10/2007 was assembled into CaMV 2X35S-CPMV-*HT* regulatory elements as follows. Construct number 736 was described previously (see PCT Publication No. WO 2010/003225 for assembly and sequence) - briefly, construct 736 was digested with restriction enzymes ApaI (immediately upstream of the ATG) and StuI (immediately downstream of the stop codon) to remove a fragment encoding SpPDI fused to HA0 from H3 A/Brisbane/10/2007. The resulting fragment was cloned into construct 972 (SEQ ID NO:1'; Figure 1A) previously digested with ApaI and StuI. The resulting construct was given number 971 (SEQ ID NO:3, Figure 1C).

2X35S-CPMV HT -SpPDI-HA0 B/Florida/4/2006 (construct number 973; SEQ ID NO:4; Figure 1D). A sequence encoding alfalfa PDI signal peptide fused to HA0 from B/Florida/4/2006 was assembled into to CaMV 2X35S-CPMV-*HT* as follows. Construct number 739 was described previously (see PCT Publication No. WO 2010/003225 for assembly and sequence) - briefly, construct 739 was digested with restriction enzymes ApaI (immediately upstream of the ATG) and StuI (immediately downstream of the stop codon) to remove a fragment encoding SpPDI fused to HA0 from B/Florida/4/2006. The resulting fragment was cloned into construct 972 previously digested with ApaI and StuI. The resulting construct was given number 973 (SEQ ID NO:4, Figure 1D).

### CaMV 2X35S-CPMV HT-based cassettes with BeYDV amplification elements

Acceptor plasmid containing LIR-Multiple cloning site (MCS)-SIR+LIR in pCAMBIA based-vector (construct 849; SEQ ID NO:9; Figure 1E). The long intergenic regions (LIR) and the short intergenic region (SIR) from Bean Yellow dwarf virus (BeYDV) were inserted into pCAMBIA binary vector as follow. A first PCR fragment containing BeYDV LIR sequence was amplified using MfeI-MIuI-AscI-LIR.c (SEQ ID NO:5):
CAATTGACGCGTGGCGCGCCCTAGCAGAAGGCATGTTGTTGTGACTCCGAGG and SbfI-AfIII-HindIII-LIR.r (SEQ ID NO:6):
CCTGCAGGCTTAAGAAGCTTGTACGAATAATTCGTATCCGACGGAA
as primers. The 2963 pb BglII restriction fragment of construct pBYGFP (kindly provided by Dr Hugh Mason, Arizona State University) was gel-extracted and used as template for the first PCR reaction. Vector pBYGFP has been previously described in Huang et al. (Biotechnology and Bioengineering 103: 706-714 (2009)). The resulting fragment was digested with SbfI and cloned into pCAMBIA2300 (Cambia, Canberra, Australia) previously digested with HindIII, treated with T4 DNA polymerase to create blunt end and finally digested with SbfI. The resulting plasmid was named pCAMBIA-LIR. A second fragment containing BeYDV LIR and SIR sequences was amplified using SacI-EcoRI-SIR.c (SEQ ID NO:7)
TACCGAGCTCGAATTCCGAGTGTACTTCAAGTCAGTTGGAAATC and SpeI-FseI-LIR.r (SEQ ID NO:8)
ACTAGTGGCCGGCCGTACGAATAATTCGTATCCGACGGAAATACCTGA as primers and the 2116 pb BglII restriction pBYGFP fragment as template. The resulting fragment was digested with SacI and cloned into pCAMBIA-LIR previously digested with EcoRI, treated with T4 DNA polymerase to create blunt end and finally digested with SacI. The resulting plasmid was named pCAMBIA-LIR-MCS-SIR+LIR. In order to change the orientation of the LIR-MCS-SIR+LIR sequence relative to the orientation of the T-DNA borders, pCAMBIA-LIR-MCS-SIR+LIR was digested with MluI and SpeI restriction enzymes and the resulting fragments were blunt-ended using T4 DNA polymerase and re-ligated. The clones obtained from the transformation of this ligation product were screened for orientation and a clone in which the LIR-MCS-SIR+LIR elements were in Left to Right T-DNA orientation was selected and kept as acceptor construct 849. An annotated LIR-MCS-SIR+LIR sequence is presented in SEQ ID NO:9 (Figure 1E).

2X35S-CPMV *HT*-SpPDI-HAO B/Florida/4/2006 in BeYDV amplification elements (construct number 853; SEQ ID NO:10; Figure 1F). A sequence encoding alfalfa PDI signal peptide fused to HA0 from B/Florida/4/2006 under the control of CaMV 2X35S-CPMV-*HT* into the BeYDV amplification elements was assembled as follows. Construct number 973 (SEQ ID NO: 4, Figure 1D) was digested with SbfI (upstream of the CaMV 2X35S promoter) and XbaI (downstream of the NOS terminator) to remove a fragment containing the whole 2X35S-CPMV *HT*-SpPDI-HA0 B/Florida/4/2006 expression cassette. The resulting fragment was cloned into construct number 849 (SEQ ID NO:9, Figure 1E) previously digested with SbfI and XbaI. The resulting construct was given number 853 (SEQ ID NO: 10, Figure 1F).

2X35S-CPMV HT -SpPDI-HA0 H1 A/California/4/2009 in BeYDV amplification elements (construct number 561; SEQ ID NO:11, Figure 1G). A sequence encoding alfalfa PDI signal peptide fused to HA0 from H1 A/California/4/2009 under the control of CaMV 2X35S-CPMV-*HT* into the BeYDV amplification elements was assembled as follows. Construct number 560 (SEQ ID NO:2, Figure 1B) was digested with SbfI (upstream of the CaMV 2X35S promoter) and StuI (downstream of the Stop codon) to remove a fragment containing the 2X35S-CPMV *HT*-SpPDI-H1 A/California/4/2009 expression cassette without NOS terminator. The resulting fragment was cloned into construct number 853 (SEQ ID NO: 10, Figure 1F) previously digested with SbfI and StuI (containing the NOS terminator). The resulting construct was given number 561 (SEQ ID NO:11, Figure 1G).

2X35S-CPMV HT -SpPDI-HA0 H3 A/Brisbane/10/2007 in BeYDV amplification elements (construct number 851; SEQ ID NO:12, Figure 1H). A sequence encoding alfalfa PDI signal peptide fused to HA0 from H3 A/Brisbane/10/2007 under the control of CaMV 2X35S-CPMV-HT into the BeYDV amplification elements was assembled as follows. Construct number 971 (SEQ ID NO:3, Figure 1C) was digested with SbfI (upstream of the CaMV 2X35S promoter) and StuI (downstream of the Stop codon) to remove a fragment containing the 2X35S-CPMV HT -SpPDI-H3 A/Brisbane/10/2007 expression cassette without NOS terminator. The resulting fragment was cloned into construct number 853 (SEQ ID NO: 10, Figure 1F) previously digested with SbfI and StuI (containing the NOS terminator). The resulting construct was given number 851 (SEQ ID NO: 12, Figure 1H).

2X35S-CPMV HT -Native Sp-HA0 H5 A/Indonesia/5/2005 in BeYDV amplification elements (construct number 852; SEQ ID NO:13, Figure 1I). A sequence encoding HA0 from H5 A/Indonesia/5/2005 with is native signal under the control of CaMV 2X35S-CPMV-HT into the BeYDV amplification elements was assembled as follows. Construct number 972 (SEQ ID NO:1 Figure 1A) was digested with SbfI (upstream of CaMV 2X35S promoter) and StuI (downstream of the Stop codon) to remove a fragment containing the 2X35S-CPMV HT - Native Sp-HA0 H5 A/Indonesia/5/2005 expression cassette without NOS terminator. The resulting fragment was cloned into construct number 853 (SEQ ID NO: 10, Figure 1F) previously digested with SbfI and StuI (containing the NOS terminator). The resulting construct was given number 852 (SEQ ID NO: 13, Figure 1I).

### Replicase expression construct

Replicase expression construct (construct number 834). The plasmid bearing the construct for the expression of BeYDV replicase was kindly provided by Dr Hugh Mason (Arizona State University). Construct number 834 corresponds to plasmid pREP110 described in Huang et al. (2009, Biotechnol. Bioeng. 103, 706-714).

### CaMV 2X35S-CPMV HT-based cassettes with BeYDV amplification elements and replicase gene under the control of LIR

Acceptor plasmid containing LIR-Multiple cloning site (MCS)-SIR-replicase-LIR into pCAMBIA based-vector (construct 848; SEQ ID NO:14, Figure 1J). The *BeYDV* replicase (C1/C2 with deleted intron) was inserted between BeYDV SIR and LIR repeat as follows. A PCR fragment was amplified using SacI-EcoRI-SIR.c (SEQ ID NO:7) and SpeI-FseI-LIR.r (SEQ ID NO:8) as primers. The 2180 pb SacI-FseI restriction fragment of construct pBYGFP.R (kindly provided by Dr Hugh Mason, Arizona State University) was gel-extracted and used as template for the PCR reaction. Vector pBYGFP.R has been previously described in Huang et al. (Biotechnology and Bioengineering 103: 706-714 (2009)). The resulting fragment was digested with SacI and cloned into construct 849 (SEQ ID NO:9, Figure 1E) previously digested with MfeI, treated with T4 DNA polymerase to create blunt end and finally digested with SacI. The resulting acceptor construct was given number 848 (SEQ ID NO:14; Figure 1J).

2X35S-CPMV HT -SpPDI-HAO B/Florida/4/2006 in BeYDV+replicase amplification system (construct number 475; SEQ ID NO:15, Figure 1K). A sequence encoding alfalfa PDI signal peptide fused to HA0 from B/Florida/4/2006 under the control of CaMV 2X35S-CPMV-*HT* into the BeYDV with replicase amplification system was assembled as follows. Construct number 973 (SEQ ID NO:4, Figure 1D) was digested with SbfI (upstream of the CaMV 2X35S promoter) and XbaI (downstream of the NOS terminator) to remove a fragment containing the whole 2X35S-CPMV *HT* -SpPDI-HA0 B/Florida/4/2006 expression cassette. The resulting fragment was cloned into construct number 848 (SEQ ID NO:14, Figure 1J) previously digested with SbfI and XbaI. The resulting construct was given number 475 (SEQ ID NO: 15, Figure 1K).

2X35S-CPMV HT -SpPDI-HAO H1 A/Califomia/4/2009in BeYDV+replicase amplification system (construct number 471; SEQ ID N0:16, Figure 1L). A sequence encoding alfalfa PDI signal peptide fused to HA0 from H1 A/California/4/2009 under the control of CaMV 2X35S-CPMV-*HT*into the BeYDV with replicase amplification system was assembled as follows. Construct number 560 (SEQ ID NO:2, Figure 1B) was digested with SbfI (upstream of the CaMV 2X35S promoter) and StuI (downstream of the Stop codon) to remove a fragment containing the 2X35S-CPMV *HT*-SpPDI-H1 A/Califomia/4/2009 expression cassette without NOS terminator. The resulting fragment was cloned into construct number 475 (SEQ ID NO: 15, Figure 1K) previously digested with SbfI and StuI (containing the NOS terminator). The resulting construct was given number 471 (SEQ ID NO:16, Figure 1L).

2X35S-CPMV HT -SpPDI-HAO H3 A/Brisbane/10/2007 in BeYDV+replicase amplification system(construct number 473; SEQ ID NO:17, Figure 1M). A sequence encoding alfalfa PDI signal peptide fused to HA0 from H3 A/Brisbane/10/2007 under the control of CaMV 2X35S-CPMV-HT into the BeYDV with replicase amplification system was assembled as follows. Construct number 971 (SEQ ID NO:3, Figure 1C) was digested with SbfI (upstream of the CaMV 2X35S promoter) and StuI (downstream of the Stop codon) to remove a fragment containing the 2X35S-CPMV HT -SpPDI-H3 A/Brisbane/10/2007 expression cassette without NOS terminator. The resulting fragment was cloned into construct number 853 (SEQ ID NO:10, Figure 1F) previously digested with SbfI and StuI (containing the NOS terminator). The resulting construct was given number 473 (SEQ ID NO: 17, Figure 1M).

2X35S-CPMV HT-Native Sp-HAO H5 A/Indonesia/5/2005 in BeYDV+replicase amplification system(construct number 474; SEQ ID NO: 18, Figure 1N). A sequence encoding HA0 from H5 A/Indonesia/5/2005 with is native signal under the control of CaMV 2X35S-CPMV-HT into the BeYDV with replicase amplification system was assembled as follows. Construct number 972 (SEQ ID NO:1, Figure 1A) was digested with SbfI (upstream of the CaMV 2X35S promoter) and StuI (downstream of the Stop codon) to remove a fragment containing the 2X35S-CPMV HT - Native Sp-HAO H5 A/Indonesia/5/2005 expression cassette without NOS terminator. The resulting fragment was cloned into construct number 853 (SEQ ID NO:10, Figure 1F) previously digested with SbfI and StuI (containing the NOS terminator). The resulting construct was given number 474 (SEQ ID NO: 18, Figure 1N).

Plastocyanin-P19 (construct number R472). The construction of p19 is described in WO 2010/0003225. Briefly, the coding sequence of p19 protein of tomato bushy stunt virus (TBSV) was linked to the alfalfa plastocyanin expression cassette by the PCR-based ligation method presented in Darveau et al. (Methods in Neuroscience 26: 77-85(1995)). In a first round of PCR, a segment of the plastocyanin promoter was amplified using primers Plasto-443c (SEQ ID NO: 19):
GTATTAGTAATTAGAATTTGGTGTC
   and supP19-plasto.r (SEQ ID NO:20)
CCTTGTATAGCTCGTTCCATTTTCTCTCAAGATG
with construct 660 (described in WO 2010/0003225) as template. In parallel, another fragment containing the coding sequence of p19 was amplified with primers supP19-1c (SEQ ID NO:21):
ATGGAACGAGCTATACAAGG
   and SupP19-SacI.r (SEQ ID NO:22):
AGTCGAGCTCTTACTCGCTTTCTTTTTCGAAG
using construct 35S:p19 as described in Voinnet et al. (2003, The Plant Journal 33: 949-956) as template. Amplification products were then mixed and used as template for a second round of amplification (assembling reaction) with primers Plasto-443c and SupP19-SacI.r. The resulting fragment was digested with BamHI (in the plastocyanin promoter) and SacI (at the end of the p19 coding sequence) and cloned into construct number 660, previously digested with the same restriction enzymes to give construct number R472. The plasmids were used to transform *Agrobacteiarm tarmefaciens* (AGL1; ATCC, Manassas, VA 20108, USA) by electroporation (Mattanovich et al., 1989, Nucleic Acids Res. 17 , 6747). The integrity of all *A*. *tarmefaciens* strains were confirmed by restriction mapping. The *A. tarmefaciens* strain comprising R472 is termed "AGL1/R472".

### Preparation of plant biomass, inoculum, agroinfiltration, and harvesting

*Nicotiana benthamiana* plants were grown from seeds in flats filled with a commercial peat moss substrate. The plants were allowed to grow in the greenhouse under a 16/8 photoperiod and a temperature regime of 25°C day/20°C night. Three weeks after seeding, individual plantlets were picked out, transplanted in pots and left to grow in the greenhouse for three additional weeks under the same environmental conditions.

*Agrobacteria* transfected with each construct were grown in a YEB medium supplemented with 10 mM 2-(N-morpholino)ethanesulfonic acid (MES), 20 µM acetosyringone, 50 µg/ml kanamycin and 25 µg/ml of carbenicillin pH5.6 until they reached an OD₆₀₀ between 0.6 and 1.6. *Agrobacterium* suspensions were centrifuged before use and resuspended in infiltration medium (10 mM MgCl₂ and 10 mM MES pH 5.6). and stored overnight at 4°C. On the day of infiltration, culture batches were diluted in 2.5 culture volumes and allowed to warm before use. Whole plants of *N. benthamiana* were placed upside down in the bacterial suspension in an air-tight stainless steel tank under a vacuum of 20-40 Torr for 2-min. Plants were returned to the greenhouse for a 2-6 day incubation period until harvest. Unless otherwise specified, all infiltrations were performed as co-infiltration with strain AGL1/R472 in a 1:1 or 1:1:1 ratio when co-infiltrated with AGL1/834 for the co-expression of the *BeYDV* replicase.

A. *tarmefaciens* strains comprising the various constructs as described herein are referred to by using an "AGL1"prefix. For example *A*. *tarmefaciens* comprising construct number 972 (Figure 1A), is termed "AGL1/972".

### Leaf sampling and total protein extraction

Following incubation, the aerial part of plants was harvested, frozen at -80°C, crushed into pieces. Total soluble proteins were extracted by homogenizing (Polytron) each sample of frozen-crushed plant material in 3 volumes of cold 50 mM Tris pH 8.0, 0.15 M NaCl, 0.1% Triton X-100 and 1 mM phenylmethanesulfonyl fluoride. After homogenization, the slurries were centrifuged at 10,000 g for 10 min at 4°C and these clarified crude extracts (supernatant) kept for analyses.

### Protein Analysis and Immunoblotting

The total protein content of clarified crude extracts was determined by the Brad ford assay (Bio-Rad, Hercules, CA) using bovine serum albumin as the reference standard. Proteins were separated by SDS-PAGE and electrotransferred onto polyvinylene difluoride (PVDF) membranes (Roche Diagnostics Corporation, Indianapolis, IN) for immunodetection. Prior to immunoblotting, the membranes were blocked with 5% skim milk and 0.1% Tween-20 in Tris-buffered saline (TBS-T) for 16-18h at 4°C.

Immunoblotting was performed by incubation with a suitable primary antibody (Table 3), in 2 µg/ml in 2% skim milk in TBS-Tween 20 0.1%. Secondary antibodies used for chemiluminescence detection were as indicated in Table 3, diluted as indicated in 2% skim milk in TBS-Tween 20 0.1%. Immunoreactive complexes were detected by chemiluminescence using luminol as the substrate (Roche Diagnostics Corporation). Horseradish peroxidase-enzyme conjugation of human IgG antibody was carried out by using the EZ-Link Plus^{®} Activated Peroxidase conjugation kit (Pierce, Rock ford, IL).

**Table 3: Electrophoresis conditions, antibodies, and dilutions for immunoblotting of expressed proteins.**

| HA subtype | Influenza strain | Electrophoresis condition | Primary antibody | Dilution | Secondary antibody | Dilution |
|---|---|---|---|---|---|---|
| H1 | A/California/04 /09 (H1N1) | Reducing | FII 10-I50F | 4 µg/ml | Goat antimouse (JIR 115-035-146) | 1:10 000 |
| H3 | A/Brisbane/10/ 2007 (H3N2) | Non-Reducing | TGA AS393 | 1:4000 | Rabbit anti-sheep (JIR 313-035-045) | 1:10 000 |
| H5 | A/Indonesia/5/ 2005 (H5N1) | Reducing | ITC IT -003-005V | 1:4000 | Goat anti-rabbit (JIR 111-035-144) | 1:10 000 |
| B | B/Florida/4/200 6 | Non-Reducing | NIBSC 07/356 | 1:2000 | Rabbit anti-sheep (JIR 313-035-045) | 1:10 000 |

| | | | | | | |
|---|---|---|---|---|---|---|
| FII: Fitzgerald Industries International, Concord, MA, USA; NIBSC: National Institute for Biological Standards and Control; JIR: Jackson ImmunoResearch, West Grove, PA, USA; ITC: Immune Technology Corporation, Woodside, NY, USA; TGA: Therapeutic Goods Administration, Australia. | | | | | | |

### Example 1 - Combining CPMV-HT expression system with BeYDV amplification system.

Sainsbury and Lomonossoff have developed an efficient system for the expression of recombinant proteins in plants (2008, Plant Physiology 148, 1212-1218). As taught in WO 2009/087391, the system uses untranslated regions (UTRs) of the genomic RNA 2 of the cowpea mosaic virus (CPMV) in which the two first translation initiation codons found in the 5'leader sequence have been deleted. When combined to the CaMV 35S promoter and the nopaline synthase (NOS) terminator, the modified CPMV UTRs were shown to enhance translation of the flanking coding region. The CPMV-based expression system was named CPMV-HT (hyperanslatable).

WO 2010 /025285 teaches that the DNA amplification system based on the replication machinery of the bean yellow dwarf virus (BeYDV) efficiently increases transient expression level of recombinant proteins in plants.

The CPMV-HT expression system for the production of influenza hemagglutinin (HA) by agroinfiltration in *Nicotiana benthamiana* was found to produce high levels of HA accumulation in several but not all of the HA types that were tested (data not shown). It was also observed that the accumulation level of expressible HA could be enhanced by replacing the CaMV 35S promoter by a modified version, named double CaMV 35S promoter bearing a duplication of upstream regulatory element (data not shown).

To further enhance HA accumulation levels, DNA constructs were prepared that combined the CPMV-HT expression system with the BeYDV amplification system. The assembly of the constructs is described in the materials and methods section (above; see Figure 1A-1M and Table 1). A schematic representation of the constructs tested is presented in Figure 2. The resulting constructs were compared to their CPMV-HT-based counterparts for their efficacy to drive high accumulation level of HA in agroinfiltrated plants. Plants producing HA under the control of CPMV-HT were harvested and frozen 5-6 days post infiltration whereas the plants producing HA under the control of CPMV-HT + BeYDV were harvested and frozen 3-4 days post infiltration. On the day of analysis, total soluble proteins were extracted from frozen biomasses and HA accumulation level was analyzed by Western blot as described above.

Western blot analysis of HA accumulation level in the biomass showed that a high level of HA from type B virus (from influenza B/Florida/4/2006) was detected in the plants transformed with constructs combining the BeYDV amplification system to the CPMV-HT expression system (AGL1/853; see Figure 1F + AGL1/834 + AGL1/R472), contrasting with the non-detectable expression of the same influenza B virus HA when using the CPMV-HT system alone (AGL1/973; see Figure 1D + AGL1/R472; Figure 3A). A comparison of the same expression strategies for H3 (A/Brisbane/10/2007; H3N2) indicates that although CPMV-HT alone drives detectable expression of H3 (AGL1/971; see Figure 1C+ AGL1/R472), the combination of the BeYDV amplification system to CPMV-HT further increased H3 accumulation level (AGL1/851; see Figure 1H + AGL1/834 + AGL1/R472; Figure 3B).

With H5 from influenza A/Indonesia/05/2005, higher accumulation was observed when using the CPMV-HT expression system alone (AGL1/972; see Figure 1A + AGL1/R472) than when compared with the constructs combining the BeYDV amplification system to the CPMV-HT expression system (AGL1/852; Figure 1I + AGL1/834 + AGL1/R472). This was particularly visible when comparing the 0.25 µg to 1 µg loads (Figure 3C). H1 accumulation from strain A/California/04/2009 (H1N1), was lower in plants transformed with AGL1/561 (see Figure 1G) + AGL1/834 + AGL1/R472 (H1 under the control of CPMV-HT + BeYDV), when compared with plants transformed with AGL1/560 (; see Figure 1B) + AGL1/R472 (H1 under the control of CPMV-HT alone (see Figure 3D). The accumulation profile of H1 was further studied to ensure that the timing of the peak of accumulation did not differ from that of other HAs tested. Maximum accumulation of H1 was observed between days 3 and 5 post infiltration when using the CPMV-HT expression system (AGL1/560 + AGL1/R472 )whereas the combined CPMV-HT/BeYDV system (AGL1/561 + AGL1/834 + AGL1/R472) resulted in lower H1 accumulation between days 2 and 4 (Figure 3D). These results indicate that the combination of BeYDV amplification system to the CPMV-HT-based expression system is not always beneficial and in some combinations, the combination may reduce HA accumulation compared to the use of a CPMV-HT expression system alone.

### Example 2. Producing HA in plants using combined CPMV-HT expression system and BeYDV amplification system in a single plasmid.

WO 2010/025285 teaches the use of a BeYDV-based DNA amplification system assembled in a single vector. In this system, the replicase genes (C1 and C2) are under the control of the viral LIR promoter. The results presented showed that the single vector BeYDV system was equivalent to the BeYDV system with the replicase genes on a separate plasmid. We have tested if a CPMV-HT + BeYDV expression system would be efficient for HA expression when assembled on a single vector. A schematic representation of the single vector for HA and replicase expression is presented in Figure 2D. In all cases tested, the single vector system bearing the C1/C2 genes (i.e. replicase in cis) under the control of the viral LIR promoter produced more HA than the dual vector system in which the replicase genes are on a separate plasmid (replicase in trans). Results are presented in Figures 4A-4D.

It is contemplated that any embodiment discussed in this specification can be implemented or combined with respect to any method or composition of the invention, and vice versa. Furthermore, compositions of the invention can be used to achieve methods of the invention.

Any terms not directly defined herein shall be understood to have the meanings commonly associated with them as understood within the art of the invention. Certain terms are discussed below, or elsewhere in the specification, to provide additional guidance to the practitioner in describing the devices, methods and the like of embodiments of the invention, and how to make or use them. It will be appreciated that the same thing may be said in more than one way. Consequently, alternative language and synonyms may be used for any one or more of the terms discussed herein. No significance is to be placed upon whether or not a term is elaborated or discussed herein. Some synonyms or substitutable methods, materials and the like are provided. Recital of one or a few synonyms or equivalents does not exclude use of other synonyms or equivalents, unless it is explicitly stated. Use of examples in the specification, including examples of terms, is for illustrative purposes only.

## Claims

1. A plant expression system comprising a first nucleic acid sequence comprising a regulatory region, operatively linked with a one or more than one comovirus enhancer, a nucleotide sequence encoding an influenza hemagglutinin wherein the influenza hemagglutinin is selected from influenza type B hemagglutinin and influenza type A subtype H3 hemagglutinin and one or more than one geminivirus amplification element, and a second nucleic acid sequence encoding a geminivirus replicase.

2. The plant expression system of claim 1, wherein the regulatory region is selected from a plastocyanin promoter, a CaMV 35S promoter, a 2x CaMV35S promoter, a CAS promoter, a RbcS promoter, a Ubi promoter, and an actin promoter.

3. The plant expression system of claim 1 or 2, wherein the one or more than one comovirus enhancer is a comovirus 5' UTR, a Cowpea Mosaic Virus (CPMV) 5'UTR, or comprises the nucleotide sequence of SEQ ID NO: 23.

4. The plant expression system of any one of claim 1-3, wherein the first nucleic acid sequence further comprises a Cowpea Mosaic Virus 3' UTR or a plastocyanin 3' UTR.

5. The plant expression system of any one of claim 1-4, wherein the first nucleic acid sequence and the second nucleic acid sequence are located on the same nucleic acid molecule, or they are located on different DNA molecules.

6. The plant expression system of any one of claim 1-5, wherein the one or more than one geminivirus amplification element is selected from a Bean Yellow Dwarf Virus long intergenic region (BeYDV LIR), and a BeYDV short intergenic region (BeYDV SIR).

7. The plant expression system of any one of claim 1-6, wherein the influenza hemagglutinin comprises a native signal peptide sequence, or a non-native signal peptide.

8. The plant expression system of any one of claim 1-7, further comprising a third nucleic acid sequence, the third nucleic acid sequence encoding a suppressor of silencing, wherein the suppressor of silencing is HcPro or pa19.

9. The plant expression system of claim 8, further comprising a forth nucleic acid sequence, the forth nucleic acid sequence encoding a chaperone protein, wherein the chaperone protein is Hsp40 or Hsp70.

10. A method of producing an influenza hemagglutinin or an influenza virus like particle (VLP) in a plant or in a portion of a plant, the method comprising introducing into the plant or in the portion of a plant the plant expression system of claim 1, and incubating the plant or the portion of a plant under conditions that permit expression of the nucleotide sequence encoding the influenza hemagglutinin, thereby producing the influenza hemagglutinin or the influenza VLP.

11. The method of claim 10, wherein in the step of introducing, the first nucleic acid sequence and the second nucleic acid sequence are located on the same molecule, or the first nucleic acid sequence and the second nucleic acid sequence are located on different molecules.

12. The method of claim 11, wherein the first nucleic acid sequence and the second nucleic acid sequence are located on different molecules, and the different molecules are introduced in the plant or in the portion of a plant at the same time.

13. The method of any one of claim 10-12, wherein in the step of incubating, the expression of the nucleotide sequence encoding the influenza hemagglutinin is a transient expression.

14. The method of claim any one of claim 10-13, further comprising a step of harvesting the plant or the portion of a plant, thereby obtaining a harvested plant or portion of a plant containing the influenza VLP.

15. The method of claim 14, further comprising a step of isolating the influenza VLP from the harvested plant or portion of a plant containing the influenza VLP, thereby obtaining an isolated influenza VLP.

## Patentansprüche

1. Pflanzenexpressionssystem, umfassend eine erste Nukleinsäuresequenz, umfassend eine mit einem oder mehr als einem Comovirusverstärker operativ verbundene regulatorische Region, eine ein Influenzahämagglutinin kodierende Nukleotidsequenz, wobei das Influenzahämagglutinin ausgewählt ist aus Influenza-Typ-B-Hämagglutinin und Influenza-Typ-A-Subtyp-H3--Hämagglutinin und einem oder mehr als einem Geminivirusamplifikationselement, und eine zweite Nukleinsäuresequenz, die eine Geminivirusreplikase kodiert.

2. Pflanzenexpressionssystem nach Anspruch 1, wobei die regulatorische Region ausgewählt ist aus einem Plastocyaninpromotor, einem CaMV35S-Promotor, einem 2x-CaMV35S-Promotor, einem CAS-Promotor, einem RbcS-Promotor, einem Ubi-Promotor und einem Actinpromotor.

3. Pflanzenexpressionssystem nach Anspruch 1 oder 2, wobei der eine oder mehr als eine Comovirusverstärker ein Comovirus-5 `UTR, ein Kuhbohnen-Mosaikvirus-(CPMV)-5'UTR ist oder die Nukleotidsequenz von SEQ ID NO: 23 umfasst.

4. Pflanzenexpressionssystem nach einem der Ansprüche 1-3, wobei die erste Nukleinsäuresequenz ferner eine Kuhbohnen-Mosaikvirus-3'UTR oder eine Plastocyanin-3'UTR umfasst.

5. Pflanzenexpressionssystem nach einem der Ansprüche 1-4, wobei sich die erste Nukleinsäuresequenz und die zweite Nukleinsäuresequenz am selben Nukleinsäuremolekül befinden oder sie sich an verschiedenen DNA-Molekülen befinden.

6. Pflanzenexpressionssystem nach einem der Ansprüche 1-5, wobei das eine oder mehr als eine Geminivirusamplifikationselement ausgewählt ist aus einer langen intergenen Bohnen-Yellow-Dwarft-Virus-Region (BeYDV LIR) und einer kurzen intergenen BeYDV-Region (BeYDV SIR).

7. Pflanzenexpressionssystem nach einem der Ansprüche 1-6, wobei das Influenzahämagglutinin eine native Signalpeptidsequenz oder ein nicht-natives Signalpeptid umfasst.

8. Pflanzenexpressionssystem nach einem der Ansprüche 1-7, ferner umfassend eine dritte Nukleinsäuresequenz, wobei die dritte Nukleinsäuresequenz einen Abschaltungssuppressor kodiert, wobei der Abschaltungssuppressor HcPro oder p19 ist.

9. Pflanzenexpressionssystem nach Anspruch 8, ferner umfassend eine vierte Nukleinsäuresequenz, wobei die vierte Nukleinsäuresequenz ein Chaperonprotein kodiert, wobei das Chaperonprotein Hsp40 oder Hsp70 ist.

10. Verfahren zur Herstellung eines Influenzuahämagglutinins oder eines influenzavirusartigen Partikels (VLP) in einer Pflanze oder in einem Pflanzenteil, wobei das Verfahren das Einbringen des Pflanzenexpressionssystems nach Anspruch 1 in die Pflanze oder den Pflanzenteil und Inhibieren der Pflanze oder des Pflanzenteils unter Bedingungen, die die Expression der das Influenzahämagglutinin kodierenden Nukleinsäuresequenz gewähren, umfasst, wodurch das Influenzahämagglutinin oder das Influenza-VLP hergestellt wird.

11. Verfahren nach Anspruch 10, wobei in der Einbringungsstufe sich die erste Nukleinsäuresequenz und die zweite Nukleinsäuresequenz am selben Molekül befinden oder sich die erste Nukleinsäuresequenz und die zweite Nukleinsäuresequenz an verschiedenen Molekülen befinden.

12. Verfahren nach Anspruch 11, wobei sich die erste Nukleinsäuresequenz und die zweite Nukleinsäuresequenz an verschiedenen Molekülen befinden und die verschiedenen Moleküle in die Pflanze oder in den Pflanzenteil gleichzeitig eingebracht werden.

13. Verfahren nach einem der Ansprüche 10-12, wobei in der Einbringungsstufe die Expression der das Influenzahämagglutinin kodierenden Nukleinsäuresequenz eine transiente Expression ist.

14. Verfahren nach einem der Ansprüche 10-13, ferner umfassend eine Stufe des Erntens der Pflanze oder des Pflanzenteils, wodurch eine geerntete Pflanze oder ein geernteter Pflanzenteil erhalten wird, die/der das Influenza-VLP enthält.

15. Verfahren nach Anspruch 14, ferner umfassend eine Stufe des Isolierens des Influenza-VLPs von der geernteten Pflanze oder dem geernteten Pflanzenteil, die/der das Influenza-VLP enthält, wodurch ein isoliertes Influenza-VLP erhalten wird.

## Revendications

1. Système d'expression d'origine végétale comprenant une première séquence d'acide nucléique comprenant une région de régulation, liée de manière fonctionnelle à un ou plus d'un amplificateur de comovirus, une séquence nucléotidique codant pour une hémagglutinine de la grippe dans laquelle l'hémagglutinine de la grippe est choisie parmi l'hémagglutinine de la grippe de type B et l'hémagglutinine de la grippe de type A sous-type H3 et un ou plus d'un élément d'amplification de géminivirus, et une deuxième séquence d'acide nucléique codant pour une réplicase de géminivirus.

2. Système d'expression d'origine végétale selon la revendication 1, dans lequel la région de régulation est choisie parmi un promoteur de plastocyanine, un promoteur CaMV 35S, un promoteur 2x CaMV 35S, un promoteur CAS, un promoteur RbcS, un promoteur Ubi et un promoteur d'actine.

3. Système d'expression d'origine végétale selon la revendication 1 ou 2, dans lequel l'un ou plus d'un amplificateur de comovirus est une 5' UTR de comovirus, une 5' UTR du virus de la mosaïque du dolique (CPMV), ou comprend la séquence nucléotidique de SEQ ID NO : 23.

4. Système d'expression d'origine végétale selon la revendication 3, dans lequel l'un ou plus d'un amplificateur de comovirus comprend en outre une 3' UTR de comovirus est une 3'UTR du virus de la mosaïque du dolique ou une 3' UTR de la plastocyanine.

5. Système d'expression d'origine végétale selon l'une quelconque des revendications 1 à 4, dans lequel la première séquence d'acide nucléique et la deuxième séquence d'acide nucléique sont situées sur la même molécule d'acide nucléique, ou elles sont situées sur des molécules d'ADN différentes.

6. Système d'expression d'origine végétale selon l'une quelconque des revendications 1 à 5, dans lequel l'un ou plus d'un élément d'amplification de géminivirus est choisi parmi la région intergénique longue du virus du nanisme jaune du haricot (BeYDV LIR) et une région intergénique courte du BeYDV (BeYDV SIR).

7. Système d'expression d'origine végétale selon l'une quelconque des revendications 1 à 6, dans lequel l'hémagglutinine de la grippe comprend une séquence native de peptide signal, ou un peptide signal non natif.

8. Système d'expression d'origine végétale selon l'une quelconque des revendications 1 à 7, comprenant en outre une troisième séquence d'acide nucléique, la troisième séquence d'acide nucléique codant pour un suppresseur de silencing, dans lequel le suppresseur de silencing est HcPro ou p19.

9. Système d'expression d'origine végétale selon la revendication 8, comprenant en outre une quatrième séquence d'acide nucléique, la quatrième séquence d'acide nucléique codant pour une protéine chaperon, dans lequel la protéine chaperon est Hsp40 ou Hsp70.

10. Procédé de production d'une hémagglutinine de la grippe ou d'une particule du type virus (VLP) de la grippe dans une plante ou une partie d'une plante, le procédé comprenant l'introduction dans la plante ou dans la partie d'une plante du système d'expression d'origine végétale selon la revendication 1, et la mise en incubation de la plante ou de la partie d'une plante dans des conditions permettant l'expression de la séquence nucléotidique codant pour l'hémagglutinine de la grippe, pour ainsi produire l'hémagglutinine de la grippe ou la VLP de la grippe.

11. Procédé selon la revendication 10, dans lequel dans l'étape d'introduction, la première séquence d'acide nucléique et la deuxième séquence d'acide nucléique sont situées sur la même molécule, ou la première séquence d'acide nucléique et la deuxième séquence d'acide nucléique sont situées sur des molécules différentes.

12. Procédé selon la revendication 11, dans lequel la première séquence d'acide nucléique et la deuxième séquence d'acide nucléique sont situées sur des molécules différentes, et les différentes molécules sont introduites dans la plante ou dans la partie d'une plante en même temps.

13. Procédé selon l'une quelconque des revendications 10 à 12, dans lequel, dans l'étape de mise en incubation, l'expression de la séquence nucléotidique codant pour l'hémagglutinine de la grippe est une expression transitoire.

14. Procédé selon l'une quelconque des revendications 10 à 13, comprenant en outre une étape de récolte de la plante ou de la partie d'une plante, pour ainsi obtenir une plante ou partie d'une plante récoltée contenant la VLP de la grippe.

15. Procédé selon la revendication 14, comprenant en outre une étape d'isolement de la VLP de la grippe à partir de la plante ou partie d'une plante récoltée contenant la VLP de la grippe, pour ainsi obtenir une VLP de la grippe isolée.
